# EUROPÄISCHE PATENTSCHRIFT

(11) **EP 1 599 480 B1**
(45) Veröffentlichungstag und Bekanntmachung des Hinweises auf die Patenterteilung: **30.06.2010**
(21) Anmeldenummer: 04706170.0
(22) Anmeldetag: 29.01.2004
(51) Int. Cl.: C07D 487/04, A61K 31/519, A61P 3/04

(54) **SUBSTITUIERTE HEXAHYDRO-PYRAZINO(1,2-A)PYRIMIDIN-4,7-DIONDERIVATE, VERFAHREN ZU IHRER HERSTELLUNG UND IHRE VERWENDUNG ALS ARZNEIMITTEL**
SUBSTITUTED HEXAHYDROPYRAZINO(1,2-A)PYRIMIDIN-4,7-DION DERIVATIVES, METHOD FOR THE PRODUCTION AND USE THEREOF AS MEDICAMENTS
DERIVES DE HEXAHYDRO-PYRAZINO(1,2-A)PYRIMIDINE-4,7-DIONES SUBSTITUES, PROCEDES DE FABRICATION ET UTILISATION EN TANT QU'AGENTS PHARMACEUTIQUES

(30) Priorität: 13.02.2003 DE 10305885; 24.10.2003 DE 10349672
(43) Veröffentlichungstag der Anmeldung: 30.11.2005
(73) Patentinhaber: Sanofi-Aventis Deutschland GmbH, 65929 Frankfurt am Main (DE)
(72) Erfinder: FLOHR, Stefanie, CH-4153 Reinach (CH); STENGELIN, Siegfried, 65817 Eppstein (DE); GOSSEL, Matthias, 65719 Hofheim (DE); KLABUNDE, Thomas, 65929 Frankfurt (DE); SPOONAMORE, James, Tuscon, AZ 85737 (US); SMRCINA, Martin, Tuscon, AZ 85737 (US)
(86) Internationale Anmeldenummer: PCT/EP2004/000769
(87) Internationale Veröffentlichungsnummer: WO 2004/072076

(56) Entgegenhaltungen:
- WO-A-01/16135
- US-B1- 6 251 902
- MASAKATSU EGUCHI, ET AL.: "Solid-Phase Synthesis and Structural Analysis of Bicyclic beta-Turn Mimetics Incorporating Functionality at the i to i+3 Positions" J. AM. CHEM. SOC., Bd. 121, Nr. 12, 1999, Seiten 12204-12205, XP002279635

## Beschreibung

Die Erfindung betrifft substituierte Hexahydro-pyrazino[1,2-a]pyrimidin-4,7-dionderivate sowie deren physiologisch verträgliche Salze und physiologisch funktionelle Derivate.

Der Erfindung lag die Aufgabe zugrunde, Verbindungen zur Verfügung zu stellen, die eine Gewichtsreduktion bei Säugetieren bewirken und die zur Prävention und Behandlung von Adipositas geeignet sind.

Die Erfindung betrifft daher Verbindungen der Formel I, 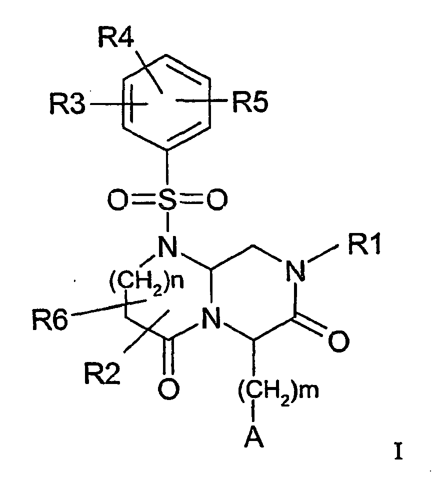 worin bedeuten
- A: 3-12 gliedriger mono-, bi- oder spirobicyclischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Akyl tragen kann;
- R11, R12, R13, R14, R15: unabhängig voneinander H, (C₁-C₆)-Alkyl, Heterocyclus;
- n: 1;
- m: 0, 1, 2, 3, 4, 5, 6;
- R1: R8, (C₁-C₆)-Alkylen-R8, (C₂-C₆)-Alkenylen-R9, (SO₂)-R8, (SO₂)-(C₁-C₆)- Alkylen-R8, (SO₂)-(C₂-C₆)-Alkenylen-R9, (C=O)-R8, (C=O)-(C₁-C₆)-Alkylen- R8, (C=O)NH-R8, (C=O)-(C₂-C₆)-Alkenylen-R9, (C=O)-NH-(C₁-C₆)-Alkylen- R8, (C=O)-NH-(C₂-C₆)-Alkenylen-R9, COO-R8, COO-(C₁-C₆)-Alkylen-R8, COO-(C₂-C₆)-Alkenylen-R9, Alkinylen-R9, (C₁-C₄-Alkyl)-Heterocyclus, wobei die Alkylengruppen mit F substituiert sein könne;
- R8, R9: unabhängig voneinander H, F, Cl, Br, I, OH, CF₃, Aryl, Heterocyclus, (C₃-C₈)- Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)- Alkyl, NH₂, CON(R11)(R12), N(R13)(R14), SO₂-CH₃, COOH, COO-(C₁-C₆)- Alkyl, CONH₂;
- R2: H, F, Cl, Br, J, OH, CF₃, CN, OCF₃, O-(C₁-C₆)-Alkyl, O-(C₁-C₄)-Alkoxy-(C₁- C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)- Alkinyl, Aryl, O-Aryl, (C₁-C₈)-Alkylen Aryl, O-(C₁-C₈)-Alkylen-Aryl, S-Aryl, CON(R11)(R12), (C₁-C₆)-Alkyl-N(R13)(R14), COOH, COO-(C₁- C₆)-Alkyl, COO-(C₂-C₆)-Alkenyl, CO-N((C₁-C₆)-Alkyl)₂, Heterocyclus, wobei der Heterocyclus nicht über ein Stickstoffatom gebunden sein darf;
- R3, R4, R5: unabhängig voneinander H, F, Cl, Br, J, OH, CF₃, NO₂, CN, OCF₃, O-(C₁-C₆)- Alkyl, O-(C₁-C₄)-Alkoxy-(C₁-C₄)-alkyl, S-(C₁-C₆)-Alkyl, (C₁-C₆)-Alkyl, (C₂-C₆)- Alkenyl, (C₃-C₈)-Cycloalkyl, O-(C₃-C₈)-Cycloalkyl, (C₃-C₈)-Cycloalkenyl, O- (C₃-C₈)-Cycloalkenyl, (C₂-C₆)-Alkinyl, Aryl, O-Aryl (C₁-C₈)-Alkylen-Aryl, O- (C₁-C₈)-Alkylen-Aryl, S-Aryl, N((C₁-C₆)-Alkyl)₂, SO₂-CH₃, COOH, COO-(C₁- C₆)-Alkyl, CO-N((C₁-C₆)-Alkyl)₂;
- R6: H, F, Cl, Br, J, OH, CF₃, NO₂, CN, OCF₃, O-(C₁-C₆)-Alkyl, O-(C₁-C₄)-Alkoxy- (C₁-C₄)-alkyl, S-(C₁-C₆)-Alkyl, (C₁-C₆)-Alkyl, (C₂-C₆)-Alkenyl, (C₃-C₈)- Cycloalkyl, O-(C₃-C₈)-Cycloalkyl, (C₃-C₈)-Cycloalkenyl, O-(C₃-C₈)- Cycloalkenyl, (C₂-C₆)-Alkinyl, (C₀-C₈)-Alkylen-Aryl, O-(C₀-C₈)-Alkylen-Aryl, S-Aryl, N((C₁-C₆)-Alkyl)₂, SO₂-CH₃, COOH, COO-(C₁-C₆)-Alkyl, CO-N((C₁- C₆)-Alkyl)₂;
sowie deren physiologisch verträglichen Salze;
mit der Maßgabe, dass die Verbindungen mit
a) R1 = CH₂-Cyclohexyl, R2, R3, R4, R5, R6 = H, m = 1, A = Phenyl und
b) R1 = 4-Cl-Phenyl, R2, R6 = H, m = 1, A = Phenyl, R3, R4, R5 = H oder Methyl, wobei genau ein Rest aus R3, R4, R5 Methyl bedeutet,
ausgenommen sind.

Bevorzugt sind Verbindungen der Formel I worin bedeuten
- A: 3-12 gliedriger mono-, bi- oder spirobicyclischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl tragen kann;
- R11, R12, R13, R14, R15: unabhängig voneinander H, (C₁-C₆)-Alkyl, Heterocyclus;
- m: 1;
- n: 1;
- R1: R8, (C₁-C₆)-Alkylen-R8, (C₂-C₆)-Alkenylen-R9, (SO₂)-R8, (SO₂)-(C₁-C₆)- Alkylen-R8, (SO₂)-(C₂-C₆)-Alkenylen-R9, (C=O)-R8, (C=O)-(C₁-C₆)-Alkylen- R8, (C=O)NH-R8, (C=O)-(C₂-C₆)-Alkenylen-R9, (C=O)-NH- (C₁-C₆)-Alkylen- R8, (C=O)-NH- (C₂-C₆)-Alkenylen-R9, COO-R8, COO-(C₂-C₆)-Alkylen-R8, COO-(C₂-C₆)-Alkenylen-R9, Alkinylen-R9, (C₁-C₄-Alkyl)-Heterocyclus;
- R8, R9: unabhängig voneinander H, F, Cl, Br, I, OH, CF₃, Aryl, Heterocyclus, (C₃-C₈)- Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)- Alkyl, NH₂, CON(R11)(R12), N(R13)(R14), SO₂-CH₃, COOH, COO-(C₁-C₆)- Alkyl, CONH₂;
- R2: H, F, Cl, Br, J, OH, CF₃, CN, OCF₃, O-(C₁-C₆)-Alkyl, O-(C₁-C₄)-Alkoxy-(C₁- C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)- Alkinyl, Aryl, O-Aryl, (C₁-C₈)-Alkylen-Aryl, O-(C₁-C₈)-Alkylen-Aryl, S-Aryl, CON(R11)(R12), (C₁-C₆)-Alkyl-N(R13)(R14), COOH, COO-(C₁- C₆)-Alkyl, COO-(C₂-C₆)-Alkenyl, CO-N((C₁-C₆)-Alkyl)₂, Heterocyclus, wobei der Heterocyclus nicht über ein Stickstoffatom gebunden sein darf;
- R3, R4, R5: unabhängig voneinander H, F, Cl, Br, J, OH, CF₃, NO₂, CN, OCF₃, O-(C₁-C₆)- Alkyl, O-(C₁-C₄)-Alkoxy-(C₁-C₄)-alkyl, S-(C₁-C₆)-Alkyl, (C₁-C₆)-Alkyl, (C₂-C₆)- Alkenyl, (C₃-C₈)-Cycloalkyl, O-(C₃-C₈)-Cycloalkyl, (C₃-C₈)-Cycloalkenyl, O- (C₃-C₈)-Cycloalkenyl, (C₂-C₆)-Alkinyl, Aryl, O-Aryl (C₀-C₈)-Alkylen-Aryl, O- (C₀-C₈)-Alkylen-Aryl, S-Aryl, N((C₁-C₆)-Alkyl)₂, SO₂-CH₃, COOH, COO-(C₁- C₆)-Alkyl, CO-N((C₁-C₆)-Alkyl)₂;
- R6: H, F, Cl, Br, J, OH, CF₃, NO₂, CN, OCF₃, O-(C₁-C₆)-Alkyl, O-(C₁-C₄)-Alkoxy- (C₁-C₄)-alkyl, S-(C₁-C₆)-Alkyl, (C₁-C₆)-Alkyl, (C₂-C₆)-Alkenyl, (C₃-C₈)- Cycloalkyl, O-(C₃-C₈)-Cycloalkyl, (C₃-C₈)-Cycloalkenyl, O-(C₃-C₈)- Cycloalkenyl, (C₂-C₆)-Alkinyl, Aryl, O-Aryl, (C₁-C₈)-Alkylen-Aryl, O-(C₁-C₈)- Alkylen-Aryl, S-Aryl, N((C₁-C₆)-Alkyl)₂, SO₂-CH₃, COOH, COO-(C₁-C₆)-Alkyl, CO-N((C₁-C₆)-Alkyl)₂;
sowie deren physiologisch verträglichen Salze.

Besonders bevorzugt sind Verbindungen der Formel I, worin bedeuten
- A: Aryl, wobei der Arylring substituiert sein kann mit F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl;
- R11, R12, R13, R14, R15: unabhängig voneinander H, (C₁-C₆)-Alkyl;
- m: 1;
- R1: (C₁-C₆)-Alkylen-R8, (C₂-C₆)-Alkenylen-R9;
- R8, R9: unabhängig voneinander H, F, Cl, Br, I, OH, CF₃, Aryl, Heterocyclus, (C₃-C₈)- Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)- Alkyl, NH₂, CON(R11)(R12), N(R13)(R14), SO₂-CH₃, COOH, COO-(C₁-C₆)- Alkyl, CONH₂;
- R2: H, F, Cl, Br, J, OH, CF₃, CN, OCF₃, O-(C₁-C₆)-Alkyl, (C₁-C₆)-Alkyl, COO-(C₂- C₆)-Alkenyl, (C₁-C₆)-Alkyl-N(R13)(R14);
- R3: H;
- R4, R5: unabhängig voneinander H, F, Cl, Br, OH, CF₃, OCF₃, O-(C₁-C₆)-Alkyl, (C₁-C₆)- Alkyl;
- R6: H;
sowie deren physiologisch verträglichen Salze.

Ganz besonders bevorzugt sind Verbindungen der Formel I, worin bedeuten
- A: Aryl, wobei der Arylring substituiert sein kann mit F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl;
- R11, R12, R13, R14, R15: unabhängig voneinander H, (C₁-C₆)-Alkyl;
- m: 1;
- n: 1;
- R1: (C₁-C₆)-Alkyl, (C₂-C₆)-Alkenyl;
- R2: H, OH, (C₁-C₆)-Alkyl, COO-(C₂-C₆)-Alkenyl, (C₁-C₆)-Alkyl-N(R13)(R14);
- R3: H;
- R4: F, Cl, Br, OH, CF₃, OCF₃, O-(C₁-C₆)-Alkyl, (C₁-C₆)-Alkyl;
- R5: H, F, Cl, Br, OH, CF₃, OCF₃, O-(C₁-C₆)-Alkyl, (C₁-C₆)-Alkyl;
- R6: H;
sowie deren physiologisch verträglichen Salze.

Können Reste oder Substituenten mehrfach in den Verbindungen der Formel I auftreten, wie zum Beispiel CON(R11)(R12), so können sie alle unabhängig voneinander die angegebenen Bedeutungen haben und gleich oder verschieden sein.

Die Erfindung bezieht sich auf Verbindungen der Formel I, in Form ihrer Racemate, enantiomerenangereicherten Mischungen und reinen Enantiomere sowie auf ihre Diastereomere und Mischungen davon.

Die Alkyl-, Alkenyl- und Alkinylreste in den Substituenten A, R1, R2, R3, R4, R5, R6, R8, R9, R10, R11, R12, R13, R14, R15 können sowohl geradkettig, verzweigt oder optional halogeniert sein. Die Alkylreste in den Substituenten A, R1, R2, R3, R4, R5, R6, R8, R9, R10, R11, R12, R13, R14, R15 können auch cyclisch sein.

Unter dem Begriff "Aryl" wird eine Phenyl oder Naphthylgruppe verstanden.

Unter Heterocyclus bzw. Heterocyclischer Rest werden Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der Heterocyclische Rest mit Benzolkernen kondensiert ist.

Geeignete "Heterocyclische Ringe" bzw. "Heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Umfasst sind weiterhin ein oder mehrfach benzoannelierte Derivate dieser Heterozyklen.

Die Heterocyclischen Ringe bzw. Heterocyclische Reste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Akyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Akyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl; CONH₂.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat; gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Verbindungen der Formel I, worin bedeuten
- A: 3-12 gliedriger mono-, bi- oder spirobicyclischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl tragen kann;
- R11, R12, R13, R14, R15: unabhängig voneinander H, (C₁-C₆)-Alkyl, Heterocyclus;
- n: 0, 1;
- m: 0, 1, 2, 3, 4, 5, 6;
- R1: R8, (C₁-C₆)-Alkylen-R8, (C₂-C₆)-Alkenylen-R9, (SO₂)-R8, (SO₂)-(C₁-C₆)- Alkylen-R8, (SO₂)-(C₂-C₆)-Alkenylen-R9, (C=O)-R8, (C=O)-(C₁-C₆)-Alkylen- R8, (C=O)NH-R8, (C=O)-(C₂-C₆)-Alkenylen-R9, (C=O)-NH- (C₁-C₆)-Alkylen- R8, (C=O)-NH- (C₂-C₆)-Alkenylen-R9, COO-R8, COO-(C₁-C₆)-Alkylen-R8, COO-(C₂-C₆)-Akenylen-R9, Alkinylen-R9, (C₁-C₄-Alkyl)-Heterocyclus, wobei die Alkylengruppen mit F substituiert sein könne;
- R8, R9: unabhängig voneinander H, F, Cl, Br, I, OH, CF₃, Aryl, Heterocyclus, (C₃-C₈)- Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)- Alkyl, NH₂, CON(R11)(R12), N(R13)(R14), SO₂-CH₃, COOH, COO-(C₁-C₆)- Alkyl, CONH₂;
- R2: H, F, Cl, Br, J, OH, CF₃, CN, OCF₃, O-(C₁-C₆)-Alkyl, O-(C₁-C₄)-Alkoxy-(C₁- C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)- Alkinyl, Aryl, O-Aryl, (C₁-C₈)-Alkylen-Aryl, O-(C₁-C₈)-Alkylen-Aryl, S-Aryl, CON(R11)(R12), (C₁-C₆)-Alkyl-N(R13)(R14), COOH, COO-(C₁- C₆)-Alkyl, COO-(C₂-C₆)-Alkenyl, CO-N((C₁-C₆)-Alkyl)₂, Heterocyclus, wobei der Heterocyclus nicht über ein Stickstoffatom gebunden sein darf;
- R3, R4, R5: unabhängig voneinander H, F, Cl, Br, J, OH, CF₃, NO₂, CN, OCF₃, O-(C₁-C₆)- Alkyl, O-(C₁-C₄)-Alkoxy-(C₁-C₄)-alkyl, S-(C₁-C₆)-Alkyl, (C₁-C₆)-Alkyl, (C₂-C₆)- Alkenyl, (C₃-C₈)-Cycloalkyl, O-(C₃-C₈)-Cycloalkyl, (C₃-C₈)-Cycloalkenyl, O- (C₃-C₈)-Cycloalkenyl, (C₂-C₆)-Alkinyl, Aryl, O-Aryl (C₁-C₈)-Alkylen-Aryl, O- (C₁-C₈)-Alkylen-Aryl, S-Aryl, N((C₁-C₆)-Alkyl)₂, SO₂-CH₃, COOH, COO-(C₁- C₆)-Alkyl, CO-N((C₁-C₆)-Alkyl)₂;
- R6: H, F, Cl, Br, J, OH, CF₃, NO₂, CN, OCF₃, O-(C₁-C₆)-Alkyl, O-(C₁-C₄)-Alkoxy- (C₁-C₄)-alkyl, S-(C₁-C₆)-Alkyl, (C₁-C₆)-Alkyl, (C₂-C₆)-Alkenyl, (C₃-C₈)- Cycloalkyl, O-(C₃-C₈)-Cycloalkyl, (C₃-C₈)-Cycloalkenyl, O-(C₃-C₈)- Cycloalkenyl, (C₂-C₆)-Alkinyl, (C₀-C₈)-Alkylen-Aryl, O-(C₀-C₈)-Alkylen-Aryl, S-Aryl, N((C₁-C₆)-Alkyl)₂, SO₂-CH₃, COOH, COO-(C₁-C₆)-Alkyl, CO-N((C₁- C₆)-Alkyl)₂;
sowie deren physiologisch verträgliche Salze zeichnen sich durch günstige Wirkungen auf den Fettstoffwechsel aus, insbesondere sind sie zur Gewichtsreduktion und nach erfolgter Gewichtsreduktion zum Erhalt eines reduzierten Gewichtes bei Säugetieren und als Anorektika geeignet. Die Verbindungen zeichnen sich sich durch ihre geringe Toxizität und ihre geringen Nebenwirkungen aus.
Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Oewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Adipositas. Die Verbindungen eignen sich weiterhin zur Prophylaxe sowie insbesondere zur Behandlung von Typ II Diabetes, der Arteriosklerose sowie zur Normalisierung des Lipidstoffwechsels und zur Behandlung des Bluthochdrucks. Die Verbindungen wirken als Melanocortin-Rezeptor Agonisten und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus und zur Behandlung von Drogenmissbrauch.
Weiterhin eignen sie sich zur Behandlung von Krebs, Arthritis, Schlafstörungen, Schlaf Apnoe, weiblicher und männlicher Sexualstörungen, Entzündungen, Akne, Pigmentierung der Haut, des metabolischen Syndroms, Störungen des Steroidstoffwechsels, Hautkrankheiten, Psoriasis, Mykosen, neurodegenerativer Krankheiten und Alzheimer-Krankheit.

Bei einem weiteren Aspekt der Erfindung können die Verbindungen der Formel I in Kombination mit einer oder mehreren weiteren pharmakologisch wirksamen Substanzen verabreicht werden, die beispielsweise ausgewählt sind aus Antidiabetika, Antiadiposita, blutdrucksenkenden Wirkstoffen, Lipidsenkern und Wirkstoffen zur Behandlung und/oder Prävention von Komplikationen, die von Diabetes verursacht werden oder mit Diabetes assoziiert sind.

Geeignete Antidiabetika umfassen Insuline, Amylin, GLP-1- und GLP-2-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylharnstoffe, Biguanide, Meglitinide, Oxadiazolidindione, Thiazolidindione, Glukosidase-Inhibitoren, Glukagon-Rezeptor-Antagonisten, GLP-1-Agonisten, Kaliumkanalöffner wie z.B. diejenigen, die in WO 97/26265 und WO 99/03861 von Novo Nordisk A/S offenbart wurden, Insulin-Sensitizer, Aktivatoren der Insulin Rezeptor Kinase, Inhibitoren von Leberenzymen, die an der Stimulation der Glukoneogenese und/oder Glykogenolyse beteiligt sind, z.B. Inhibitoren der Glycogenphosphorylase, Modulatoren der Glukoseaufnahme und Glukoseausscheidung, den Fettstoffwechsel verändernde Verbindungen wie antihyperlipidämische Wirkstoffe und antilipidämische Wirkstoffe, z.B. HMGCoA-Reduktase-Inhibitoren, Inhibitoren des Cholesteroltransports/der Cholesterolaufnahme, Inhibitoren der Gallensäurerückresorption oder Inhibitoren des mikrosomalen Triglycerid-Transfer Proteins (MTP), Verbindungen, die die Nahrungsmitteleinnahme verringern, PPAR- und RXR-Agonisten und Wirkstoffe, die auf den ATP-abhängigen Kaliumkanal der Betazellen wirken.
Bei einer Ausführungsform der Erfindung werden die vorliegenden Verbindungen in Kombination mit Insulin verabreicht.
Bei einer weiteren Ausführungsform werden die vorliegenden Verbindungen in Kombination mit einem Sulphonylharnstoff wie z.B. Tolbutamid, Glibenclamid, Glimepirid, Glipizid, Gliquidon, Glisoxepid, Glibornurid oder Gliclazid verabreicht.
Bei einer anderen Ausführungsform werden die vorliegenden Verbindungen in Kombination mit einem Biguanid wie z.B. Metformin verabreicht.
Bei wieder einer anderen Ausführungsform werden die vorliegenden Verbindungen in Kombination mit einem Meglitinid wie z.B. Repaglinid verabreicht.
Bei noch einer weiteren Ausführungsform werden die vorliegenden Verbindungen in Kombination mit einem Thiazolidindion wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer weiteren Ausführungsform werden die vorliegenden Verbindungen in Kombination mit einem α-Glukosidase-Inhibitor wie z.B. Miglitol oder Acarbose verabreicht.
Bei einer anderen Ausführungsform werden die vorliegenden Verbindungen in Kombination mit einem Wirkstoff verabreicht, der auf den ATP-abhängigen Kaliumkanal der Betazellen wirkt, wie z.B. Tolbutamid, Glibenclamid, Glimepirid, Glipizid, Gliclazid oder Repaglinid. Bei noch einer anderen Ausführungsform werden die vorliegenden Verbindungen in Kombination mit einem antihyperlidämischen Wirkstoff oder einem antilipidämischen Wirkstoff wie z.B. Cholestyramin, Colestipol, Clofibrat, Fenofibrat, Gemfibrozil, Lovastatin, Pravastatin, Simvastatin, Atorvastatin, Cerivastatin, Fluvastatin, Probucol, Ezetimibe oder Dextrothyroxin verabreicht.
Bei einer weiteren Ausführungsform werden die vorliegenden Verbindungen in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin; etc. verabreicht.
Weiterhin können die erfindungsgemäßen Verbindungen in Kombination mit einem oder mehreren Antiadiposita oder appetitregulierenden Wirkstoffen verabreicht werden.
Solche Wirkstoffe können ausgewählt werden aus der Gruppe bestehend aus CART-Agonisten, NPY-Antagonisten, MCH-Antagonisten, Orexin-Antagonisten, H3-Antagonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Serotonin- und Noradrenalin-Wiederaufnahme-Inhibitoren, 5HT-Modulatoren, MAO-Hemmer, Bombesin-Agonisten, Galanin-Antagonisten, Wachstumshormon, Wachstumshormon freisetzende Verbindungen, TRH-Agonisten, Modulatoren der Entkopplungsproteine 2 oder 3, Leptin-Agonisten, Dopamin-Agonisten (Bromcriptin, Doprexin), Lipase/Amylase-Inhibitoren, Antagonisten des Cannabinoid Rezeptors 1, Modulatoren des die Acylierung stimulierende Protein (ASP), PPAR-Modulatoren, RXR-Modulatoren, hCNTF-Mimetika oder TR-β-Agonisten.
Bei einer Ausführungsform der Erfindung ist das Antiadipositum Leptin oder modifiziertes Leptin.
Bei einer anderen Ausführungsform ist das Antiadipositum Dexamphetamin oder Amphetamin. Bei einer anderen Ausführungsform ist das Antiadipositum Fenfluramin oder Dexfenfluramin. Bei noch einer anderen Ausführungsform ist das Antiadipositum Sibutramin oder die mono- und bisdemethylierten Wirkmetabolite von Sibutramin.
Bei einer weiteren Ausführungsform ist das Antiadipositum Orlistat.
Bei einer anderen Ausführungsform ist das Antiadipositum Mazindol, Diethylpropion oder Phentermin.
Weiterhin können die vorliegenden Verbindungen in Kombination mit einem oder mehreren antihypertensiven Wirkstoffen verabreicht werden. Beispiele für antihypertensive Wirkstoffe sind Betablocker wie Alprenolol, Atenol, Timolol, Pindolol, Propranolol und Metoprolol, ACE (Angiotensin Converting Enzym)-Hemmer wie z.B. Benazepril, Captopril, Enalapril, Fosinopril, Lisinopril, Quinapril und Rampril, Calciumkanal-Blocker wie Nifedipin, Felodipin, Nicardipin, Isradipin, Nimodipin, Diltiazem und Verapamil, sowie Alphablocker wie Doxazosin, Urapidil, Prazosin und Terazosin. Weiterhin kann verwiesen werden auf Remington: The Science and Practice of Pharmacy, 19. Auflage, Gennaro, Hrsg., Mack Publishing Co., Easton, PA, 1995.
Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.
Die Wirksamkeit der Verbindungen wurde wie folgt getestet:

### Biologisches Prüfmodell:

Die Prüfung der anorektischen Wirkung erfolgte an weiblichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit den Vehikel-behandelten Kontrolltieren verglichen.

**Tabelle 1: Anorektische Wirkung, gemessen als Reduktion des kumulierten Milchkonsums behandelter im Vergleich zu Kontrolltieren.**

| Beispiel | Orale Dosis [mg/kg] | Anzahl der Tiere / Kumulierter Milchkonsum der behandelten Tiere N / [mL] | Anzahl der Tiere / Kumulierter Milchkonsum der Kontrolltiere N / [mL] | Reduktion des kumulierten Milchkonsums in % der Kontrolle |
|---|---|---|---|---|
| 2 | 50 | 10/4,90 | 10/5,48 | 11 |

Aus der Tabelle ist anzulesen, dass die Verbindungen der Formel I eine gute anorektische Wirkung zeigen und damit sehr gut als Antiadipositum geeignet sind.

Die nachfolgend aufgeführten Beispiele und Herstellungsmethoden dienen zur Erläuterung der Erfindung, ohne diese jedoch einzuschränken.

### Allgemeine Verfahren

Die bei der Synthese verwendeten Ausgangsmaterialien wurden von Chemikalienlieferanten wie Aldrich, Acros, Sigma, Fluka, Nova Biochem, Advanced Chemtech, Bachem, Lancaster und anderen Firmen bezogen.

Bei der Synthese wurden die funktionellen Gruppen der verwendeten Aminosäurederivate zur Vermeidung von Nebenreaktionen bei den Kopplungsschritten durch Schutzgruppen geschützt. Beispiele für geeignete Schutzgruppen und ihre Verwendung sind in The Peptides, supra, 1981 und in Bd. 9, Udenfriend und Meienhofer (Hrsg.) 1987 beschrieben (wird hier durch Bezugnahme aufgenommen).

Zur Herstellung der erfindungsgemäßen Verbindungen kamen allgemeine Methoden der Festphasensynthese zur Anwendung. Solche Methoden werden zum Beispiel von Steward und Young in Solid Phase Peptide Synthesis (Freeman & Co., San Francisco 1969) beschrieben (wird hier durch Bezugnahme aufgenommen).

Wenn nicht anders angegeben, wurden die Verbindungen mit TentaGel HL12019 Resin (Rapp Polymere, Tübingen) synthetisiert. Dieses handelsübliche Polymer enthält einen Bromacetal-Linker. Diese Art der Kopplung kann durch das von Vojkovsky, T. et al., J. Org. Chem. 1998, 63, 3162-3163, und Patek, M., Vortrag bei Combinatorial Chemistry 2000, London, 11. - 14. 7. 2000 (wird hier durch Bezugnahme aufgenommen) beschriebene Verfahren in alle Arten von Hydroxy-TentaGel eingebaut werden.

Im ersten Syntheseschritt (allgemeines Syntheseschema, siehe Schema 1) wurde Amin in DMSO zum Austausch von Brom in der Bromacetalbindung bei einer hohen Temperatur verwendet. An das dabei entstehende sekundäre Amin auf dem Polymer wurde Fmocgeschützte Aminosäure gekoppelt. Die Kopplung erfolgte mittels DIC/HOAt oder HATU/DIEA, gewöhnlich in DMF. Die Kopplung wurde 16 Stunden lang bei Raumtemperatur (RT) oder 4 - 5 Stunden lang bei 55°C durchgeführt. Die Aufhebung des Schutzes der Fmoc-Gruppe erfolgte mit 50 % Piperidin in DMF (5 + 15 Minuten). Zur Bestimmung der Substitution kann die Menge freigesetztes Fmoc aus der Absorbanz der Lösung bei 302 nm nach Aufhebung des Schutzes, dem Volumen der Waschflüssigkeit und dem Gewicht des bei der Synthese eingesetzten Polymers entsprechend der Beschreibung in Krchnak, V. et al., Collect. Czech. Chem. Commun. 53 (1988) 2542 (wird hier durch Bezugnahme aufgenommen) ermittelt werden.

Die freie Aminogruppe der an die Festphase gebundenen Struktur wurde dann mit Fmoc-beta-alanin (oder Fmoc-alpha-aminosäure oder substituierter Beta-Aminosäure) gekoppelt. Die Kopplung erfolgte mit NN-Diisopropylcarbodiimid (DIC) in Anwesenheit von HOBt, gewöhnlich in DMF. Die Vollständigkeit der Kopplung wurde durch den Ninhydrintest überwacht.

Die Aufhebung des Schutzes der Fmoc-Gruppe wurde 5 + 15 Minuten lang mit 50 % Piperidin in DMF durchgeführt. Die Menge freigesetztes Fmoc wurde aus der Absorbanz der Lösung bei 302 nm nach Aufhebung des Schutzes, dem Volumen der Waschflüssigkeit und dem Gewicht des bei der Synthese eingesetzten Polymers ermittelt.
Die freie Aminogruppe der an die Festphase gebundenen Struktur wurde dann mit bis zu 2 Äquivalenten eines geeigneten Sulfonylchlorid/DIEA in DCM oder Acetonitril sulfonyliert.
Die Vollständigkeit der Sulfonylierung wurde durch den Ninhydrintest überwacht.

Nach Abschluß des Zusammenbaus des Vorläufers der linearen Verbindung auf dem Polymer wurde die Feststoffphase nacheinander mit DMF und DCM oder THF gewaschen und im Vakuum getrocknet.

Die zyklative Abspaltung der angestrebten Verbindung erfolgte mit Ameisensäure 18 - 24 Stunden lang bei Raumtemperatur, 6 Stunden lang bei 50°C oder durch Kombination der beiden Bedingungen. Das Polymer wurde abfiltriert und mit DCM oder Ameisensäure gewaschen. Die Waschflüssigkeit wurde in die Ameisensäure-Lösung eingebracht. Die Lösung wurde abgedampft. Der Rückstand wurde in einem Gemisch aus Wasser und Acetonitril gelöst und gefriergetrocknet.

Die getrocknete Verbindung wurde durch HPLC mit einem geeigneten Gradienten von 0,1 % TFA in Wasser und Acetonitril (ACN) gereinigt. Nach dem Auffangen des Peaks, der das angestrebte synthetische Produkt enthält, wurde die Lösung der Verbindung gefriergetrocknet. Zur Bestätigung, daß die richtige Verbindung synthetisiert worden war, wurde die Verbindung einer qualitativen Bestimmung mit Elektrospray-Massenspektrum (LC/MS) und/oder einer NMR-Analyse unterzogen.

Zur Analyse mittels HPLC wurde eine Probe der Verbindung mit dem HPLC-System der Firma Beckman (bestehend aus dem Lösungsmittelzuführsystem 126, dem programmierbaren Detektormodul 166 und dem Autosampler 507e und gesteuert mit Datenstation mit Gold Nouveau-Sofware) mit einer YMC ODS-AM 4,6 x 250 mm-Säule (S-5
(5 µm), YMC, Inc. Wilmington, NC, USA) bei 230 nm analysiert. Bei dieser Einstellung wurde eine Durchlaufgeschwindigkeit von 1ml/min und ein Gradient aus Wasser/0,1 % TFA-Puffer und ACN (HPL-Qualität) als Elutionsmittel verwendet.

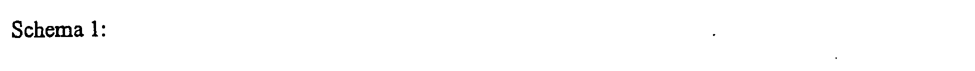

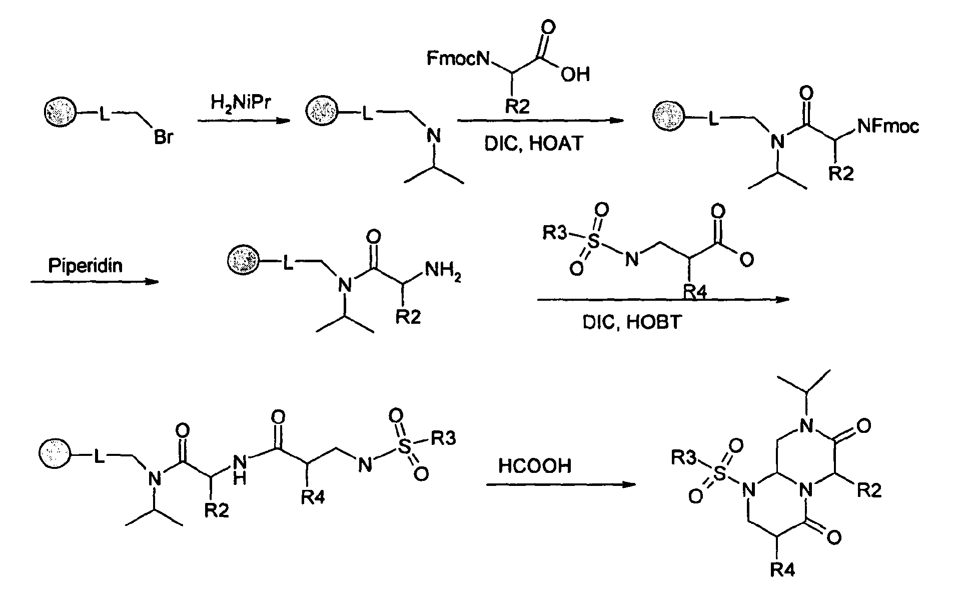

Die Verbindungen können in Analogie zur beschriebenen Synthese am Harz auch in Lösung hergestellt werden. (Schema 2). Anstatt des funktionalisierten Harzes wird in der ersten Stufe 2-Brom-1,1-diethoxy-ethan mit einem primären Amin zur Reaktion gebracht.

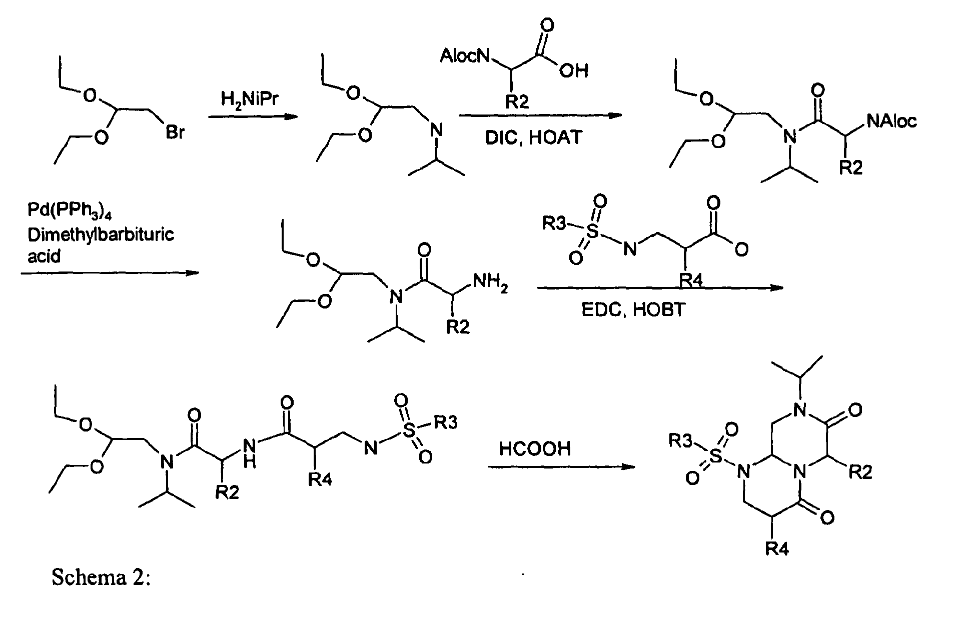

Das erhaltene Produkt wird mit der Aminosäure analog zur Festphasensynthese umgesetzt. Als Aminoschutzgruppe der Aminosäure kann anstatt der FMOC die Allyloxycarbonylschutzgruppe (Aloc) verwendet werden, die nach literaturbekannten Methoden eingeführt (Aloc-Cl, Triethylamin) und abgespalten (Pd(PPh₃)₄, Dimethylbarbitursäure)wird.

Die saure Zyklisierung des so erhaltenen linearen Vorläufers und anschließende weitere Funktionalisierung verläuft analog, wie oben beschrieben.

Zur Reinigung des Produkts wurde eine Probe der gefriergetrockneten Rohsubstanz in einem Gemisch aus 0, 1 %iger wäßriger TFA mit 10 - 50 % Acetonitril oder in Essigsäure gelöst. Die Lösung der Verbindung wurde gewöhnlich durch eine Spritze filtriert, die mit einem 0,45-µm-Filter ACRODISC 13 CR PTFE (Gelman Sciences; Ann Arbor, MI, USA) verbunden war. Ein entsprechendes Volumen der filtrierten Lösung der Verbindung wurde in eine halbpräparative C 18-Säule (YMC ODS-AM, S-5 (5 µm), 20 x 150 mm, YMC, Inc., Wilmington, NC, USA) eingespritzt. Die Durchlaufgeschwindigkeit eines Gradienten aus Wasser/0,1 % TFA-Puffer und ACN (HPL-Qualität) als Elutionsmittel wurde mittels des SYSTEM GOLD HPLC der Firma Beckman (System Gold, programmierbares Lösungsmittelmodul 126 und programmierbares Detektormodul 166, gesteuert mit SYSTEM GOLD-Software) aufrechterhalten. Die Elution der Verbindung wurde mittels UV-Detektion bei 230 oder 280 nm überwacht. Nach Identifizierung des Peaks der zu synthetisierenden Verbindung mit LC/MS wurde die Verbindung aufgefangen, gefriergetrocknet und einer biologischen Prüfung unterzogen.
Nach der Reinigung wurden Verbindungen mit basischen Gruppen als Trifluoracetate gewonnen. Hydrochloride dieser Verbindungen lassen sich durch Behandlung des Trifluoracetats der Verbindung mit einem Überschuß an HCl/Dioxan leicht darstellen. Nach dem Abdampfen der Lösungsmittel wurde das Hydrochlorid der Verbindung mit Diethylether ausgefällt und durch Filtrieren isoliert.

LC/MS erfolgte mit PE Sciex API 150EX und Sciex MassChrom-Software, ausgestattet mit einem Liquid Handler Gilson 215, zwei Flüssigkeitsmodulen Shimadzu LC-10AD, einem Detektor Shimadzu SPD-10A, einer Säule Keystone Betasil C-18 (2 x 30 mm, 3 µm, Durchlaufgeschwindigkeit des Acetonitril-/Wasser-/0,1 %TFA-Gradienten 0,7 ml/min) im ES+-Modus.

Bei der NMR-Analyse wurden die Proben in DMSO-d₆ (Aldrich) mit einem Avance DPX 300 der Firma Bruker gemessen.

### Abkürzungen

Wenn nicht anders angegeben, haben die Abkürzungen in den nachstehenden Beispielen folgende Bedeutung:
ACN = Acetonitril
Aloc = Allyloxycarbonyl
DIC = Diisopropylcarbodiimid
EDC = 1-(3-Dimethylaminopropyl)-3-ethylcarbodiimid
FMOC = 9-Fluorenylmethyloxycarbonyl
DCE = 1,2-Dichlorethan
DIEA = Diisopropylethylamin
NaBH₃CN = Natriumcyanoborhydrid
DMAP = N,N-Dimethylaminopyridin
DMF = N,N-Dimethylformamid
THF = Tetrahydrofuran
DIC = Diisopropylcarbodiimid
DMSO = Dimethylsulfoxid
DCM = Dichlormethan (auch als Methylenchlorid bezeichnet)
HOBt = 1-Hydroxybenzotriazol
HOAt = 1-Hydroxy-7-azabenzotriazol
HATU = Dimethylamino-([1,2,3]triazolo[4,5-b]pyridin-3-yloxy)-methylen-dimethylammoniumhexafluorphosphat
HOAc = Essigsäure
Et3N = Triethylamin
HCl = Salzsäure
HBr = Bromwasserstoffsäure
HPLC = Hochleistungs-Flüssigkeitschromatographie

Die nachfolgenden Beispiele dienen zur näheren Erläuterung der Erfindung, ohne dieselbe auf in den Beispielen beschriebene Produkte und Ausführungsformen einzuschränken.

### Beispiel 1

### 6-(4-Chlor-benzyl)-1-(2,4-dichlor-benzolsulfonyl)-8-isopropyl-hexahydro-pyrazino[1,2-a]pyrimidin-4,7-dion

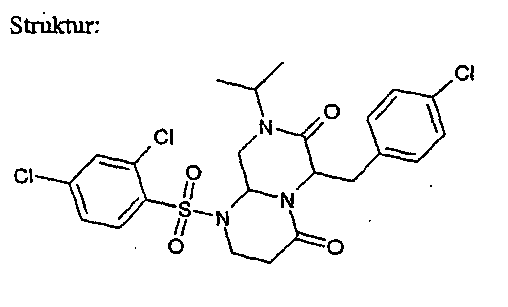

0,5 g TentaGel HL12019 (Bromacetal-Linker, S = 0,5 mMol/g, Rapp Polymere, Tübingen) wurden mit DMSO gewaschen. 20 Äquivalente 2M-Isopropylamin-Lösung (Reagenz 1) in DMSO wurden zugesetzt und das Gemisch wurde 15 Stunden lang bei 60°C in einem geschlossenen Gefäß aufbewahrt. Das Polymer wurde 7mal mit DMF gewaschen. Fmoc-4-chlorphenylalanin (Reagenz 2) (3 Äquivalente) wurde mit HOAt (3 Äquivalente) und DIC (3 Äquivalente) in DMF an das sekundäre Amin auf dem Polymer gekoppelt. Die Endkonzentration betrug 0,2 - 0,3 M. Das Reaktionsgemisch wurde über Nacht bei Raumtemperatur stehengelassen. Das Polymer wurde 6mal mit DMF gewaschen. Die Fmoc-Schutzgruppe wurde mit 50 % Piperidin in DMF abgespalten (5 + 15 Minuten). Fmoc-beta-alanin (3 Äquivalente) wurde dann mit HOBt (3 Äquivalente) und DIC (3 Äquivalente) in DMF (Endkonzentration: 0,2 M) über einen Zeitraum von mindestens 4 Stunden angekoppelt. Die Fmoc-Schutzgruppe wurde mit 50 % Piperidin in DMF abgespalten (5 + 15 Minuten). Das Polymer wurde 5mal mit DMF und 4mal mit DCM gewaschen und mit einer Lösung von 1,5 Äquivalenten 2,4-Dichlorbenzolsulfonylchlorid (Reagenz 3) und 3 Äquivalenten DIEA in Acetonitril (Endkonzentration: 0,1 - 0,15 M) versetzt und bei Raumtemperatur 5 Stunden lang umgesetzt. Anschließend wurde es 5mal mit DMF und 5mal mit DCM gewaschen und im Vakuum getrocknet.

Zur zyklativen Abspaltung wurde das trockene Polymer mit 10 ml Ameisensäure versetzt und bei Raumtemperatur 16 Stunden lang geschüttelt. Das Polymer wurde abfiltriert und mit DCM gewaschen. Die vereinigten Filtrate wurden im Vakuum verdampft. Die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 543,06 (berechnet, monoisotop); Meßwert (M+H)⁺: 544,3.

### Beispiel 2

### 6-(4-Chlor-benzyl)-1-(5-chlor-2-methoxy-benzolsulfonyl)-8-isopropyl-hexahydro-pyrazino[1,2-a]pyrimidin-4,7-dion

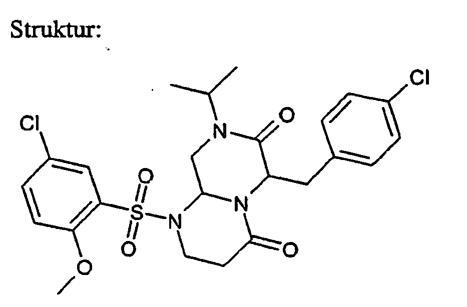

0,3 g TentaGel HL12019 (Bromacetal-Linker, S = 0,5 mMol/g, Rapp Polymere, Tübingen) wurden mit DMSO gewaschen. 20 Äquivalente 2M-Isopropylamin-Lösung (Reagenz 1) in DMSO wurden zugesetzt und das Gemisch wurde 15 Stunden lang bei 60°C in einem geschlossenen Gefäß aufbewahrt. Das Polymer wurde 7mal mit DMF gewaschen. Fmoc-4-chlorphenylalanin (Reagenz 2) (3 Äquivalente) wurde mit HATU (3 Äquivalente) und DIEA (9 Äquivalente) in DMF an das sekundäre Amin auf dem Polymer gekoppelt. Die Endkonzentration betrug 0,2 - 0,3 M. Das Reaktionsgemisch wurde 4 Stunden lang bei 55°C belassen. Das Polymer wurde 6mal mit DMF gewaschen. Die Fmoc-Schutzgruppe wurde mit 50 % Piperidin in DMF abgespalten (5 + 15 Minuten).
Fmoc-beta-alanin (3 Äquivalente) wurde dann mit HOBt (3 Äquivalente) und DIC (3 Äquivalente) in DMF (Endkonzentration: ca. 0,2 M) über einen Zeitraum von mindestens 4 Stunden angekoppelt. Die Fmoc-Schutzgruppe wurde mit 50 % Piperidin in DMF abgespalten (5 + 15 Minuten).
Das Polymer wurde 5mal mit DMF und 4mal mit DCM gewaschen und mit einer Lösung von 1,5 Äquivalenten 2-Methoxy-5-chlorbenzolsulfonylchlorid (Reagenz 3) und 3 Äquivalenten DIEA in Acetonitril (Endkonzentration: 0,1 - 0,15 M) versetzt und bei Raumtemperatur 5 Stunden lang umgesetzt. Anschließend wurde es 5mal mit DMF und 5mal mit THF gewaschen und im Vakuum getrocknet.
Zur zyklativen Abspaltung wurde das trockene Polymer mit 10 ml Ameisensäure versetzt und bei Raumtemperatur 16 Stunden lang geschüttelt. Das Polymer wurde abfiltriert und mit DCM gewaschen. Die vereinigten Filtrate wurden im Vakuum verdampft. Die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 539,10 (berechnet, monoisotop); Meßwert (M+H)⁺: 540,3.

### Beispiel 3

### 6-Benzyl-1-(5-chlor-2-methoxy-benzolsulfonyl)-8-isopropyl-hexahydro-pyrazino[1,2-a]pyrimidin-4,7-dion

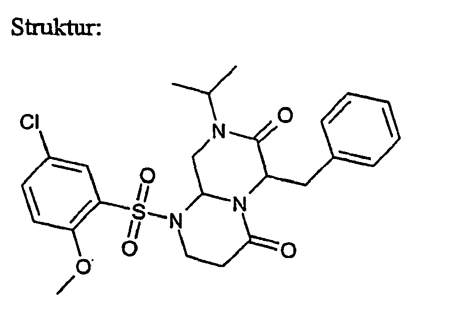

0,3 g TentaGel HL12019 (Bromacetal-Linker, S = 0,5 mMol/g, Rapp Polymere, Tübingen) wurden mit DMSO gewaschen. 20 Äquivalente 2M-Isopropylamin-Lösung in DMSO wurden zugesetzt und das Gemisch wurde 15 Stunden lang bei 60°C in einem geschlossenen Gefäß aufbewahrt. Das Polymer wurde 7mal mit DMF gewaschen.
Fmoc-phenylalanin (3 Äquivalente) wurde mit HATU (3 Äquivalente) und DIEA (9 Äquivalente) in DMF an das sekundäre Amin auf dem Polymer gekoppelt. Die Endkonzentration betrug 0,2 - 0,3 M. Das Reaktionsgemisch wurde 4 Stunden lang bei 55°C belassen. Das Polymer wurde 6mal mit DMF gewaschen. Die Fmoc-Schutzgruppe wurde mit 50 % Piperidin in DMF abgespalten (5 + 15 Minuten).
Fmoc-beta-alanin (3 Äquivalente) wurde dann mit HOBt (3 Äquivalente) und DIC (3 Äquivalente) in DMF (Endkonzentration: ca. 0,2 M) über einen Zeitraum von mindestens 4 Stunden angekoppelt. Die Fmoc-Schutzgruppe wurde mit 50 % Piperidin in DMF abgespalten (5 + 15 Minuten).
Das Polymer wurde 5mal mit DMF und 4mal mit DCM gewaschen und mit einer Lösung von 1,5 Äquivalenten 2-Methoxy-5-chlorbenzolsulfonylchlorid und 3 Äquivalenten DIEA in Acetonitril (Endkonzentration: 0,1 - 0,15 M) versetzt und bei Raumtemperatur 5 Stunden lang umgesetzt. Anschließend wurde es 5mal mit DMF und 5mal mit THF gewaschen und im Vakuum getrocknet.
Zur zyklativen Abspaltung wurde das trockene Polymer mit 10 ml Ameisensäure versetzt und bei Raumtemperatur 16 Stunden lang geschüttelt. Das Polymer wurde abfiltriert und mit DCM gewaschen. Die vereinigten Filtrate wurden im Vakuum verdampft. Die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 545,14 (berechnet, monoisotop); Meßwert (M+H)⁺: 506,3.

### Beispiel 4

### 1-(5-Chlor-2-methoxy-benzolsulfonyl)-6-(3,4-dichlor-benzyl)-8-isopropyl-hexahydro-pyrazino[1,2-a]pyrimidin-4,7-dion

### Struktur:

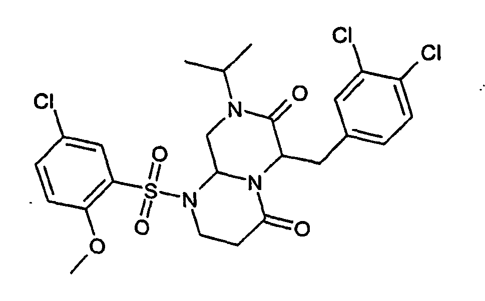

0,3 g TentaGel HL12019 (Bromacetal-Linker, S = 0,5 mMol/g, Rapp Polymere, Tübingen) wurden mit DMSO gewaschen. 20 Äquivalente 2M-Isopropylamin-Lösung in DMSO wurden zugesetzt und das Gemisch wurde 15 Stunden lang bei 60°C in einem geschlossenen Gefäß aufbewahrt. Das Polymer wurde 7mal mit DMF gewaschen.
Fmoc-3,4-dichlorphenylalanin (3 Äquivalente) wurde mit HATU (3 Äquivalente) und DIEA (9 Äquivalente) in DMF an das sekundäre Amin auf dem Polymer gekoppelt. Die Endkonzentration betrug 0,2 - 0,3 M. Das Reaktionsgemisch wurde 4 Stunden lang bei 55°C belassen. Das Polymer wurde 6mal mit DMF gewaschen. Die Fmoc-Schutzgruppe wurde mit 50 % Piperidin in DMF abgespalten (5 + 15 Minuten).
Fmoc-beta-alanin (3 Äquivalente) wurde dann mit HOBt (3 Äquivalente) und DIC (3 Äquivalente) in DMF (Endkonzentration: ca. 0,2 M) über einen Zeitraum von mindestens 4 Stunden angekoppelt. Die Fmoc-Schutzgruppe wurde mit 50 % Piperidin in DMF abgespalten (5 + 15 Minuten).
Das Polymer wurde 5mal mit DMF und 4mal mit DCM gewaschen und mit einer Lösung von 1,5 Äquivalenten 2-Methoxy-5-chlorbenzolsulfonylchlorid, 3 Äquivalenten DIEA in Acetonitril (Endkonzentration: 0,1 - 0,15 M) versetzt und bei Raumtemperatur 5 Stunden lang umgesetzt. Anschließend wurde es 5mal mit DMF und 5mal mit THF gewaschen und im Vakuum getrocknet.
Zur zyklativen Abspaltung wurde das trockene Polymer mit 10 ml Ameisensäure versetzt und bei Raumtemperatur 16 Stunden lang geschüttelt. Das Polymer wurde abfiltriert und mit DCM gewaschen. Die vereinigten Filtrate wurden im Vakuum verdampft. Die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 573,07 (berechnet, monoisotop); Meßwert (M+H)⁺: 574,3.

### Beispiel 5

### 8-Allyl-1-(naphthalene-2-sulfonyl)-6-(4-nitro-benzyl)-hexahydro-pyrazino[1,2- a]pyrimidin-4,7-dion

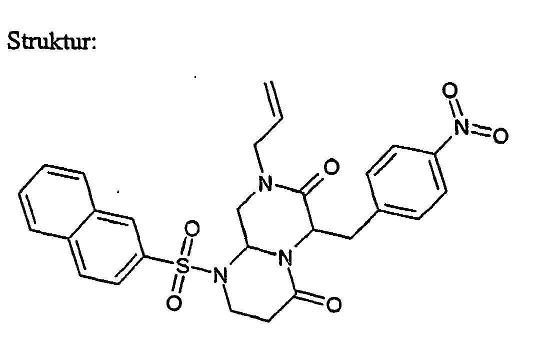

0,3 g TentaGel HL12019 (Bromacetal-Linker, S = 0,5 mMol/g, Rapp Polymere, Tübingen) wurden mit DMSO gewaschen. 20 Äquivalente 2M-Allylamin-Lösung in DMSO wurden zugesetzt und das Gemisch wurde 15 Stunden lang bei 60°C in einem geschlossenen Gefäß aufbewahrt. Das Polymer wurde 7mal mit DMF gewaschen.
Fmoc-4-nitrophenylalanin (3 Äquivalente) wurde mit HATU (3 Äquivalente) und DIEA (9 Äquivalente) in DMF an das sekundäre Amin auf dem Polymer gekoppelt. Die Endkonzentration betrug 0,2 - 0,3 M. Das Reaktionsgemisch wurde 4 Stunden lang bei 55°C belassen. Das Polymer wurde 6mal mit DMF gewaschen. Die Fmoc-Schutzgruppe wurde mit 50 % Piperidin in DMF abgespalten (5 + 15 Minuten).
Fmoc-beta-alanin (3 Äquivalente) wurde dann mit HOBt (3 Äquivalente) und DIC (3 Äquivalente) in DMF (Endkonzentration: ca. 0,2 M) über einen Zeitraum von mindestens 4 Stunden angekoppelt. Die Fmoc-Schutzgruppe wurde mit 50 % Piperidin in DMF abgespalten (5 + 15 Minuten).
Das Polymer wurde 5mal mit DMF und 4mal mit DCM gewaschen und mit einer Lösung von 1,5 Äquivalenten 2-Naphthylsulfonylchlorid und 3 Äquivalenten DIEA in Acetonitril (Endkonzentration: 0,1 - 0,15 M) versetzt und bei Raumtemperatur 5 Stunden lang umgesetzt. Anschließend wurde es 5mal mit DMF und 5mal mit THF gewaschen und im Vakuum getrocknet.
Zur zyklativen Abspaltung wurde das trockene Polymer mit 10 ml Ameisensäure versetzt und bei Raumtemperatur 16 Stunden lang geschüttelt. Das Polymer wurde abfiltriert und mit DCM gewaschen. Die vereinigten Filtrate wurden im Vakuum verdampft. Die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 534,16 (berechnet, monoisotop); Meßwert (M+H)⁺: 535,3.

### Beispiel 6

### 6-(4-Chlor-benzyl)-1-(5-chlor-2-methoxy-benzolsulfonyl)-3-hydroxy-8-isopropyl- hexahydro-pyrazino[1,2-a]pyrimidin-4,7-dion

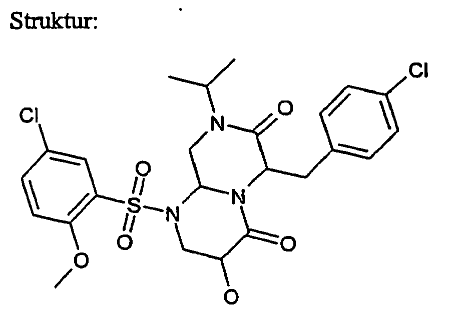

0,5 g TentaGel HL12019 (Bromacetal-Linker, S = 0,5 mMol/g, Rapp Polymere, Tübingen) wurden mit DMSO gewaschen. 20 Äquivalente 2M-Isopropylamin-Lösung in DMSO wurden zugesetzt und das Gemisch wurde 15 Stunden lang bei 60°C in einem geschlossenen Gefäß aufbewahrt. Das Polymer wurde 7mal mit DMF gewaschen.
Fmoc-4-chlorphenylalanin (3 Äquivalente) wurde mit HOAt (3 Äquivalente) und DIC (3 Äquivalente) in DMF an das sekundäre Amin auf dem Polymer gekoppelt. Die Endkonzentration betrug 0,2 - 0,3 M. Das Reaktionsgemisch wurde über Nacht bei Raumtemperatur stehengelassen. Das Polymer wurde 6mal mit DMF gewaschen. Die Fmoc-Schutzgruppe wurde mit 50 % Piperidin in DMF abgespalten (5 + 15 Minuten).
Fmoc-isoserin (3 Äquivalente) wurde dann mit HOBt (3 Äquivalente) und DIC (3 Äquivalente) in DMF (Endkonzentration: ca. 0,2 M) über einen Zeitraum von mindestens 4 Stunden angekoppelt. Die Fmoc-Schutzgruppe wurde mit 50 % Piperidin in DMF abgespalten (5 + 15 Minuten).
Das Polymer wurde 5mal mit DMF und 4mal mit DCM gewaschen und mit einer Lösung von 1,5 Äquivalenten 2-Methoxy-5-chlorbenzolsulfonylchlorid und 3 Äquivalenten DIEA in DCM (Endkonzentration: 0,1 - 0,15 M) versetzt und bei Raumtemperatur 5 Stunden lang umgesetzt. Anschließend wurde es 5mal mit DMF und 5mal mit DCM gewaschen und im Vakuum getrocknet.
Zur zyklativen Abspaltung wurde das trockene Polymer mit 10 ml Ameisensäure versetzt und bei Raumtemperatur 16 Stunden lang geschüttelt. Das Polymer wurde abfiltriert und mit DCM gewaschen. Die vereinigten Filtrate wurden im Vakuum verdampft. Die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 555,10 (berechnet, monoisotop); Meßwert (M+H)⁺: 556,3.

### Beispiel 7

### 5-(4-Chlor-benzyl)-1-(5-chlor-2-methoxy-benzolsulfonyl)-7-isopropyl-tetrahydro- imidazo[1,2-a]pyrazin-3,6-dion

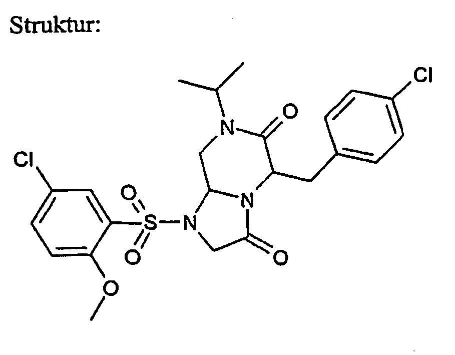

0,3 g TentaGel HL12019 (Bromacetal-Linker, S = 0,5 mMol/g, Rapp Polymere, Tübingen) wurden mit DMSO gewaschen. 20 Äquivalente 2M-Isopropylamin-Lösung in DMSO wurden zugesetzt und das Gemisch wurde 15 Stunden lang bei 60°C in einem geschlossenen Gefäß aufbewahrt. Das Polymer wurde 7mal mit DMF gewaschen.
Fmoc-4-chlorphenylalanin (3 Äquivalente) wurde mit HOAt (3 Äquivalente) und DIC (3 Äquivalente) in DMF an das sekundäre Amin auf dem Polymer gekoppelt. Die Endkonzentration betrug 0,2 - 0,3 M. Das Reaktionsgemisch wurde über Nacht bei Raumtemperatur stehengelassen. Das Polymer wurde 6mal mit DMF gewaschen. Die Fmoc-Schutzgruppe wurde mit 50 % Piperidin in DMF abgespalten (5 + 15 Minuten).
Fmoc-glycin (3 Äquivalente) wurde dann mit HOBt (3 Äquivalente) und DIC (3 Äquivalente) in DMF (Endkonzentration: ca. 0,2 M) über einen Zeitraum von mindestens 4 Stunden angekoppelt. Die Fmoc-Schutzgruppe wurde mit 50 % Piperidin in DMF abgespalten (5 + 15 Minuten).
Das Polymer wurde 5mal mit DMF und 4mal mit Acetonitril gewaschen und mit einer Lösung von 1,5 Äquivalenten 2-Methoxy-5-chlorbenzolsulfonylchlorid und 3 Äquivalenten DIEA in Acetonitril (Endkonzentration: 0,1 - 0,15 M) versetzt und bei Raumtemperatur 5 Stunden lang umgesetzt. Anschließend wurde es 5mal mit DMF und 5mal mit DCM gewaschen und im Vakuum getrocknet.
Zur zyklativen Abspaltung wurde das trockene Polymer mit 10 ml Ameisensäure versetzt und bei Raumtemperatur 24 Stunden lang geschüttelt. Das Polymer wurde abfiltriert und mit DCM gewaschen. Die vereinigten Filtrate wurden im Vakuum verdampft. Die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 525,09 (berechnet, monoisotop); Meßwert (M+H)⁺: 526,3.

### Beispiel 8

### 5-(4-Chlor-benzyl)-1-(5-chlor-2-methoxy-benzolsulfonyl)-7-isopropyl-2-methyl- tetrahydroimidazo[1,2-a]pyrazin-3,6-dion

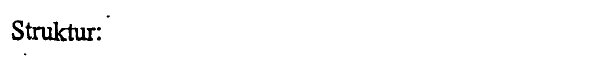

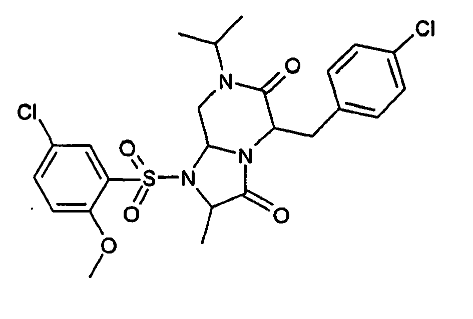

0,3 g TentaGel HL12019 (Bromacetal-Linker, S = 0,5 mMol/g, Rapp Polymere, Tübingen) wurden mit DMSO gewaschen. 20 Äquivalente 2M-Isopropylamin-Lösung in DMSO wurden zugesetzt und das Gemisch wurde 15 Stunden lang bei 60°C in einem geschlossenen Gefäß aufbewahrt. Das Polymer wurde 7mal mit DMF gewaschen.
Fmoc-4-chlorphenylalanin (3 Äquivalente) wurde mit HOAt (3 Äquivalente) und DIC (3 Äquivalente) in DMF an das sekundäre Amin auf dem Polymer gekoppelt. Die Endkonzentration betrug 0,2 - 0,3 M. Das Reaktionsgemisch wurde über Nacht bei Raumtemperatur stehengelassen. Das Polymer wurde 6mal mit DMF gewaschen. Die Fmoc-Schutzgruppe wurde mit 50 % Piperidin in DMF abgespalten (5 + 15 Minuten). Fmoc-(R)-alanin (3 Äquivalente) wurde dann mit HOBt (3 Äquivalente) und DIC (3 Äquivalente) in DMF (Endkonzentration: ca. 0,2 M) über einen Zeitraum von mindestens 4 Stunden angekoppelt. Die Fmoc-Schutzgruppe wurde mit 50 % Piperidin in DMF abgespalten (5 + 15 Minuten).
Das Polymer wurde 5mal mit DMF und 4mal mit Acetonitril gewaschen und mit einer Lösung von 1,5 Äquivalenten 2-Methoxy-5-chlorbenzolsulfonylchlorid und 3 Äquivalenten DIEA in Acetonitril (Endkonzentration: 0,1 - 0,15 M) versetzt und bei Raumtemperatur 5 Stunden lang umgesetzt. Anschließend wurde es 5mal mit DMF und 5mal mit DCM gewaschen und im Vakuum getrocknet.
Zur zyklativen Abspaltung wurde das trockene Polymer mit 10 ml Ameisensäure versetzt und bei Raumtemperatur 24 Stunden lang geschüttelt. Das Polymer wurde abfiltriert und mit DCM gewaschen. Die vereinigten Filtrate wurden im Vakuum verdampft. Die Rohsubstanz wurde in einem Gemisch aus Acetonitril und Wasser gelöst und gefriergetrocknet. Die reine Titelverbindung wurde nach Reinigung mittels HPLC abgetrennt. Dabei kam das unter "Allgemeine Verfahren" beschriebene System und Verfahren zur Anwendung. MG = 539,10 (berechnet, monoisotop); Meßwert (M+H)⁺: 540,3.

### Beispiel 9

### 1-(5-Chlor-2-methoxy-benzolsulfonyl)-6-cyclohexylmethyl-8-isopropyl-hexahydro-pyrazino[1,2-a]pyrimidin-4,7-dion

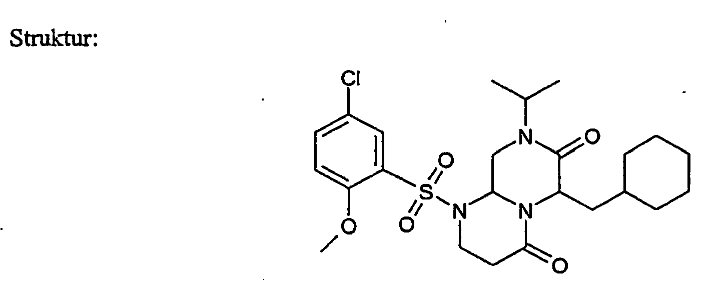

Die Verbindung in Beispiel 9 wurde nach dem in Beispiel 2 beschriebenen Verfahren unter Verwendung folgender Reagenzien synthetisiert:
Reagenz 1: Isopropylamin
Reagenz 2: Fmoc-cyclohexylalanin
Reagenz 3: 2-Methoxy-5-chlorbenzol-sulfonylchlorid
MG = 511,19 (berechnet, monoisotop); Meßwert (M+H)⁺: 512,3.

### Beispiel 10

### 1-(5-Chlor-2-methoxy-benzolsulfonyl)-6-cyclohexyl-8-isopropyl-hexahydro-pyrazino[1,2-a]pyrimidin-4,7-dion

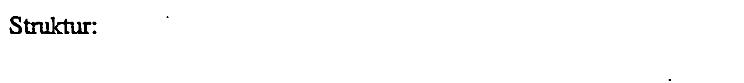

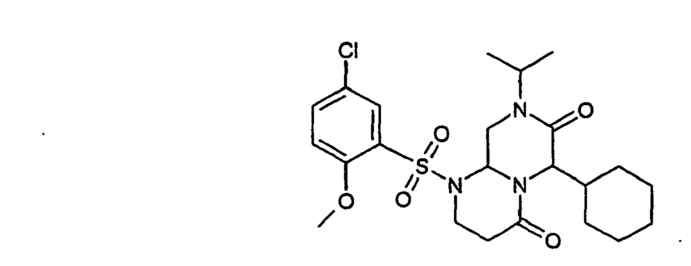

Die Verbindung in Beispiel 10 wurde nach dem in Beispiel 2 beschriebenen Verfahren unter Verwendung folgender Reagenzien synthetisiert:
Reagenz 1: Isopropylamin
Reagenz 2: Fmoc-cyclohexylglycin
Reagenz 3: 2-Methoxy-5-chlorbenzol-sulfonylchlorid
MG = 497,18 (berechnet, monoisotop); Meßwert (M+H)⁺: 498,3.

### Beispiel 11

### 1-(5-Chlor-2-methoxy-benzolsulfonyl)-8-isopropyl-6-phenethyl-hexahydro-pyrazino[1,2-a]pyrimidin-4,7-dion

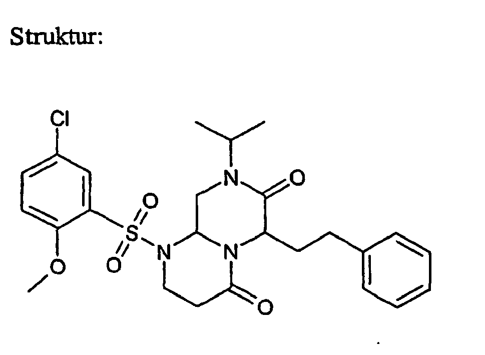

Die Verbindung in Beispiel 11 wurde nach dem in Beispiel 2 beschriebenen Verfahren unter Verwendung folgender Reagenzien synthetisiert:
Reagenz 1: Isopropylamin
Reagenz 2: Fmoc-homophenylalanin
Reagenz 3: 2-Methoxy-5-chlorbenzol-sulfonylchlorid
MG = 519,18 (berechnet, monoisotop); Meßwert (M+H)⁺: 520,3.

### Beispiel 12

### 1-(5-Chlor-2-methoxy-benzolsulfonyl)-6-indan-1-yl-8-isopropyl-hexahydro-pyrazino[1,2-a]pyrimidin-4,7-dion

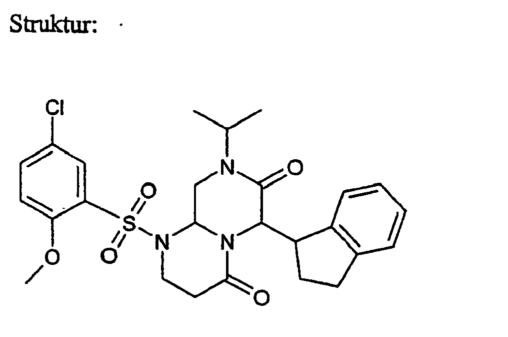

Die Verbindung in Beispiel 12 wurde nach dem in Beispiel 2 beschriebenen Verfahren unter Verwendung folgender Reagenzien synthetisiert:
Reagenz 1: Isopropylamin
Reagenz 2: Fmoc-indanylglycin
Reagenz 3: 2-Methoxy-5-chlorbenzol-sulfonylchlorid
MG = 531,16 (berechnet, monoisotop); Meßwert (M+H)⁺: 532,3.

### Beispiel 13

### 1-(5-Chlor-2-methoxy-benzolsulfonyl)-6-[2-(4-hydroxy-phenyl)-ethyl]-8-isopropyl-hexahydro-pyrazino[1,2-a]pyrimidin-4,7-dion

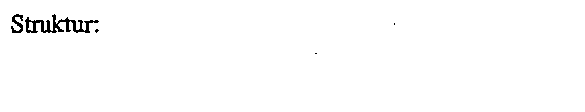

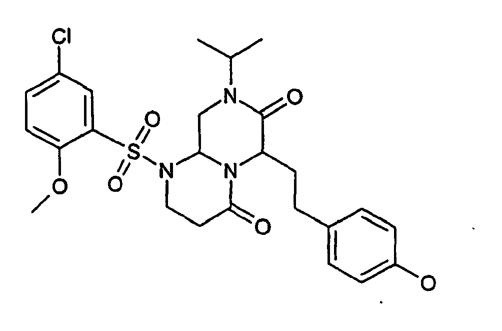

Die Verbindung in Beispiel 13 wurde nach dem in Beispiel 2 beschriebenen Verfahren unter Verwendung folgender Reagenzien synthetisiert:
Reagenz 1: Isopropylamin
Reagenz 2: Fmoc-homotyrosin
Reagenz 3: 2-Methoxy-5-chlorbenzol-sulfonylchlorid
MG = 535,15 (berechnet, monoisotop); Meßwert (M+H)⁺: 536,3.

### Beispiel 14

### 8-Isopropyl-6-(4-methoxy-benzyl)-1-(2-methoxy-5-methyl-benzolsulfonyl)-hexahydro-pyrazino[1,2-a]pyrimidin-4,7-dion

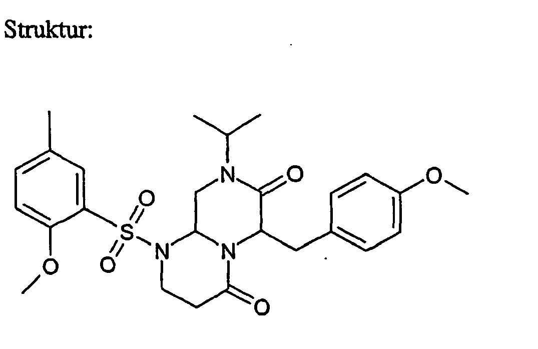

Die Verbindung in Beispiel 14 wurde nach dem in Beispiel 2 beschriebenen Verfahren unter Verwendung folgender Reagenzien synthetisiert:
Reagenz 1: Isopropylamin
Reagenz 2: Fmoc-O-methyltyrosin
Reagenz 3: 2-Methoxy-5-methylbenzol-sulfonylchlorid
MG = 515,21 (berechnet, monoisotop); Meßwert (M+H)⁺: 516,3.

### Beispiel 15

### 6-(4-Fluor-benzyl)-8-isopropyl-1-(2-methoxy-5-methyl-benzolsulfonyl)-hexahydro-pyrazino[1,2-a]pyrimidin-4,7-dion

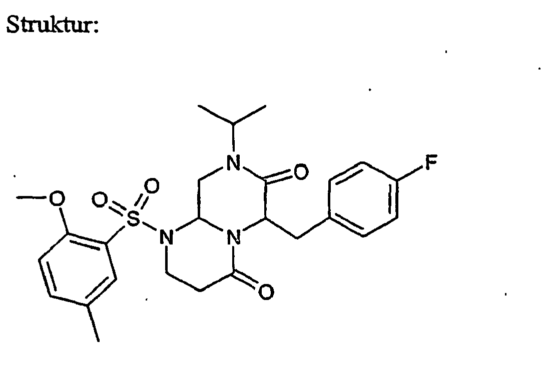

Die Verbindung in Beispiel 15 wurde nach dem in Beispiel 2 beschriebenen Verfahren unter Verwendung folgender Reagenzien synthetisiert:
Reagenz 1: Isopropylamin
Reagenz 2: Fmoc-4-fluorphenylalanin
Reagenz 3: 2-Methoxy-5-methylbenzol-sulfonylchlorid
MG = 503,19 (berechnet, monoisotop); Meßwert (M+H)⁺: 504,3.

### Beispiel 16

### 8-Isopropyl-1-(2-methoxy-5-methyl-benzolsulfonyl)-6-(4-methyl-benzyl)-hexahydro-pyrazino[1,2-a]pyrimidin-4,7-dion

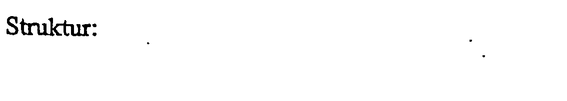

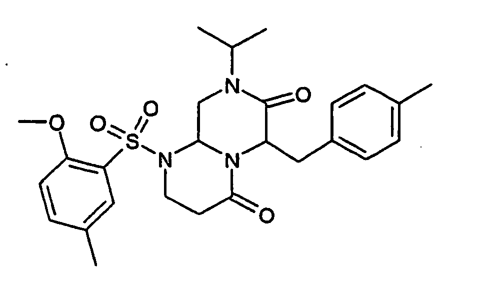

Die Verbindung in Beispiel 16 wurde nach dem in Beispiel 2 beschriebenen Verfahren unter Verwendung folgender Reagenzien synthetisiert:
Reagenz 1: Isopropylamin
Reagenz 2: Fmoc-4-methylphenylalanin
Reagenz 3: 2-Methoxy-5-methylbenzol-sulfonylchlorid
MG = 499,21 (berechnet, monoisotop); Meßwert (M+H)⁺: 500,3.

### Beispiel 17

### 6-(4-Brom-benzyl)-1-(5-chlor-2-methoxy-benzolsulfonyl)-8-isopropyl-hexahydro-pyrazino[1,2-a]pyrimidin-4,7-dion

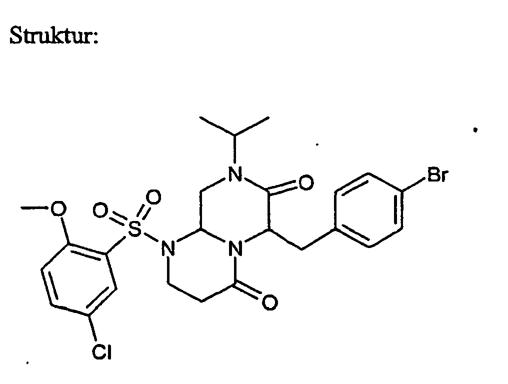

Die Verbindung in Beispiel 17 wurde nach dem in Beispiel 2 beschriebenen Verfahren unter Verwendung folgender Reagenzien synthetisiert:
Reagenz 1: Isopropylamin
Reagenz 2: Fmoc-4-bromphenylalanin
Reagenz 3: 2-Methoxy-5-chlorbenzol-sulfonylchlorid
MG = 583,05 (berechnet, monoisotop); Meßwert (M+H)⁺: 584,3.

### Beispiel 18

### 1-(5-Chlor-2-methoxy-benzolsulfonyl)-6-(4-fluor-benzyl)-8-isopropyl-hexahydro-pyrazino[1,2-a]pyrimidin-4,7-dion

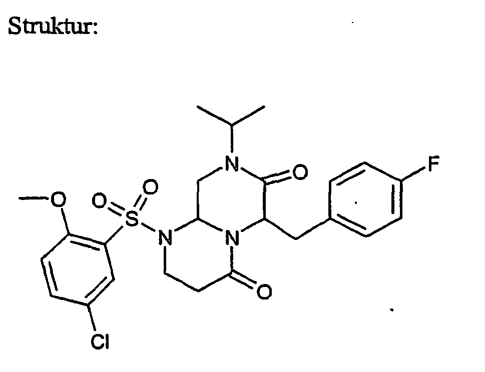

Die Verbindung in Beispiel 18 wurde nach dem in Beispiel 2 beschriebenen Verfahren unter Verwendung folgender Reagenzien synthetisiert:
Reagenz 1: Isopropylamin
Reagenz 2: Fmoc-4-fluorphenylalanin
Reagenz 3: 2-Methoxy-5-chlorbenzol-sulfonylchlorid
MG = 523,13 (berechnet, monoisotop); Meßwert (M+H)⁺: 524,3.

### Beispiel 19

### 1-(5-Chlor-2-methoxy-benzolsulfonyl)-8-isopropyl-6-(4-methyl-benzyl)-hexahydro-pyrazino[1,2-a]pyrimidin-4,7-dion

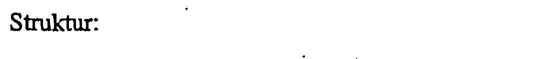

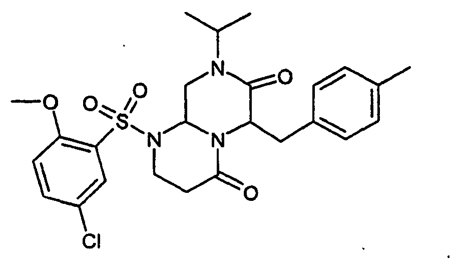

Die Verbindung in Beispiel 19 wurde nach dem in Beispiel 2 beschriebenen Verfahren unter Verwendung folgender Reagenzien synthetisiert:
Reagenz 1: Isopropylamin
Reagenz 2: Fmoc-4-methylphenylalanin
Reagenz 3: 2-Methoxy-5-chlorbenzol-sulfonylchlorid
MG = 519,16 (berechnet, monoisotop); Meßwert (M+H)⁺: 520,3.

### Beispiel 20

### 1-(4-Brom-2-ethyl-benzolsulfonyl)-8-isopropyl-6-(4-methoxy-benzyl)-hexahydro-pyrazino[1,2-a]pyrimidin-4,7-dion

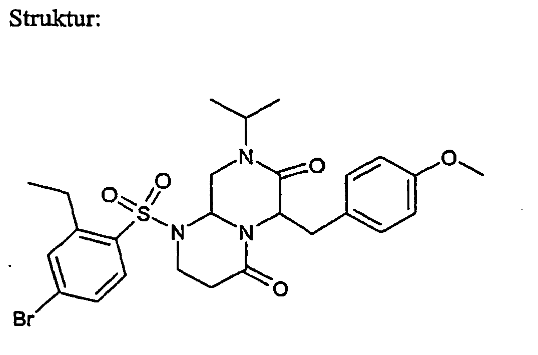

Die Verbindung in Beispiel 20 wurde nach dem in Beispiel 2 beschriebenen Verfahren unter Verwendung folgender Reagenzien synthetisiert:
Reagenz 1: Isopropylamin
Reagenz 2: Fmoc-O-methyltyrosin
Reagenz 3: 2-Ethyl-4-brombenzol-sulfonylchlorid
MG = 577,12 (berechnet, monoisotop); Meßwert (M+H)⁺: 578,3.

### Beispiel 21

### 6-(4-Brom-benzyl)-1-(4-brom-2-ethyl-benzolsulfonyl)-8-isopropyl-hexahydro-pyrazino[1,2-a]pyrimidin-4,7-dion

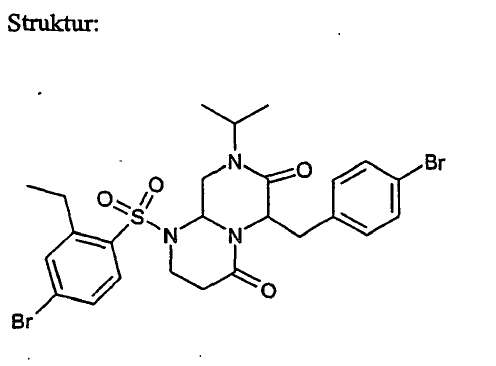

Die Verbindung in Beispiel 21 wurde nach dem in Beispiel 2 beschriebenen Verfahren unter Verwendung folgender Reagenzien synthetisiert:
Reagenz 1: Isopropylamin
Reagenz 2: Fmoc-4-bromphenylalanin
Reagenz 3: 2-Ethyl-4-brombenzol-sulfonylchlorid
MG = 625,02 (berechnet, monoisotop); Meßwert (M+H)⁺: 626,4.

### Beispiel 22

### 1-(4-Brom-2-ethyl-benzolsulfonyl)-6-(4-fluor-benzyl)-8-isopropyl-hexahydro- pyrazino[1,2-a]pyrimidin-4,7-dion

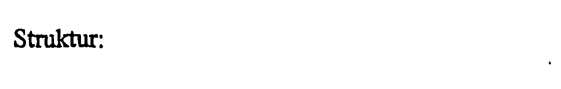

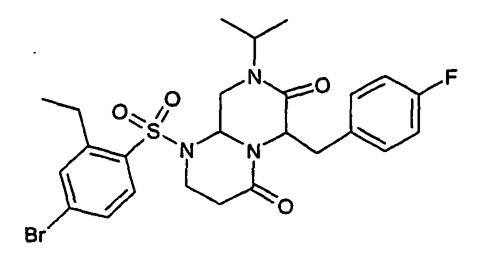

Die Verbindung in Beispiel 22 wurde nach dem in Beispiel 2 beschriebenen Verfahren unter Verwendung folgender Reagenzien synthetisiert:
Reagenz 1: Isopropylamin
Reagenz 2: Fmoc-4-fluorphenylalanin
Reagenz 3: 2-Ethyl-4-brombenzol-sulfonylchlorid
MG = 565,10 (berechnet, monoisotop); Meßwert (M+H)⁺: 566,3.

### Beispiel 23

### 1-(4-Brom-2-ethyl-benzolsulfonyl)-8-isopropyl-6-(4-methyl-benzyl)-hexahydro- pyrazino[1,2-a]pyrimidin-4,7-dion

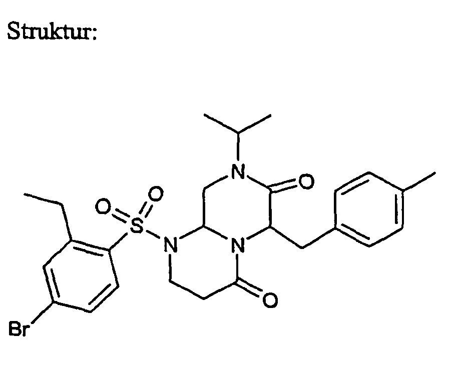

Die Verbindung in Beispiel 23 wurde nach dem in Beispiel 2 beschriebenen Verfahren unter Verwendung folgender Reagenzien synthetisiert:
Reagenz 1: Isopropylamin
Reagenz 2: Fmoc-4-methylphenylalanin
Reagenz 3: 2-Ethyl-4-brombenzol-sulfonylchlorid
MG = 561,13 (berechnet, monoisotop); Meßwert (M+H)⁺: 562,3.

### Beispiel 24

### 8-Isopropyl-6-(4-methoxy-benzyl)-1-(3-trifluormethyl-benzolsulfonyl)-hexahydro-pyrazino[1,2-a]pyrimidin-4,7-dion

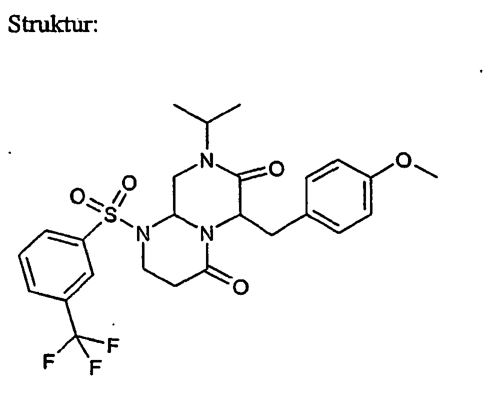

Die Verbindung in Beispiel 24 wurde nach dem in Beispiel 2 beschriebenen Verfahren unter Verwendung folgender Reagenzien synthetisiert:
Reagenz 1: Isopropylamin
Reagenz 2: Fmoc-O-methyltyrosin
Reagenz 3: 3-Trifluormethylbenzol-sulfonylchlorid
MG = 539,17 (berechnet, monoisotop); Meßwert (M+H)⁺: 540,3.

### Beispiel 25

### 6-(4-Brom-benzyl)-8-isopropyl-1-(3-trifluormethyl-benzolsulfonyl)-hexahydro- pyrazino[1,2-a]pyrimidin-4,7-dion

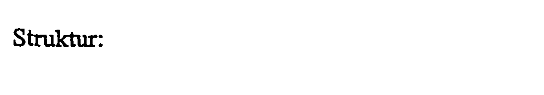

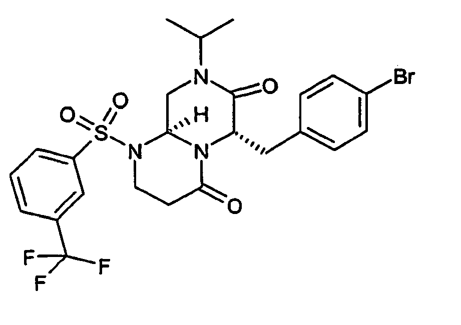

Die Verbindung in Beispiel 25 wurde nach dem in Beispiel 2 beschriebenen Verfahren unter Verwendung folgender Reagenzien synthetisiert:
Reagenz 1: Isopropylamin
Reagenz 2: Fmoc-4-bromphenylalanin
Reagenz 3: 3-Trifluormethylbenzol-sulfonylchlorid
MG = 587,07 (berechnet, monoisotop); Meßwert (M+H)⁺: 588,3.

### Beispiel 26

### 6-(4-Fluor-benzyl)-8-isopropyl-1-(3-trifluormethyl-benzolsulfonyl)-hexahydro- pyrazino[1,2-a]pyrimidin-4,7-dion

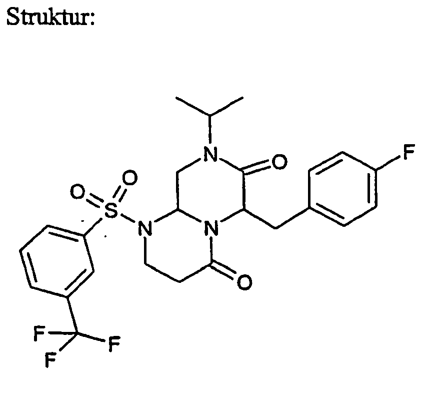

Die Verbindung in Beispiel 26 wurde nach dem in Beispiel 2 beschriebenen Verfahren unter Verwendung folgender Reagenzien synthetisiert:
Reagenz 1: Isopropylamin
Reagenz 2: Fmoc-4-fluorphenylalanin
Reagenz 3: 3-Trifluormethylbenzol-sulfonylchlorid
MG = 527,15 (berechnet, monoisotop); Meßwert (M+H)⁺: 528,3.

### Beispiel 27

### 8-Isopropyl-6-(4-methyl-benzyl)-1-(3-trifluormethyl-benzolsulfonyl)-hexahydro- pyrazino[1,2-a]pyrimidin-4,7-dion

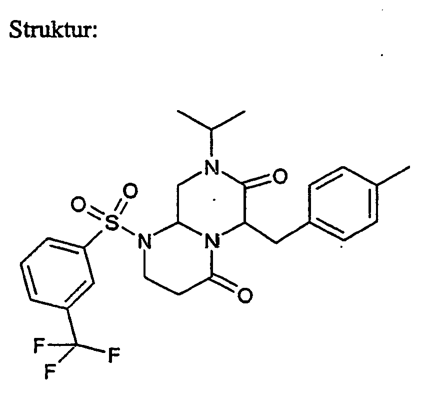

Die Verbindung in Beispiel 27 wurde nach dem in Beispiel 2 beschriebenen Verfahren unter Verwendung folgender Reagenzien synthetisiert:
Reagenz 1: Isopropylamin
Reagenz 2: Fmoc-4-methylphenylalanin
Reagenz 3: 3-Trifluormethylbenzol-sulfonylchlorid
MG = 523,18 (berechnet, monoisotop); Meßwert (M+H)⁺: 524,3.

### Beispiel 28

### 1-(2,5-Dimethyl-benzolsulfonyl)-8-isopropyl-6-(4-methoxy-benzyl)-hexahydro-pyrazino[1,2-a]pyrimidin-4,7-dion

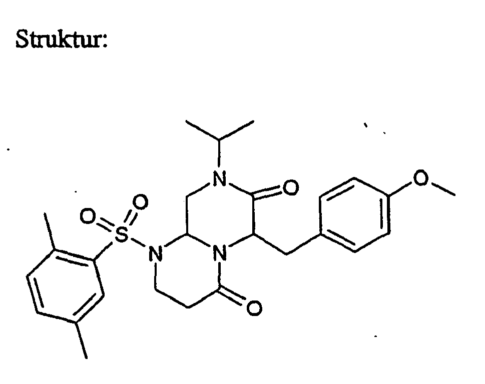

Die Verbindung in Beispiel 28 wurde nach dem in Beispiel 2 beschriebenen Verfahren unter Verwendung folgender Reagenzien synthetisiert:
Reagenz 1: Isopropylamin
Reagenz 2: Fmoc-O-methyltyrosin
Reagenz 3: 2,5-Dimethylbenzol-sulfonylchlorid
MG = 499,21 (berechnet, monoisotop); Meßwert (M+H)⁺: 500,3.

### Beispiel 29

### 6-(4-Brom-benzyl)-1-(2,5-dimethyl-benzolsulfonyl)-8-isopropyl-hexahydro-pyrazino[1,2-a]pyrimidin-4,7-dion

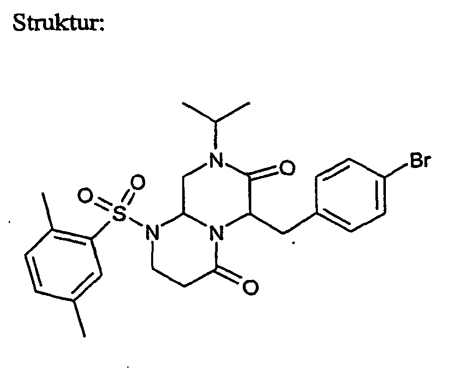

Die Verbindung in Beispiel 29 wurde nach dem in Beispiel 2 beschriebenen Verfahren unter Verwendung folgender Reagenzien synthetisiert:
Reagenz 1: Isopropylamin
Reagenz 2: Fmoc-4-bromphenylalanin
Reagenz 3: 2,5-Dimethylbenzol-sulfonylchlorid
MG = 547,11 (berechnet, monoisotop); Meßwert (M+H)⁺: 548,3.

### Beispiel 30

### 1-(2,5-Dimethyl-benzolsulfonyl)-6-(4-fluor-benzyl)-8-isopropyl-hexahydro-pyrazino[1,2-a]pyrimidin-4,7-dion

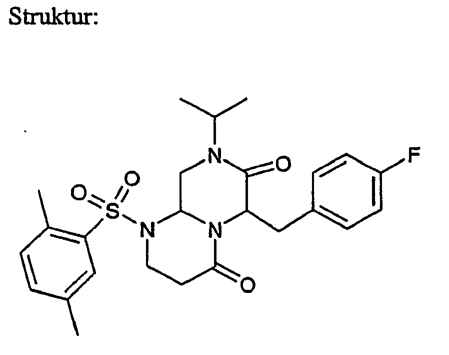

Die Verbindung in Beispiel 30 wurde nach dem in Beispiel 2 beschriebenen Verfahren unter Verwendung folgender Reagenzien synthetisiert:
Reagenz 1: Isopropylamin
Reagenz 2: Fmoc-4-fluorphenylalanin
Reagenz 3: 2,5-Dimethylbenzol-sulfonylchlorid
MG = 487,19 (berechnet, monoisotop); Meßwert (M+H)⁺: 488,3.

### Beispiel 31

### 1-(2,5-Dimethyl-benzolsulfonyl)-8-isopropyl-6-(4-methyl-benzyl)-hexahydro-pyrazino[1,2-a]pyrimidin-4,7-dion

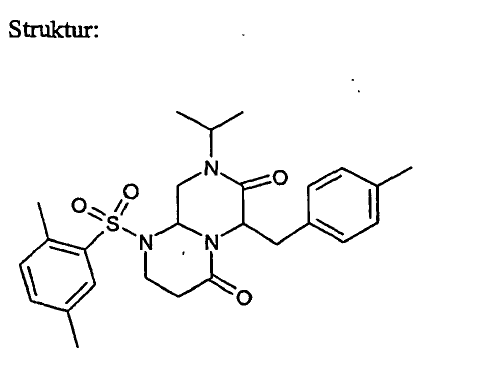

Die Verbindung in Beispiel 31 wurde nach dem in Beispiel 2 beschriebenen Verfahren unter Verwendung folgender Reagenzien synthetisiert:
Reagenz 1: Isopropylamin
Reagenz 2: Fmoc-4-methylphenylalanin
Reagenz 3: 2,5-Dimethylbenzol-sulfonylchlorid
MG = 483,22 (berechnet, monoisotop); Meßwert (M+H)⁺: 484,3.

### Beispiel 32

### 6-(4-Chlor-benzyl)-1-(4-chlor-2,5-dimethyl-benzolsulfonyl)-8-isopropyl-hexahydro-pyrazino[1,2-a]pyrimidin-4,7-dion

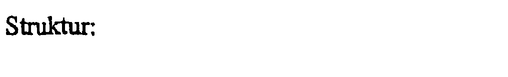

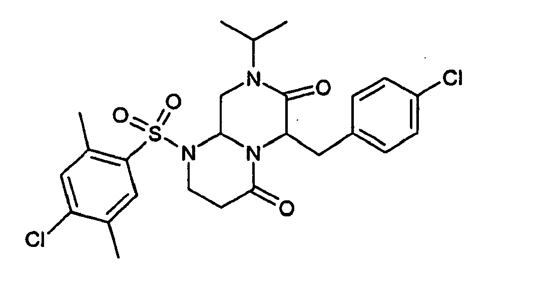

Die Verbindung in Beispiel 32 wurde nach dem in Beispiel 1 beschriebenen Verfahren unter Verwendung folgender Reagenzien synthetisiert:
Reagenz 1: Isopropylamin
Reagenz 2: Fmoc-4-chlorphenylalanin
Reagenz 3: 4-Chlor-2,5-Dimethylbenzol-sulfonylchlorid
MG = 537,13 (berechnet, monoisotop); Meßwert (M+H)⁺: 538,3.

### Beispiel 33

### 6-(4-Chlor-benzyl)-8-isopropyl-1-(2-nitro-benzolsulfonyl)-hexahydro-pyrazino[1,2-a]pyrimidin-4,7-dion

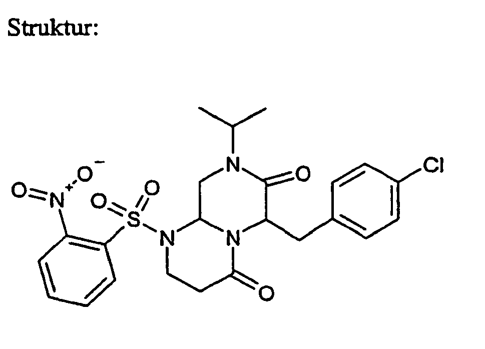

Die Verbindung in Beispiel 33 wurde nach dem in Beispiel 1 beschriebenen Verfahren unter Verwendung folgender Reagenzien synthetisiert:
Reagenz 1: Isopropylamin
Reagenz 2: Fmoc-4-chlorphenylalanin
Reagenz 3: 2-Nitrobenzol-sulfonylchlorid
MG = 520,12 (berechnet, monoisotop); Meßwert (M+H)⁺: 521,3.

### Beispiel 34

### 6-(4-Chlor-benzyl)-1-(2,4-dichlor-5-methyl-benzolsulfonyl)-8-isopropyl-hexahydro-pyrazino[1,2-a]pyrimidin-4,7-dion

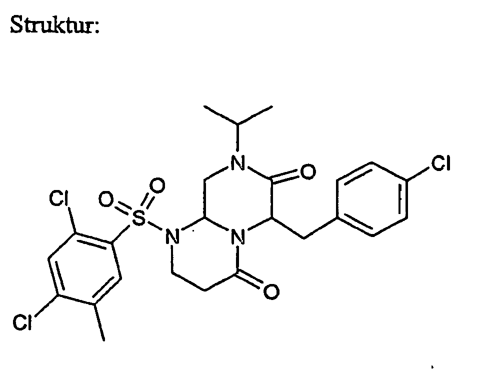

Die Verbindung in Beispiel 34 wurde nach dem in Beispiel 1 beschriebenen Verfahren unter Verwendung folgender Reagenzien synthetisiert:
Reagenz 1: Isopropylamin
Reagenz 2: Fmoc-4-chlorphenylalanin
Reagenz 3: 2,4-Dichlor-5-methylbenzol-sulfonylchlorid
MG = 557,07 (berechnet, monoisotop); Meßwert (M+H)⁺: 558,3.

### Beispiel 35

### 6-(4-Chlor-benzyl)-1-(2-chlor-4-trifluormethyl-benzolsulfonyl)-8-isopropyl-hexahydro-pyrazino[1,2-a]pyrimidin-4,7-dion

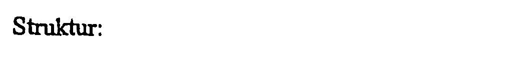

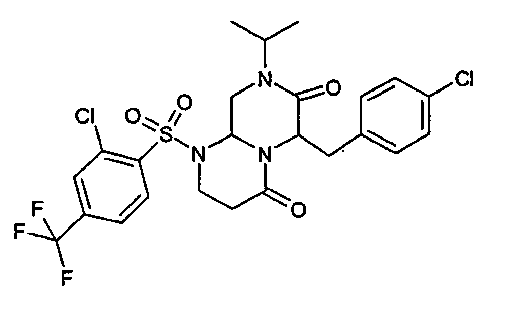

Die Verbindung in Beispiel 35 wurde nach dem in Beispiel 1 beschriebenen Verfahren unter Verwendung folgender Reagenzien synthetisiert:
Reagenz 1: Isopropylamin
Reagenz 2: Fmoc-4-chlorphenylalanin
Reagenz 3: 2-Chlor-4-trifluormethylbenzol-sulfonylchlorid
MG = 577,08 (berechnet, monoisotop); Meßwert (M+H)⁺: 578,3.

### Beispiel 36

### 6-(4-Chlor-benzyl)-8-isopropyl-1-(2-methyl-5-nitro-benzolsulfonyl)-hexahydro- pyrazino[1,2-a]pyrimidin-4,7-dion

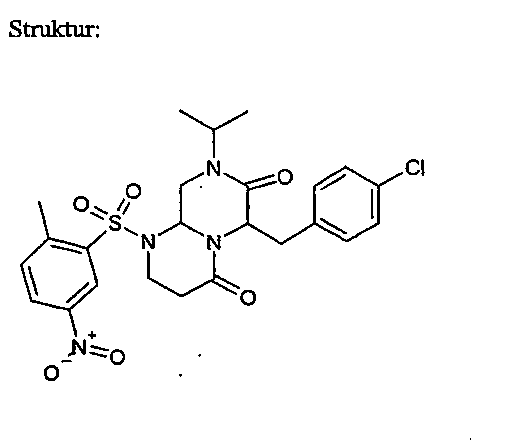

Die Verbindung in Beispiel 36 wurde nach dem in Beispiel 1 beschriebenen Verfahren unter Verwendung folgender Reagenzien synthetisiert:
Reagenz 1: Isopropylamin
Reagenz 2: Fmoc-4-chlorphenylalanin
Reagenz 3: 2-Methyl-5-nitrobenzol-sulfonylchlorid
MG = 534,13 (berechnet, monoisotop); Meßwert (M+H)⁺: 535,3.

### Beispiel 37

### 1-(4-Brom-2-trifluormethoxy-benzolsulfonyl)-6-(4-chlor-benzyl)-8-isopropyl-hexahydro-pyrazino[1,2-a]pyrimidin-4,7-dion

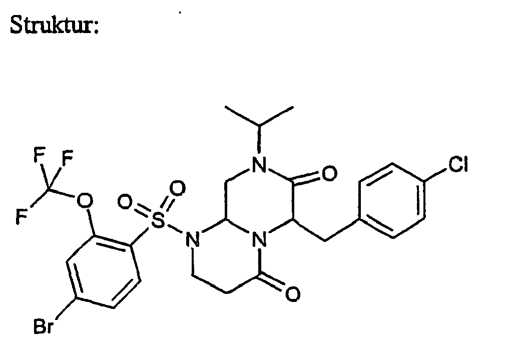

Die Verbindung in Beispiel 37 wurde nach dem in Beispiel 1 beschriebenen Verfahren unter Verwendung folgender Reagenzien synthetisiert:
Reagenz 1: Isopropylamin
Reagenz 2: Fmoc-4-chlorphenylalanin
Reagenz 3: 4-Brombenzol-2-trifluormethoxy-sulfonylchlorid
MG = 637,03 (berechnet, monoisotop); Meßwert (M+H)⁺: 638,4.

### Beispiel 38

### 6-(1-Benzyl-1H-imidazol-4-ylmethyl)-1-(4-brom-2-ethyl-benzolsulfonyl)-8-(2-pyridin-4- yl-ethyl)-hexahydro-pyrazino[1,2-a]pyrimidin-4,7-dion

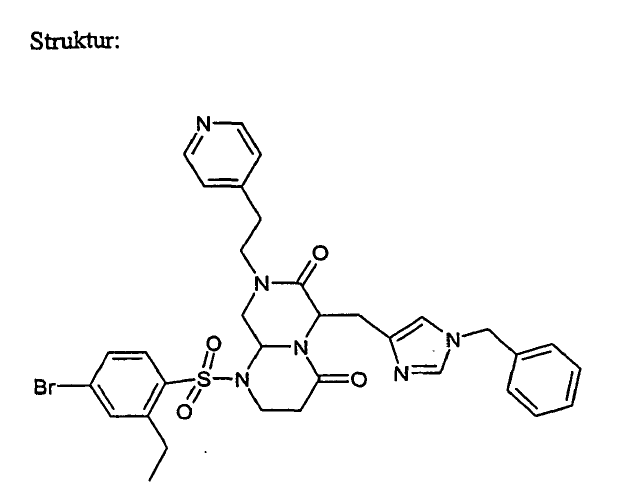

Die Verbindung in Beispiel 38 wurde nach dem in Beispiel 2 beschriebenen Verfahren unter Verwendung folgender Reagenzien synthetisiert:
Reagenz 1: 2-(4-Pyridyl)ethylamin
Reagenz 2: Fmoc-histidin(benzyl)
Reagenz 3: 4-Brom-2-ethyl-benzol-sulfonylchlorid
MG = 690,16 (berechnet, monoisotop); Meßwert (M+H)⁺: 691,4.

### Beispiel 39

### 1-(5-Chlor-2-methoxy-benzolsulfonyl)-8-isopropyl-6-(4-nitro-benzyl)-hexahydro-pyrazino[1,2-a]pyrimidin-4,7-dion

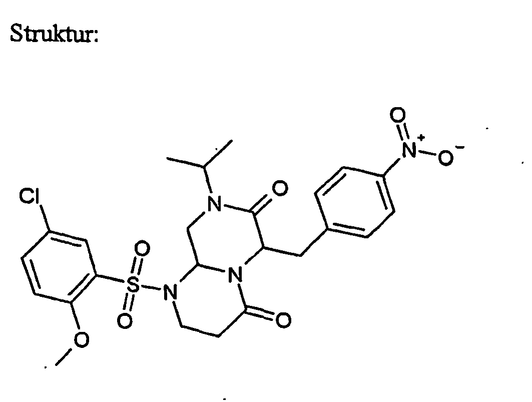

Die Verbindung in Beispiel 39 wurde nach dem in Beispiel 2 beschriebenen Verfahren unter Verwendung folgender Reagenzien synthetisiert:
Reagenz 1: Isopropylamin
Reagenz 2: Fmoc-4-nitrophenylalanin
Reagenz 3: 2-Methoxy-5-chlorbenzol-sulfonylchlorid
MG = 550,13 (berechnet, monoisotop); Meßwert (M+H)⁺: 551,3.

### Beispiel 40

### 6-(4-Chlor-benzyl)-8-isopropyl-1-(naphthalen-2-sulfonyl)-hexahydro-pyrazino[1,2-a]pyrimidin-4,7-dion

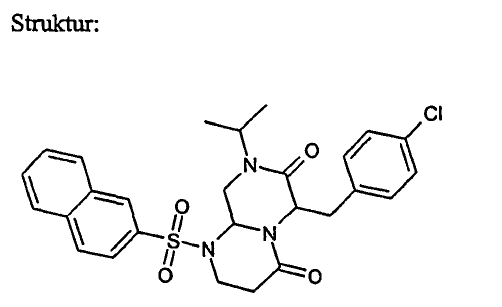

Die Verbindung in Beispiel 40 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 2-Naphthyl-sulfonylchlorid
MG = 525,15 (berechnet, monoisotop); Meßwert (M+H)⁺: 526,3.

### Beispiel 41

### 6-(3,4-Dichlor-benzyl)-8-isopropyl-1-(naphthalen-2-sulfonyl)-hexahydro-pyrazino[1,2-a]pyrimidin-4,7-dion

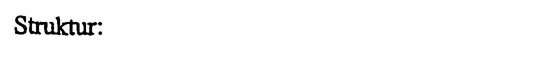

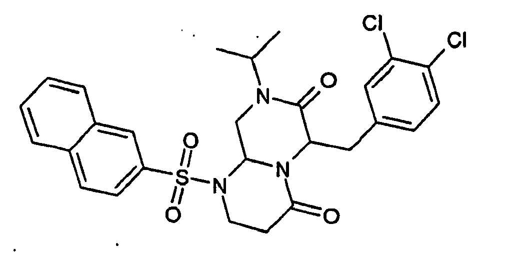

Die Verbindung in Beispiel 41 wurde nach dem in Beispiel 2 beschriebenen Verfahren unter Verwendung folgender Reagenzien synthetisiert:
Reagenz 1: Isopropylamin
Reagenz 2: Fmoc-3,4-dichlorphenylalanin
Reagenz 3: 2-Naphthyl-sulfonylchlorid
MG = 559,11 (berechnet, monoisotop); Meßwert (M+H)⁺: 560,3.

### Beispiel 42

### 6-(3,4-Dichlor-benzyl)-1-(3,4-dimethoxy-benzolsulfonyl)-8-isopropyl-hexahydro-pyrazino[1,2-a]pyrimidin-4,7-dion

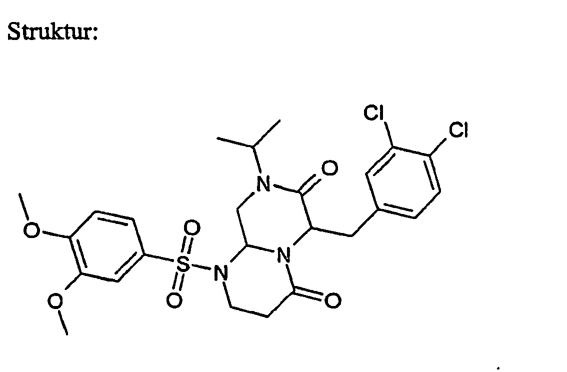

Die Verbindung in Beispiel 42 wurde nach dem in Beispiel 2 beschriebenen Verfahren unter Verwendung folgender Reagenzien synthetisiert:
Reagenz 1: Isopropylamin
Reagenz 2: Fmoc-3,4-dichlorphenylalanin
Reagenz 3: 3,4-Dimethoxybenzol-sulfonylchlorid
MG = 569,12 (berechnet, monoisotop); Meßwert (M+H)⁺: 570,3.

### Beispiel 43

### 8-Allyl-1-(4-brom-2-ethyl-benzolsulfonyl)-6-(4-chlor-benzyl)-hexahydro-pyrazino[1,2-a]pyrimidin-4,7-dion

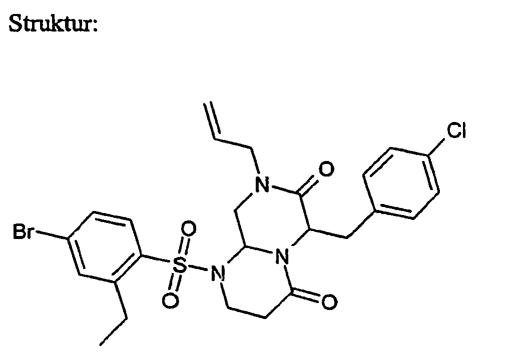

Die Verbindung in Beispiel 43 wurde nach dem in Beispiel 2 beschriebenen Verfahren unter Verwendung folgender Reagenzien synthetisiert:
Reagenz 1: Allylamin
Reagenz 2: Fmoc-4-chlorphenylalanin
Reagenz 3: 2-Ethyl-4-brombenzol-sulfonylchlorid
MG = 579,06 (berechnet, monoisotop); Meßwert (M+H)⁺: 580,3.

### Beispiel 44

### 8-Allyl-1-(4-brom-2-ethyl-benzolsulfonyl)-6-(4-nitro-benzyl)-hexahydro-pyrazino[1,2-a]pyrimidin-4,7-dion

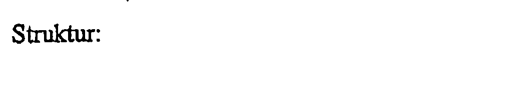

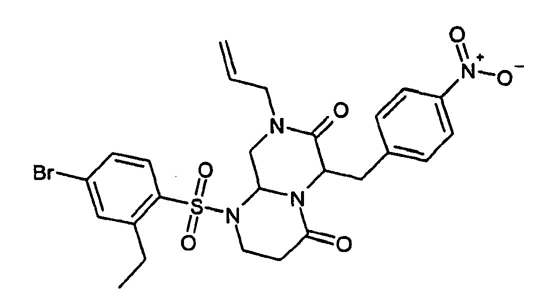

Die Verbindung in Beispiel 44 wurde nach dem in Beispiel 2 beschriebenen Verfahren unter Verwendung folgender Reagenzien synthetisiert:
Reagenz 1: Allylamin
Reagenz 2: Fmoc-4-nitrophenylalanin
Reagenz 3: 2-Ethyl-4-brombenzol-sulfonylchlorid
MG = 590,08 (berechnet, monoisotop); Meßwert (M+H)⁺: 591,3.

### Beispiel 45

### 8-Allyl-1-(5-chlor-2-methoxy-benzolsulfonyl)-6-(3,4-dichlor-benzyl)-hexahydro- pyrazino[1,2-a]pyrimidin-4,7-dion

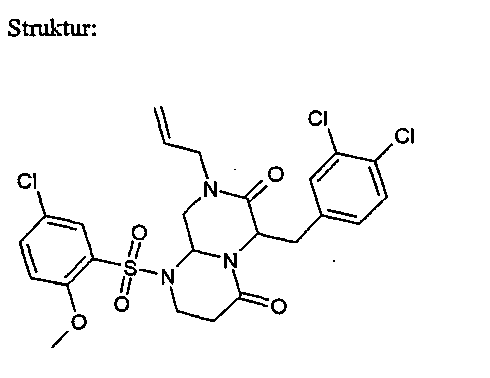

Die Verbindung in Beispiel 45 wurde nach dem in Beispiel 2 beschriebenen Verfahren unter Verwendung folgender Reagenzien synthetisiert:
Reagenz 1: Allylamin
Reagenz 2: Fmoc-3,4-dichlorphenylalanin
Reagenz 3: 5-Chlor-2-methoxy-benzol-sulfonylchlorid
MG = 571,05 (berechnet, monoisotop); Meßwert (M+H)⁺: 572,3.

### Beispiel 46

### 8-Allyl-1-(naphthalen-2-sulfonyl)-6-(4-nitro-benzyl)-hexahydro-pyrazino[1,2- a]pyrimidin-4,7-dion

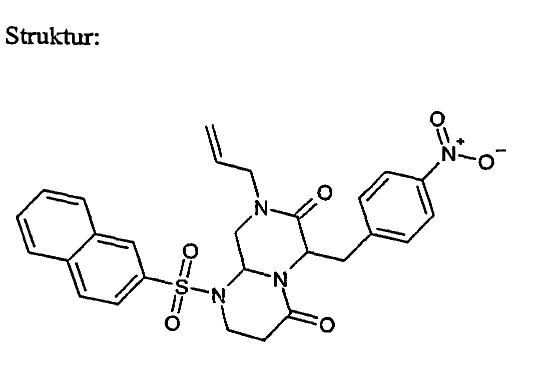

Die Verbindung in Beispiel 46 wurde nach dem in Beispiel 2 beschriebenen Verfahren unter Verwendung folgender Reagenzien synthetisiert:
Reagenz 1: Allylamin
Reagenz 2: Fmoc-4-nitrophenylalanin
Reagenz 3: 2-Naphthyl-sulfonylchlorid
MG = 534,16 (berechnet, monoisotop); Meßwert (M+H)⁺: 535,3.

### Beispiel 47

### 1-(5-Chlor-2-methoxy-benzolsulfonyl)-8-isopropyl-6-pyridin-4-ylmethyl-hexahydro-pyrazino[1,2-a]pyrimidin-4,7-dion

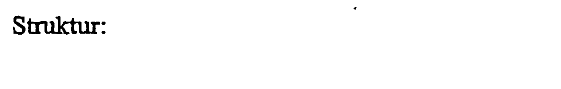

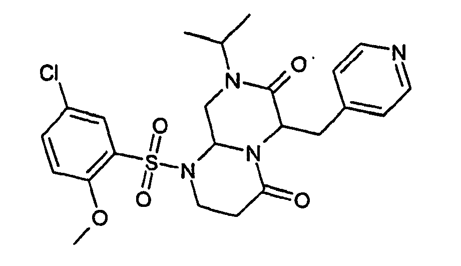

Die Verbindung in Beispiel 47 wurde nach dem in Beispiel 1 beschriebenen Verfahren unter Verwendung folgender Reagenzien synthetisiert:
Reagenz 1: Isopropylamin
Reagenz 2: Fmoc-4-pyridylalanin
Reagenz 3: 2-Methoxy-5-chlorbenzol-sulfonylchlorid
MG = 506,14 (berechnet, monoisotop); Meßwert (M+H)⁺: 507,3.

### Beispiel 48

### 1-(5-Brom-2-methoxy-benzolsulfonyl)-6-(4-chlor-benzyl)-8-isopropyl-hexahydro-pyrazino[1,2-a]pyrimidin-4,7-dion

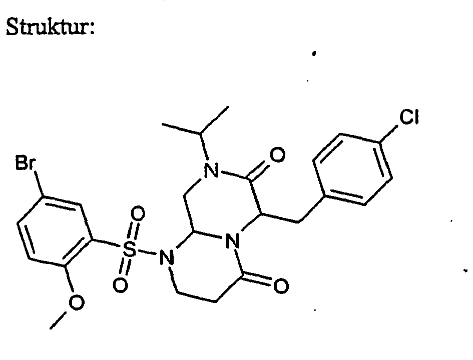

Die Verbindung in Beispiel 48 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 5-Brom-2-methoxybenzol-sulfonylchlorid
MG = 583,05 (berechnet, monoisotop); Meßwert (M+H)⁺: 584,3.

### Beispiel 49

### 6-(4-Chlor-benzyl)-8-isopropyl-1-(2-methoxy-5-methyl-benzolsulfonyl)-hexahydro-pyrazino[1,2-a]pyrimidin-4,7-dion

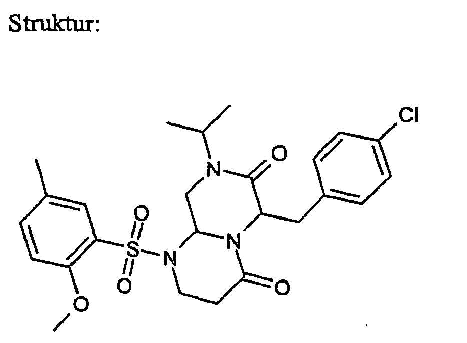

Die Verbindung in Beispiel 49 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 2-Methoxy-5-methylbenzol-sulfonylchlorid
MG = 519,16 (berechnet, monoisotop); Meßwert (M+H)⁺: 520,3.

### Beispiel 50

### 6-(4-Chlor-benzyl)-8-isopropyl-1-(2-trifluormethoxy-benzolsulfonyl)-hexahydro-pyrazino[1,2-a]pyrimidin-4,7-dion

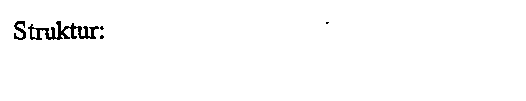

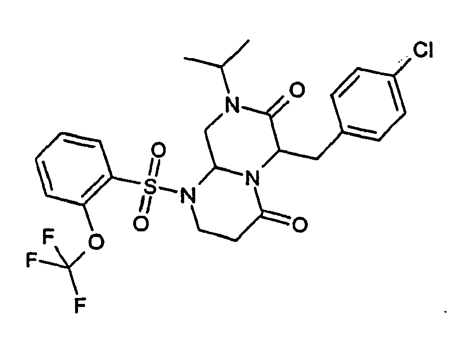

Die Verbindung in Beispiel 50 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 2-Trifluormethoxy-benzol-sulfonylchlorid
MG = 559,12 (berechnet, monoisotop); Meßwert (M+H)⁺: 560,3.

### Beispiel 51

### 6-(4-Chlor-benzyl)-8-isopropyl-1-(2-methansulfonyl-benzolsulfonyl)-hexahydro-pyrazino[1,2-a]pyrimidin-4,7-dion

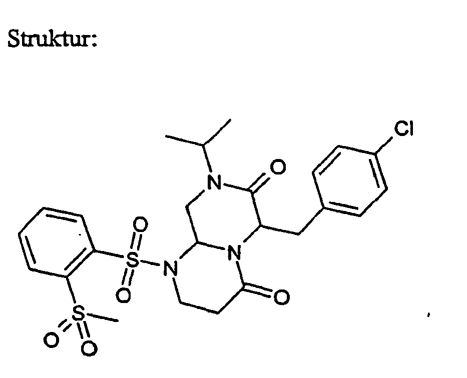

Die Verbindung in Beispiel 51 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 2-Methylsulfonyl-benzol-sulfonylchlorid
MG = 553,11 (berechnet, monoisotop); Meßwert (M+H)⁺: 554,3.

### Beispiel 52

### 3-[6-(4-Chlor-benzyl)-8-isopropyl-4,7-dioxo-hexahydro-pyrazino[1,2-a]pyrimidin-1- sulfonyl]-benzonitril

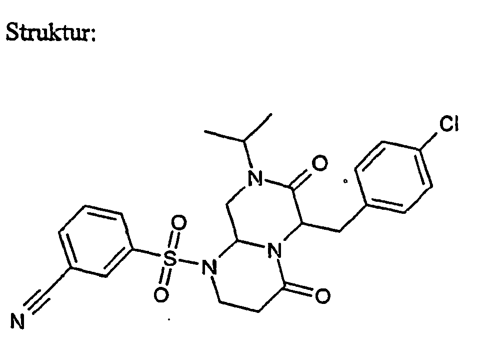

Die Verbindung in Beispiel 52 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 3-Cyanobenzol-sulfonylchlorid
MG = 500,13 (berechnet, monoisotop); Meßwert (M+H)⁺: 501,3.

### Beispiel 53

### 6-(4-Chlor-benzyl)-8-isopropyl-1-(3-trifluormethyl-benzolsulfonyl)-hexahydro- pyrazino[1,2-a]pyrimidin-4,7-dion

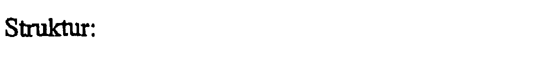

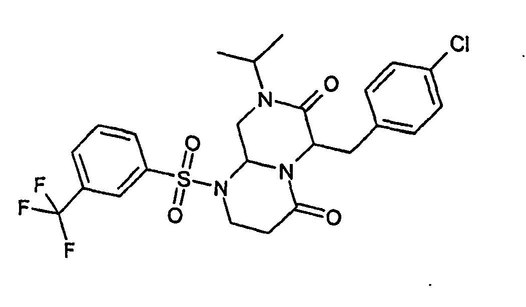

Die Verbindung in Beispiel 53 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 3-Trifluormethylbenzol-sulfonylchlorid
MG = 543,12 (berechnet, monoisotop); Meßwert (M+H)⁺: 544,3.

### Beispiel 54

### 6-(4-Chlor-benzyl)-8-isopropyl-1-(2,4,6-trichlor-benzolsulfonyl)-hexahydro- pyrazino[1,2-a]pyrimidin-4,7-dion

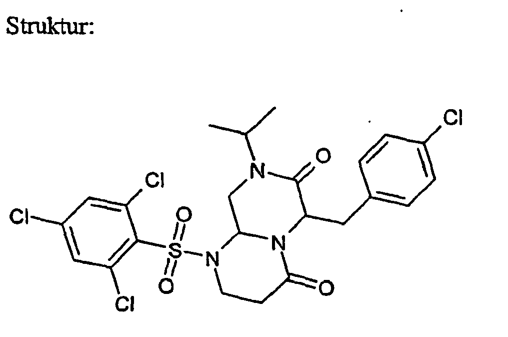

Die Verbindung in Beispiel 54 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 2,4,6-Trichlorbenzol-sulfonylchlorid
MG = 577,02 (berechnet, monoisotop); Meßwert (M+H)⁺: 578,3.

### Beispiel 55

### 6-(4-Chlor-benzyl)-1-(2,5-dimethoxy-benzolsulfonyl)-8-isopropyl-hexahydro-pyrazino[1,2-a]pyrimidin-4,7-dion

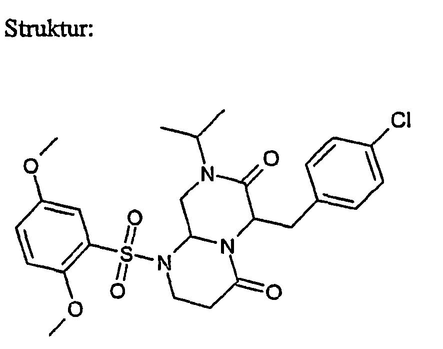

Die Verbindung in Beispiel 55 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 2,5-Dimethoxybenzol-sulfonylchlorid
MG = 535,15 (berechnet, monoisotop); Meßwert (M+H)⁺: 536,3.

### Beispiel 56

### 6-(4-Chlor-benzyl)-1-(2,5-dichlor-benzolsulfonyl)-8-isopropyl-hexahydro-pyrazino[1,2-a]pyrimidin-4,7-dion

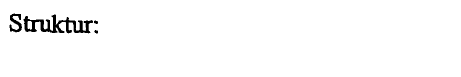

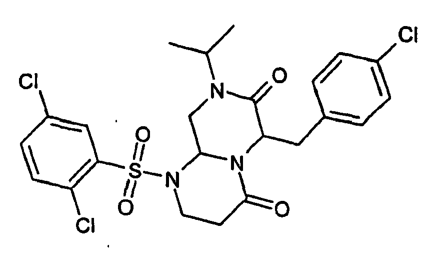

Die Verbindung in Beispiel 56 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 2,5-Dichlorbenzol-sulfonylchlorid
MG = 543,06 (berechnet, monoisotop); Meßwert (M+H)⁺: 544,3.

### Beispiel 57

### 6-(4-Chlor-benzyl)-1-(5-chlor-2-methoxy-benzolsulfonyl)-8-isopropyl-4,7-dioxo- octahydropyrazino[1,2-a]pyrimidin-2-carbonsäureallylester

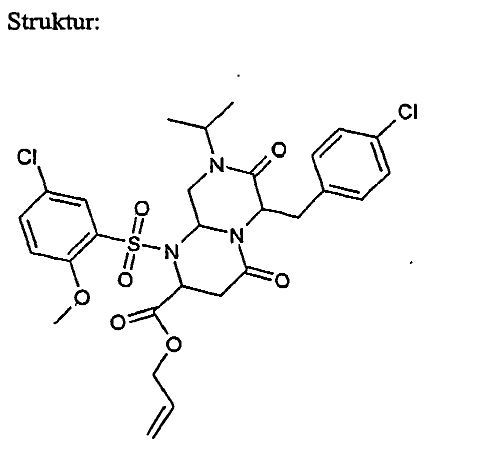

Die Verbindung in Beispiel 57 wurde nach dem in Beispiel 1 beschriebenen Verfahren unter Verwendung der nachstehend aufgeführten Reagenzien und mit folgenden Modifikationen synthetisiert:
Reagenz 1: Isopropylamin
Reagenz 2: Fmoc-4-chlorphenylalanin
Nach diesem Schritt und nach Aufhebung des Fmoc-Schutzes wurde die Kupplung von Fmoc-beta-alanin aus dem Verfahren in Beispiel 1 unter den gleichen Bedingungen durch eine Fmoc-Asp(OH)-O-Allyl-Kopplung ersetzt. Nach Aufhebung des Fmoc-Schutzes wurde die Synthese entsprechend dem Verfahren für Beispiel 1 mit Reagenz 3 fortgesetzt.
Reagenz 3: 5-Chlor-2-methoxybenzol-sulfonylchlorid
MG = 623,13 (berechnet, monoisotop); Meßwert (M+H)⁺: 624,4.

### Beispiel 58

### 1-(5-Chlor-2-methoxy-benzolsulfonyl)-8-isopropyl-6-(4-methoxy-benzyl)-hexahydro-pyrazino[1,2-a]pyrimidin-4,7-dion

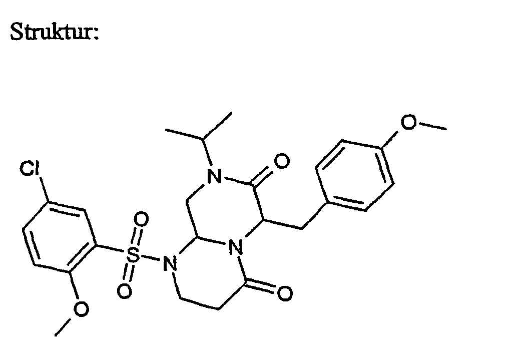

Die Verbindung in Beispiel 58 wurde nach dem in Beispiel 1 beschriebenen Verfahren unter Verwendung folgender Reagenzien synthetisiert:
Reagenz 1: Isopropylamin
Reagenz 2: Fmoc-O-methyltyrosin
Reagenz 3: 5-Chlor-2-methoxybenzol-sulfonylchlorid
MG = 535,15 (berechnet, monoisotop); Meßwert (M+H)⁺: 536,3.

### Beispiel 59

### 2-(4-Amino-butyl)-6-(4-chlor-benzyl)-1-(5-chlor-2-methoxy-benzolsulfonyl)-8-isopropyl-hexahydro-pyrazino[1,2-a]pyrimidin-4,7-dion

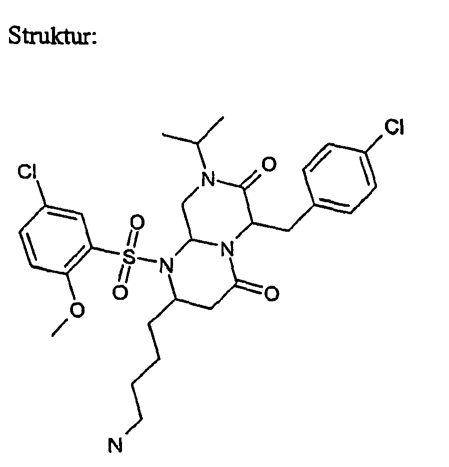

Die Verbindung in Beispiel 59 wurde nach dem in Beispiel 1 beschriebenen Verfahren unter Verwendung der nachstehend aufgeführten Reagenzien und mit folgenden Modifikationen synthetisiert:
Reagenz 1: Isopropylamin
Reagenz 2: Fmoc-4-chlorphenylalanin
Nach diesem Schritt und nach Aufhebung des Fmoc-Schutzes wurde die Kupplung von Fmoc-beta-alanin aus dem Verfahren in Beispiel 1 unter den gleichen Bedingungen durch eine Fmoc-beta-homolysin(Boc)-Kupplung ersetzt. Nach Aufhebung des Fmoc-Schutzes wurde die Synthese entsprechend dem Verfahren für Beispiel 1 mit Reagenz 3 fortgesetzt.
Reagenz 3: 5-Chlor-2-methoxybenzol-sulfonylchlorid
MG = 610,18 (berechnet, monoisotop); Meßwert (M+H)⁺: 611,4.

### Beispiel 60

### 6-(4-Chlor-benzyl)-8-ethyl-1-(2-methoxy-5-methyl-benzolsulfonyl)-hexahydro-pyrazino[1,2-a]pyrimidin-4,7-dion

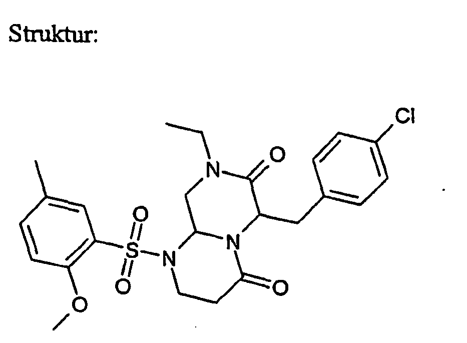

Die Verbindung in Beispiel 60 wurde nach dem in Beispiel 2 beschriebenen Verfahren unter Verwendung folgender Reagenzien synthetisiert:
Reagenz 1: Ethylamin
Reagenz 2: Fmoc-4-chlorphenylalanin
Reagenz 3: 2-Methoxy-5-methylbenzol-sulfonylchlorid
MG = 505,14 (berechnet, monoisotop); Meßwert (M+H)⁺: 506,3.

### Beispiel 61

### 6-(4-Chlor-benzyl)-1-(2-methoxy-5-methyl-benzolsulfonyl)-8-methyl-hexahydro- pyrazin[1,2-a]pyrimidin-4,7-dion

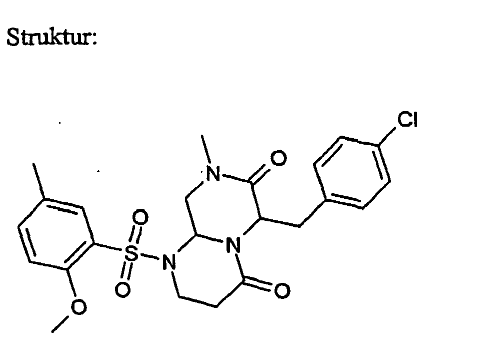

Die Verbindung in Beispiel 61 wurde nach dem in Beispiel 2 beschriebenen Verfahren unter Verwendung folgender Reagenzien synthetisiert:
Reagenz 1: Methylamin
Reagenz 2: Fmoc-4-chlorphenylalanin
Reagenz 3: 2-Methoxy-5-methylbenzol-sulfonylchlorid
MG = 491,13 (berechnet, monoisotop); Meßwert (M+H)⁺: 492,3.

### Beispiel 62

### 6-(4-Chlor-benzyl)-8-isobutyl-1-(2-methoxy-5-methyl-benzolsulfonyl)-hexahydro-pyrazino[1,2-a]pyrimidin-4,7-dion

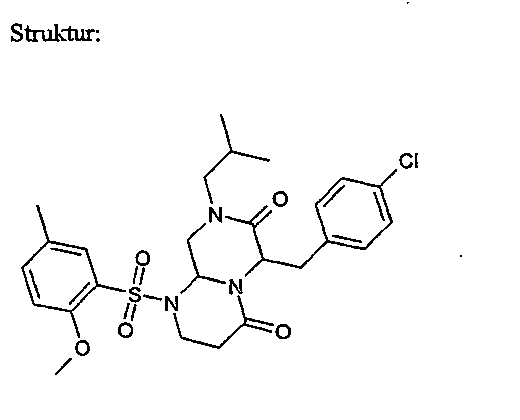

Die Verbindung in Beispiel 62 wurde nach dem in Beispiel 2 beschriebenen Verfahren unter Verwendung folgender Reagenzien synthetisiert:
Reagenz 1: Isobutylamin
Reagenz 2: Fmoc-4-chlorphenylalanin
Reagenz 3: 2-Methoxy-5-methylbenzol-sulfonylchlorid
MG = 533,18 (berechnet, monoisotop); Meßwert (M+H)⁺: 534,3.

### Beispiel 63

### 6-(4-Chlor-benzyl)-8-isobutyl-1-(2-methoxy-5-methyl-benzolsulfonyl)-hexahydropyrazino[1,2-a]pyrimidin-4,7-dion

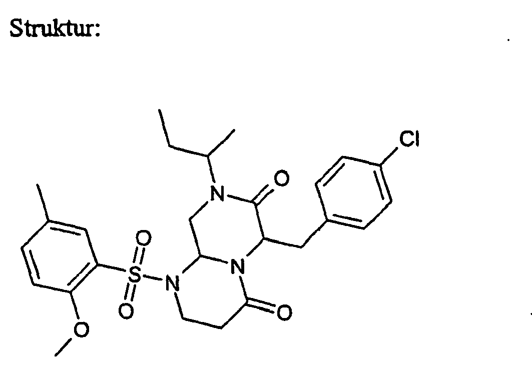

Die Verbindung in Beispiel 63 wurde nach dem in Beispiel 2 beschriebenen Verfahren unter Verwendung folgender Reagenzien synthetisiert:
Reagenz 1: 2-Butylamin
Reagenz 2: Fmoc-4-chlorphenylalanin
Reagenz 3: 2-Methoxy-5-methylbenzol-sulfonylchlorid
MG = 533,18 (berechnet, monoisotop); Meßwert (M+H)⁺: 534,3.

### Beispiel 64

### 1-(5-Chlor-2-methoxy-benzolsulfonyl)-8-isopropyl-6-pyridin-3-ylmethyl-hexahydro-pyrazino[1,2-a]pyrimidin-4,7-dion

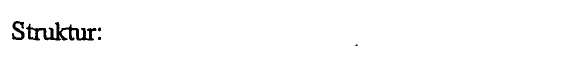

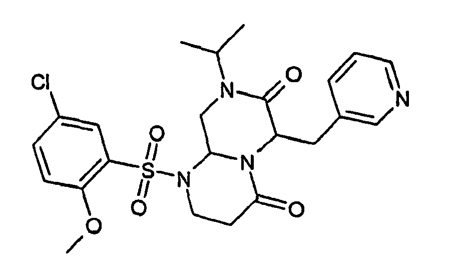

Die Verbindung in Beispiel 64 wurde nach dem in Beispiel 1 beschriebenen Verfahren unter Verwendung folgender Reagenzien synthetisiert:
Reagenz 1: Isopropylamin
Reagenz 2: Fmoc-3-pyridylalanin
Reagenz 3: 5-Chlor-2-methoxybenzol-sulfonylchlorid
MG = 506,14 (berechnet, monoisotop); Meßwert (M+H)⁺: 507,3.

### Beispiel 65

### 6-(4-Chlor-benzyl)-8-cyclopropyl-1-(2-methoxy-5-methyl-benzolsulfonyl)-hexahydro-pyrazino[1,2-a]pyrimidin-4,7-dion

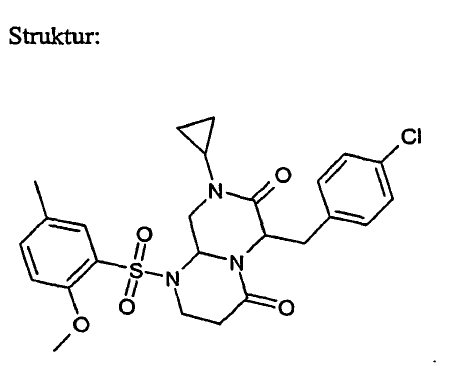

Die Verbindung in Beispiel 65 wurde nach dem in Beispiel 2 beschriebenen Verfahren unter Verwendung folgender Reagenzien synthetisiert:
Reagenz 1: Cyclopropylamin
Reagenz 2: Fmoc-4-chlorphenylalanin
Reagenz 3: 2-Methoxy-5-methylbenzol-sulfonylchlorid
MG = 517,14 (berechnet, monoisotop); Meßwert (M+H)⁺: 518,3.

### Beispiel 66

### 6-(4-Chlor-benzyl)-8-cyclopentyl-1-(2-methoxy-5-methyl-benzolsulfonyl)-hexahydro-pyrazino[1,2-a]pyrimidin-4,7-dion

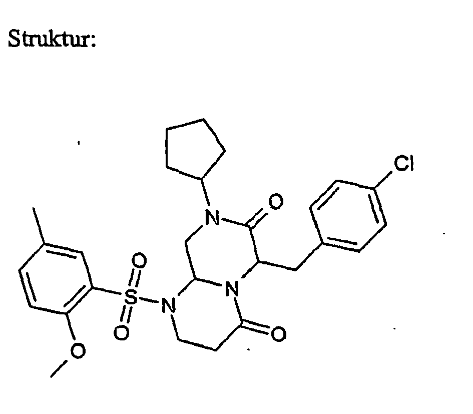

Die Verbindung in Beispiel 66 wurde nach dem in Beispiel 2 beschriebenen Verfahren unter Verwendung folgender Reagenzien synthetisiert:
Reagenz 1: Cyclopentylamin
Reagenz 2: Fmoc-4-chlorphenylalanin
Reagenz 3: 2-Methoxy-5-methylbenzol-sulfonylchlorid
MG = 545,18 (berechnet, monoisotop); Meßwert (M+H)⁺: 546,3.

### Beispiel 67

### 6-(4-Chlor-benzyl)-1-(2-methoxy-5-methyl-benzolsulfonyl)-8-propyl-hexahydro- pyrazino[1,2-a]pyrimidin-4,7-dion

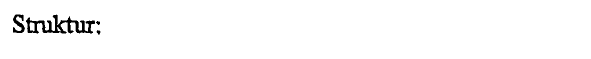

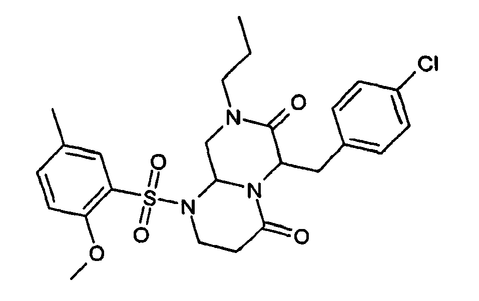

Die Verbindung in Beispiel 67 wurde nach dem in Beispiel 2 beschriebenen Verfahren unter Verwendung folgender Reagenzien synthetisiert:
Reagenz 1: n-Propylamin
Reagenz 2: Fmoc-4-chlorphenylalanin
Reagenz 3: 2-Methoxy-5-methylbenzol-sulfonylchlorid
MG = 519,16 (berechnet, monoisotop); Meßwert (M+H)⁺: 520,3.

### Beispiel 68

### 8-Allyl-6-(4-chlor-benzyl)-1-(2-methoxy-5-methyl-benzolsulfonyl)-hexahydro-pyrazino[1,2-a]pyrimidin-4,7-dion

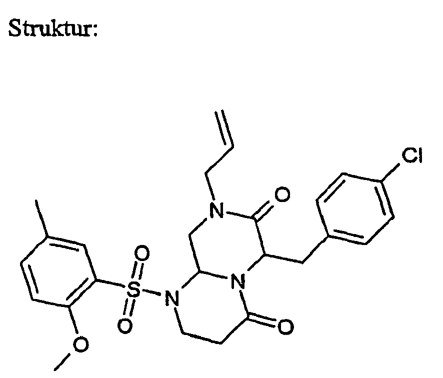

Die Verbindung in Beispiel 68 wurde nach dem in Beispiel 2 beschriebenen Verfahren unter Verwendung folgender Reagenzien synthetisiert:
Reagenz 1: Allylamin
Reagenz 2: Fmoc-4-chlorphenylalanin
Reagenz 3: 2-Methoxy-5-methylbenzol-sulfonylchlorid
MG = 517,14 (berechnet, monoisotop); Meßwert (M+H)⁺: 518,2.

### Beispiel 69

### 1-(5-Brom-2-methoxy-benzolsulfonyl)-6-(4-chlor-benzyl)-8-ethyl-hexahydro-pyrazino[1,2-a]pyrimidin-4,7-dion

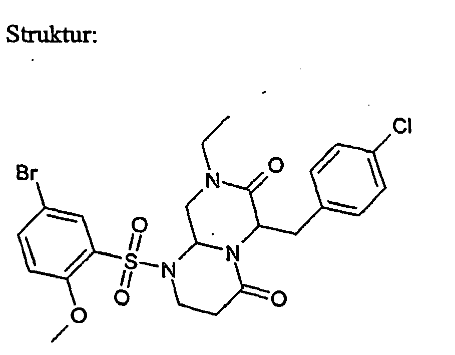

Die Verbindung in Beispiel 69 wurde nach dem in Beispiel 2 beschriebenen Verfahren unter Verwendung folgender Reagenzien synthetisiert:
Reagenz 1: Ethylamin
Reagenz 2: Fmoc-4-chlorphenylalanin
Reagenz 3: 2-Methoxy-5-brombenzol-sulfonylchlorid
MG = 569,04 (berechnet, monoisotop); Meßwert (M+H)⁺: 570,3.

### Beispiel 70

### 6-(4-Chlor-benzyl)-1-(5-chlor-2-methoxy-benzolsulfonyl)-8-ethyl-hexahydro-pyrazino[1,2-a]pyrimidin-4,7-dion

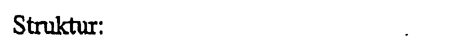

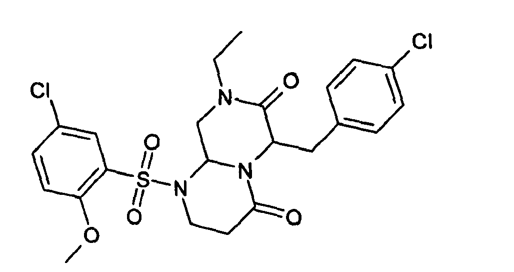

Die Verbindung in Beispiel 70 wurde nach dem in Beispiel 2 beschriebenen Verfahren unter Verwendung folgender Reagenzien synthetisiert:
Reagenz 1: Ethylamin
Reagenz 2: Fmoc-4-chlorphenylalanin
Reagenz 3: 2-Methoxy-5-chlorbenzol-sulfonylchlorid
MG = 525,09 (berechnet, monoisotop); Meßwert (M+H)⁺: 526,3.

### Beispiel 71

### 1-(4-Brom-2-ethyl-benzolsulfonyl)-6-(4-chlor-benzyl)-8-isopropyl-hexahydro- pyrazino[1,2-a]pyrimidin-4,7-dion

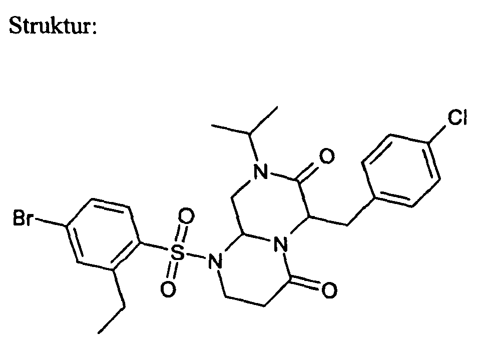

Die Verbindung in Beispiel 71 wurde nach dem in Beispiel 2 beschriebenen Verfahren unter Verwendung folgender Reagenzien synthetisiert:
Reagenz 1: Isopropylamin
Reagenz 2: Fmoc-4-chlorphenylalanin
Reagenz 3: 4-Brom-2-ethyl-benzol-sulfonylchlorid
MG = 581,08 (berechnet, monoisotop); Meßwert (M+H)⁺: 582,3.

### Beispiel 72

### 6-(4-Chlor-benzyl)-1-(2,5-dimethyl-benzolsulfonyl)-8-ethyl-hexahydro-pyrazino[1,2-a]pyrimidin-4,7-dion

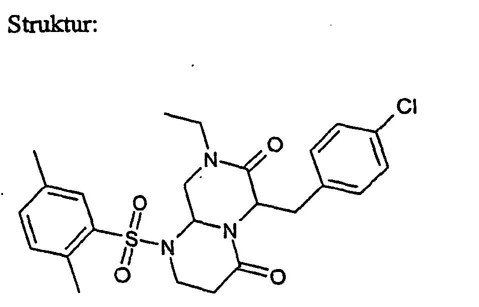

Die Verbindung in Beispiel 72 wurde nach dem in Beispiel 2 beschriebenen Verfahren unter Verwendung folgender Reagenzien synthetisiert:
Reagenz 1: Ethylamin
Reagenz 2: Fmoc-4-chlorphenylalanin
Reagenz 3: 2,5-Dimethylbenzol-sulfonylchlorid
MG = 489,15 (berechnet, monoisotop); Meßwert (M+H)⁺: 490,3.

### Beispiel 73

### 2-Chlor-5-[6-(4-chlor-benzyl)-8-isopropyl-4,7-dioxo-hexahydro-pyrazino[1,2-a]pyrimidin-1-sulfonyl]-benzolsulfonamid

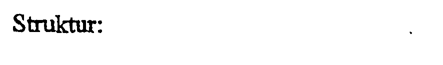

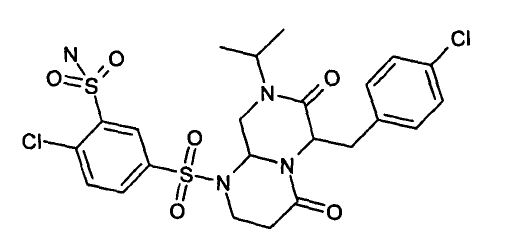

Die Verbindung in Beispiel 73 wurde nach dem in Beispiel 1 beschriebenen Verfahren unter Verwendung folgender Reagenzien synthetisiert:
Reagenz 1: Isopropylamin
Reagenz 2: Fmoc-4-chlorphenylalanin
Reagenz 3: 4-Chlor-3-sulfonylamido-benzol-sulfonylchlorid
MG = 588,07 (berechnet, monoisotop); Meßwert (M+H)⁺: 589,3.

### Beispiel 74

### 6-(4-Chlor-benzyl)-1-(2,4-dichlor-6-methyl-benzolsulfonyl)-8-isopropyl-hexahydro-pyrazino[1,2-a]pyrimidin-4,7-dion

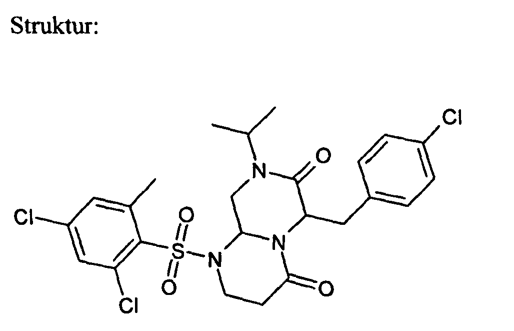

Die Verbindung in Beispiel 74 wurde nach dem in Beispiel 1 beschriebenen Verfahren unter Verwendung folgender Reagenzien synthetisiert:
Reagenz 1: Isopropylamin
Reagenz 2: Fmoc-4-chlorphenylalanin
Reagenz 3: 2-Methyl-4,6-dichlorbenzol-sulfonylchlorid
MG = 557,08 (berechnet, monoisotop); Meßwert (M+H)⁺: 558,3.

### Beispiel 75

### 6-(4-Chlor-benzyl)-8-isopropyl-1-(2-methoxy-4-methyl-benzolsulfonyl)-hexahydro-pyrazino[1,2-a]pyrimidin-4,7-dion

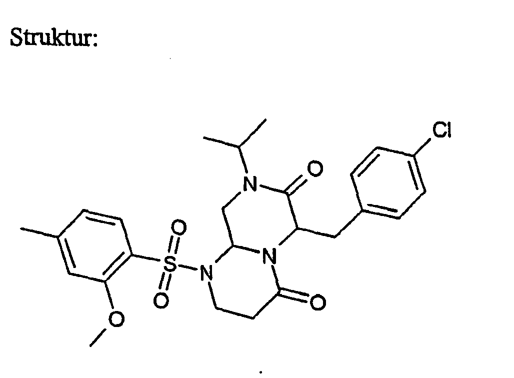

Die Verbindung in Beispiel 75 wurde nach dem in Beispiel 1 beschriebenen Verfahren unter Verwendung folgender Reagenzien synthetisiert:
Reagenz 1: Isopropylamin
Reagenz 2: Fmoc-4-chlorphenylalanin
Reagenz 3: 2-Methoxy-4-methylbenzol-sulfonylchlorid
MG = 519,16 (berechnet, monoisotop); Meßwert (M+H)⁺: 520,3.

### Beispiel 76

### 6-(4-Chlor-benzyl)-8-isopropyl-1-(4-methoxy-benzolsulfonyl)-hexahydro-pyrazino[1,2-a]pyrimidin-4,7-dion

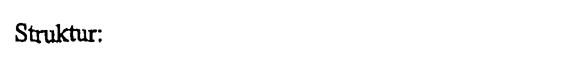

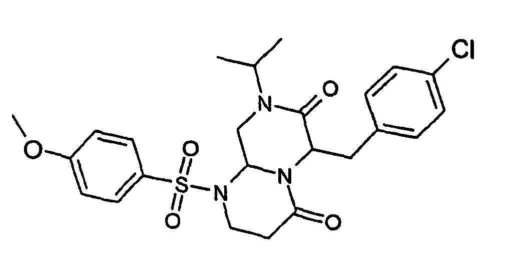

### a) 3-(4-Chlor-phenyl)-N-(2,2-diethoxy-ethyl)-N-isopropyl-2-[3-(4-methoxybenzolsulfonylamino)-propionylamino]-propionamid

Zu einer Lösung aus 124 mg 3-(4-Methoxy-benzolsulfonylamino)-propionsäure in 1 mL DMF werden 52 mg EDC, 45 mg HOBt und 100 µl N-Ethylmorpholin gegeben. Dazu tropft man eine Lösung aus 100 mg 2-Amino-3-(4-chlor-phenyl)-N-(2,2-diethoxy-ethyl)-N-isopropyl-propionamid in 1 mL DMF und lässt 12 h rühren. Die Reaktionslösung wird filtriert mit Essigsäureethylester versetzt und anschließend, mit 5% wässriger Natriumhydrogencarbonatlösung und wässriger Natriumchloridlösung extrahiert. Nach Trocknung der organischen Phase mit Chromabond XTR wird unter vermindertem Druck eingeengt und der Rückstand über HPLC (Knauer Eurospher-100-10-C18, Wasser (0.1 % Trifluoressigsäure)/Acetonitril (0.1% Trifluoressigsäure) = 80/20 -> 10/90) getrennt. Man erhält das gewünschte Produkt mit MG = 598.16 (berechnet); Meßwert (M-C₂H₆O+H)⁺: 552.1

### b) 6-(4-Chlor-benzyl)-8-isopropyl-1-(4-methoxy-benzolsulfonyl)-hexahydro-pyrazino[1,2-a]pyrimidin-4,7-dion

Eine Lösung von 167 mg 3-(4-Chlor-phenyl)-N-(2,2-diethoxy-ethyl)-N-isopropyl-2-[3-(4-methoxy benzolsulfonylamino)-propionylamino]-propionamid in 3 ml Ameisensäure wird 12 h bei Raumtemperatur gerührt. Die Reaktionslösung wird unter vemindertem Druck eingeengt und der Rückstand über HPLC (Knauer Eurospher-100-10-C18, Wasser (0.1 % Trifluoressigsäure)/Acetonitril (0.1 % Trifluoressigsäure) = 80/20 -> 10/90) getrennt. Man erhält das gewünschte Produkt mit MG = 506.02 (berechnet); Meßwert (M+H)⁺: 506.34

### Beispiel 77

### 1-(4-Chlor-benzolsulfonyl)-6-(4-chlor-benzyl)-8-isopropyl-hexahydro-pyrazino[1,2-a]pyrimidin-4,7-dion

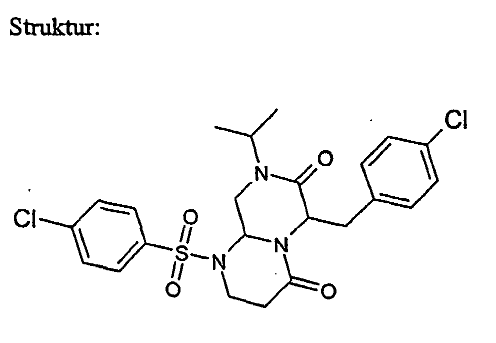

### a) 3-(4-Chlor-phenyl)-N-(2,2-diethoxy-ethyl)-N-isopropyl-2-[3-(4-chlor-benzolsulfonylamino)-propionylamino]-propionamid

Die Synthese erfolgt analog zu Beispiel 76a) ausgehend von 3-(4-Chlor-benzolsulfonylamino)-propionsäure. Man erhält das gewünschte Produkt mit MG = 602.58 (berechnet); Meßwert (M-C₂H₆O+H)⁺: 556.1

### b) 6-(4-Chlor-benzyl)-8-isopropyl-1-(4-chlor-benzolsulfonyl)-hexahydro-pyrazino[1,2-a]pyrimidin-4,7-dion

Die Synthese erfolgt analog zu Beispiel 76b) ausgehend von 3-(4-Chlor-phenyl)-N-(2,2-diethoxy-ethyl)-N-isopropyl-2-[3-(4-chlor- benzolsulfonylamino)-propionylamino]-propionamid. Man erhält das gewünschte Produkt mit MG = 510.44 (berechnet); Meßwert (M+H)⁺: 510.30

### Beispiel 78

### 6-(4-Chlor-benzyl)-1-(3,4-dimethoxy-benzolsulfonyl)-8-isopropyl-hexahydro-pyrazino[1,2-a]pyrimidin-4,7-dion

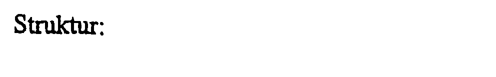

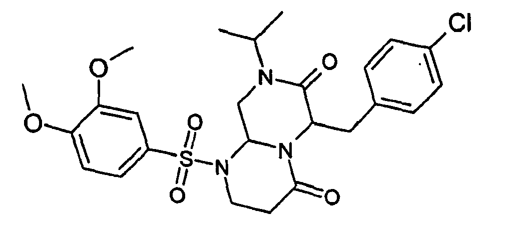

### a) 3-(3,4-Dimethoxy-benzolsulfonylamino)-propionsäure

Zu einer Lösung von 1.5 g 3-Aminopropionsäure in 20 mL 1N NaOH-Lösung wird eine Lösung aus 3.8 g 3,4-Dimethoxy-benzolsulfonylchlorid in 5 mL Dioxan getropft. Unter pH-Kontrolle (pH>7) lässt man 12 h rühren, senkt den pH unter 7 durch Zugabe von Zitronensäure und extrahiert die Reaktionslösung anschließend mit Methylenchlorid. Die organische Phase wird über Magnesiumsulfat getrocknet, unter vermindertem Druck eingeengt und ohne weitere Reinigung im nächsten Reaktionsschritt eingesetzt. Man erhält das gewünschte Produkt mit MG = 289.31 (berechnet); Meßwert (M+H)⁺: 290.1

### b) 3-(3,4-Dimethoxy-phenyl)-N-(2,2-diethoxy-ethyl)-N-isopropyl-2-[3-(4-chlor-benzolsulfonylamino)-propionylamino]-propionamid

Die Synthese erfolgt analog zu Beispiel 76a) ausgehend von 3-(3,4-Dimethoxybenzolsulfonylamino)-propionsäure. Man erhält das gewünschte Produkt mit MG = 628.19 (berechnet); Meßwert (M+H)⁺: 582.3

### c) 6-(4-Chlor-benzyl)-1-(3,4-dimethoxy-benzolsulfonyl)-8-isopropyl-hexahydro-pyrazino[1,2-a]pyrimidin-4,7-dion

Die Synthese erfolgt analog zu Beispiel 76b) ausgehend von 3-(3, 4-Dimethoxy-phenyl)-N-(2,2-diethoxy-ethyl)-N-isopropyl-2-[3-(4-chlor- benzolsulfonylamino)-propionylamino]-propionamid . Man erhält das gewünschte Produkt mit MG = 536.05 (berechnet); Meßwert (M+H)⁺: 536.36

### Beispiel 79

### 1-(3-Chlor-benzolsulfonyl)-6-(4-chlor-benzyl)-8-isopropyl-hexahydro-pyrazino[1,2-a]pyrimidin-4,7-dion

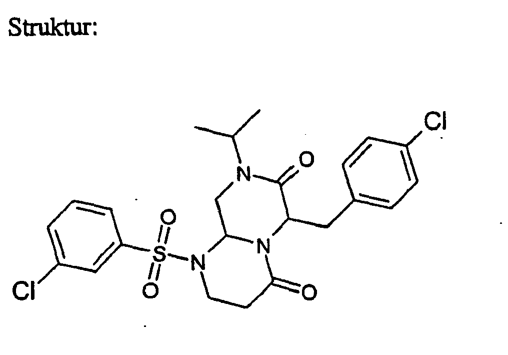

### a) 3-(2-Chlor-benzolsulfonylamino)-propionsäure

Die Synthese erfolgt analog zu Beispiel 78a) ausgehend von 2-Chlor-benzolsulfonyl chlorid. Man erhält das gewünschte Produkt mit MG = 263.70 (berechnet); Meßwert (M+H)⁺: 264.05

### b) 3-(4-Chlor-phenyl)-N-(2,2-diethoxy-ethyl)-N-isopropyl-2-[3-(2-chlor-benzolsulfonylamino)-propionylamino]-propionamid

Die Synthese erfolgt analog zu Beispiel 76a) ausgehend von 3-(2-Chlor-benzolsulfonylamino)-propionsäure. Man erhält das gewünschte Produkt mit Produkt mit MG = 602.58 (berechnet); Meßwert (M-C₂H₆O+H)⁺: 556.7

### c) 1-(3-Chlor-benzolsulfonyl)-6-(4-chlor-benzyl)-8-isopropyl-hexahydro-pyrazino[1,2-a]pyrimidin-4,7-dion

Die Synthese erfolgt analog zu Beispiel 76b) ausgehend von 3-(4-Chlor-phenyl)-N-(2,2-diethoxy-ethyl)-N-isopropyl-2-[3-(2-chlor-benzolsulfonylamino)-propionylamino]-propionamid. Man erhält das gewünschte Produkt mit MG = 510.44 (berechnet); Meßwert (M+H)⁺: 510.10

### Beispiel 80

### 6-(4-Chlor-benzyl)-1-(4-fluor-benzolsulfonyl)-8-isopropyl-hexahydro-pyrazino[1,2-a]pyrimidin-4,7-dion

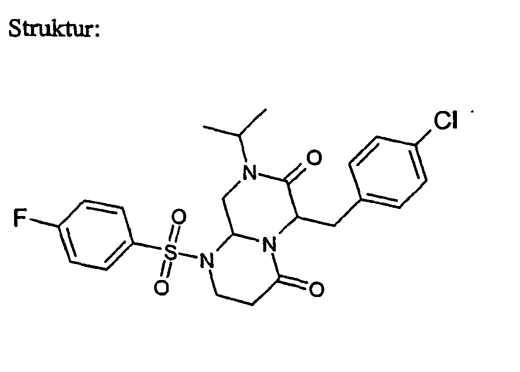

### a) 3-(4-Chlor-phenyl)-N-(2,2-diethoxy-ethyl)-N-isopropyl-2-[3-(4-fluor-benzolsulfonylamino)-propionylamino]-propionamid

Die Synthese erfolgt analog zu Beispiel 78a) ausgehend von 3-(4-Fluor-benzolsulfonylamino)-propionsäure. Man erhält das gewünschte Produkt mit MG = 586.13 (berechnet); Meßwert (M-C₂H₆O+H)⁺: 540.7

### b) 6-(4-Chlor-benzyl)-8-isopropyl-1-(4-Fluor-benzolsulfonyl)-hexahydro-pyrazino[1,2-a]pyrimidin-4,7-dion

Die Synthese erfolgt analog zu Beispiel 76b) ausgehend von 3-(4-Chlor-phenyl)-N-(2,2-diethoxy-ethyl)-N-isopropyl-2-[3-(4-fluor- benzolsulfonylamino)-propionylamino]-propionamid . Man erhält das gewünschte Produkt mit MG = 493.99 (berechnet); Meßwert (M+H)⁺: 494.13

### Beispiel 81

### 6-(4-Chlor-benzyl)-1-(2,5-dimethoxy-benzolsulfonyl)-8-isopropyl-hexahydro-pyrazino[1,2-a]pyrimidin-4,7-dion

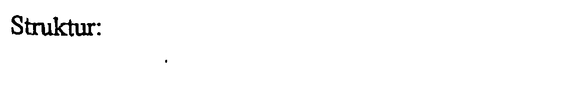

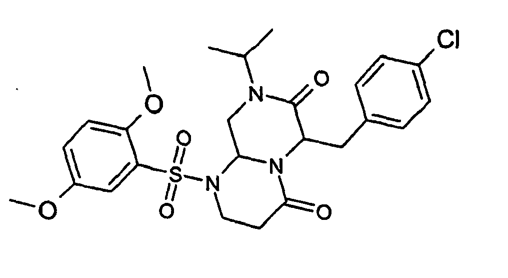

### a) 3-(2,5-Dimethoxy-benzolsulfonylamino)-propionsäure

Die Synthese erfolgt analog zu Beispiel 78a) ausgehend von 2,5-Dimethoxy-benzolsulfonyl chlorid. Man erhält das gewünschte Produkt mit MG = 289.31 (berechnet); Meßwert (M+H)⁺: 290.1

### b) 3-(2,5-Dimethoxy-phenyl)-N-(2,2-diethoxy-ethyl)-N-isopropyl-2-[3-(4-chlor-benzolsulfonylamino)-prvpionylamino]-propionamid

Die Synthese erfolgt analog zu Beispiel 76a) ausgehend von 3-(2,5-Dimethoxy-benzolsulfonylamino)-propionsäure. Man erhält das gewünschte Produkt mit MG = 628.19 (berechnet); Meßwert (M-C₂H₆O+H)⁺: 582.7

### c) 6-(4-Chlor-benzyl)-1-(2,5-dimethoxy-benzolsulfonyl)-8-isopropyl-hexahydro-pyrazino[1,2- a]pyrimidin-4,7-dion

Die Synthese erfolgt analog zu Beispiel 76b) ausgehend von 3-(2,5-Dimethoxy-phenyl)-N-(2,2-diethoxy-ethyl)-N-isopropyl-2-[3-(4-chlor- benzolsulfonylamino)-propionylamino]-propionamid . Man erhält das gewünschte Produkt mit MG = 536.05 (berechnet); Meßwert (M+H)⁺: 536.16

### Beispiel 82

### 6-(4-Chlor-benzyl)-1-(3-fluor-benzolsulfonyl)-8-isopropyl-hexahydro-pyrazino[1,2-a]pyrimidin-4,7-dion

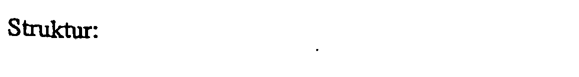

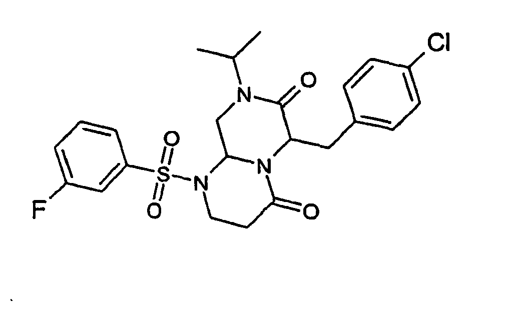

### a) 3-(3-Fluor-benzolsulfonylamino)-propionsäure

Die Synthese erfolgt analog zu Beispiel 78a) ausgehend von 3-Fluor-benzolsulfonyl chlorid. Man erhält das gewünschte Produkt mit MG = 247.25 (berechnet); Meßwert (M+H)⁺: 248.05

### b) 3-(4-Chlor-phenyl)-N-(2,2-diethoxy-ethyl)-N-isopropyl-2-[3-(3-fluor-benzolsulfonylamino)-propionylamino]-propionamid

Die Synthese erfolgt analog zu Beispiel 76a) ausgehend von 3-(3-Fluor-benzolsulfonylamino)-propionsäure. Man erhält das gewünschte Produkt mit MG = 586.13 (berechnet); Meßwert (M-C₂H₆O+H)⁺: 540.7

### c) 6-(4-Chlor-benzyl)-8-isopropyl-1-(3-Fluor-benzolsulfonyl)-hexahydro-pyrazino[1,2-a]pyrimidin-4,7-dion

Die Synthese erfolgt analog zu Beispiel 76b) ausgehend von 3-(4-Chlor-phenyl)-N-(2,2-diethoxy-ethyl)-N-isopropyl-2-[3-(3-fluor- benzolsulfonylamino)-propionylamino]-propionamid. Man erhält das gewünschte Produkt mit MG = 493.99 (berechnet); Meßwert (M+H)⁺: 494.25

### Beispiel 83

### 1-Benzolsulfonyl-6-(4-chlor-benzyl)-8-isopropyl-hexahydro-pyrazino[1,2-a]pyrimidin- 4,7-dion

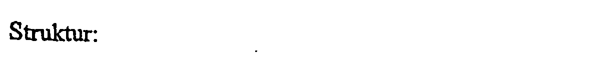

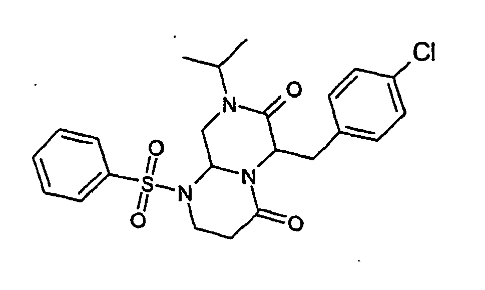

### a) 3-(4-Chlor-phenyl)-N-(2,2-diethoxy-ethyl)-N-isopropyl-2-[3-(benzolsulfonylamino)-propionylamino]-propionamid

Die Synthese erfolgt analog zu Beispiel 78a) ausgehend von 3-(Benzolsulfbnylamino)-propionsäure. Man erhält das gewünschte Produkt mit MG = 568.13 (berechnet); Meßwert (M-C₂H₆O+H)⁺: 522.7

### b) 6-(4-Chlor-benzyl)-8-isopropyl-1-(benzolsulfonyl)-hexahydro-pyrazino[1,2-a]pyrimidin-4,7-dion

Die Synthese erfolgt analog zu Beispiel 76b) ausgehend von 3-(4-Chlor-phenyl)-N-(2,2-diethoxy-ethyl)-N-isopropyl-2-[3-(benzolsulfonylamino)-propionylamino]-propionamid. Man erhält das gewünschte Produkt mit MG = 475.99 (berechnet); Meßwert (M+H)⁺: 476.14

### Beispiel 84

### 6-(4-Chlor-benzyl)-1-(2-fluor-benzolsulfonyl)-8-isopropyl-hexahydro-pyrazino[1,2-a]pyrimidin-4,7-dion

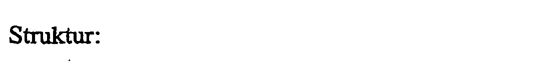

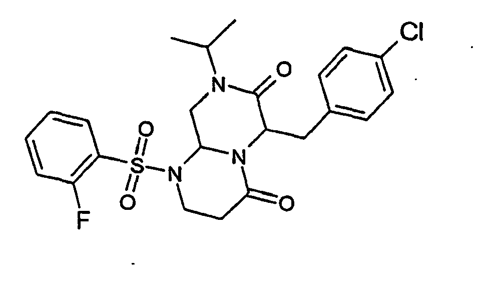

### a) 3-(2-Fluor-benzolsulfonylamino)-propionsäure

Die Synthese erfolgt analog zu Beispiel 78a) ausgehend von 2-Fluor-benzolsulfonyl chlorid. Man erhält das gewünschte Produkt mit MG = 247.25 (berechnet); Meßwert (M+H)⁺: 248.05

### b) 3-(4-Chlor-phenyl)-N-(2,2-diethoxy-ethyl)-N-isopropyl-2-[3-(2-fluor-benzolsulfonylamino)-propionylamino]-propionamid

Die Synthese erfolgt analog zu Beispiel 76a) ausgehend von 3-(2-Fluor-benzolsulfonylamino)-propionsäure. Man erhält das gewünschte Produkt mit MG = 586.13 (berechnet); Meßwert (M-C₂H₆O+H⁺: 540.7

### c) 6-(4-Chlor-benzyl)-8-isopropyl-1-(2-Fluor-benzolsulfonyl)-hexahydro-pyrazino[1,2-a]pyrimidin-4,7-dion

Die Synthese erfolgt analog zu Beispiel 76b) ausgehend von 3-(4-Chlor-phenyl)-N-(2,2-diethox-y-ethyl)-N-isopropyl-2-[3-(2-fluor- benzolsulfonylamino)-propionylamino]-propionamid . Man erhält das gewünschte Produkt mit MG = 493.99 (berechnet); Meßwert (M+H)⁺: 494.13

### Beispiel 85

### 6-(4-Chlor-benzyl)-1-(2,4-difluor-benzolsulfonyl)-8-isopropyl-hexahydro-pyrazino[1,2-a]pyrimidin-4,7-dion

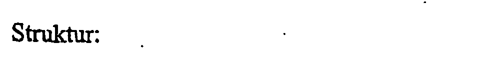

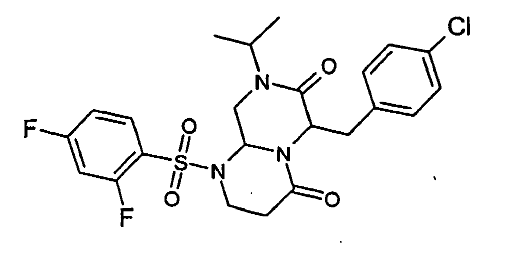

### a) 3-(2,4-Difluor-benzolsulfonylamino)-propionsäure

Die Synthese erfolgt analog zu Beispiel 78a) ausgehend von 2,4-Difluor-benzolsulfonyl chlorid. Man erhält das gewünschte Produkt mit MG = 265.25 (berechnet); Meßwert (M+H)⁺: 266.05

### b) 3-(4-Chlor-phenyl)-N-(2,2-diethoxy-ethyl)-N-isopropyl-2-[3-(2,4-difluor-benzolsulfonylamino)-propionylamino]-propionamid

Die Synthese erfolgt analog zu Beispiel 76a) ausgehend von 3-(2,4-Difluor-benzolsulfonylamino)-propionsäure. Man erhält das gewünschte Produkt mit MG = 604.13 (berechnet); Meßwert (M-C₂H₆O+H)⁺: 558.7

### c) 6-(4-Chlor-benzyl)-8-isopropyl-1-(2,4-Difluor-benzolsulfonyl)-hexahydro-pyrazino[1,2-a]pyrimidin-4,7-dion

Die Synthese erfolgt analog zu Beispiel 76b) ausgehend von 3-(4-Chlor-phenyl)-N-(2,2-diethoxy-ethyl)-N-isopropyl-2-[3-(2,4-difluor- benzolsulfonylamino)-propionylamino]-propionamid . Man erhält das gewünschte Produkt mit MG = 511.99 (berechnet); Meßwert (M+H)⁺: 512.12

### Beispiel 86

### 6-(4-Chlor-benzyl)-1-(3,4-difluor-benzolsulfonyl)-8-isopropyl-hexahydro-pyrazino[1,2-a]pyrimidin-4,7-dion

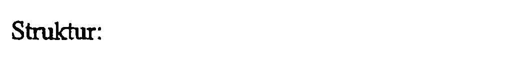

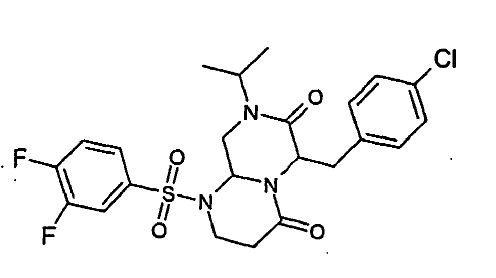

### a) 3-(3,4-Difluor-benzolsulfonylamino)-propionsäure

Die Synthese erfolgt analog zu Beispiel 78a) ausgehend von 3,4-Difluor-benzolsulfonyl chlorid. Man erhält das gewünschte Produkt mit MG = 265.25 (berechnet); Meßwert (M+H)⁺: 266.05

### b) 3-(4-Chlor-phenyl)-N-(2,2-diethoxy-ethyl)-N-isopropyl-2-[3-(3,4-difluor-benzolsulfonylamino)-propionylamino]-propionamid

Die Synthese erfolgt analog zu Beispiel 76a) ausgehend von 3-(3,4-Difluor-benzolsulfonylamino)-propionsäure. Man erhält das gewünschte Produkt mit MG = 604.13 (berechnet); Meßwert (M+H)⁺: 558.7

### c) 6-(4-Chlor-benzyl)-8-isopropyl-1-(3,4-Difluor-benzolsulfonyl)-hexahydro-pyrazino[1,2-a]pyrimidin-4,7-dion

Die Synthese erfolgt analog zu Beispiel 76b) ausgehend von 3-(4-Chlor-phenyl)-N-(2,2-diethoxy-ethyl)-N-isopropyl-2-[3-(3,4-difluor- benzolsulfonylamino)-propionylamino]-propionamid . Man erhält das gewünschte Produkt mit MG = 511.99 (berechnet); Meßwert (M+H)⁺: 512.12

## Patentansprüche

1. Verbindungen der Formel I, 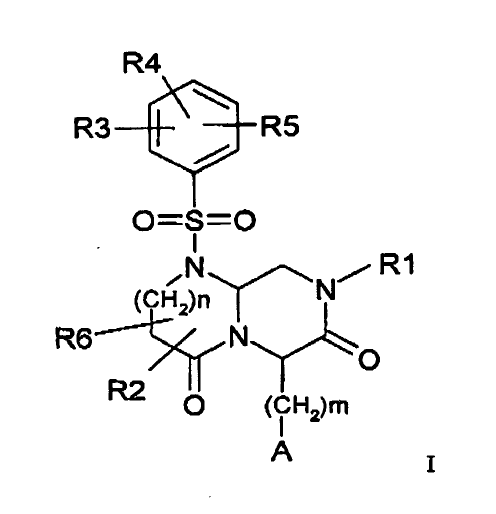 worin bedeuten
A 3-12 gliedriger mono-, bi- oder spirobicyclischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)-Alkyl oder COO-(C₁-C₆)-Alkyl tragen kann;
R11, R12, R13, R14, R15 unabhängig voneinander H, (C₁-C₆)-Alkyl, Heterocyclus;
n 1;
m 0, 1, 2, 3, 4, 5, 6;
R1 R8, (C₁-C₆)-Alkylen-R8, (C₂-C₆)-Alkenylen-R9, (S₂)-R8, (SO₂)-(C₁-C₆)- Alkylen-R8, (SO₂)-(C₂-C₆)-Alkenylen-R9, (C=O)-R8, (C=O)-(C₁-C₆)-Alkylen- R8, (C=O)NH-R8, (C=O)-(C₂-C₆)-Alkenylen-R9, (C=O)-NH- (C₁-C₆)- Alkylen-R8, (C=O)-NH- (C₂-C₆)-Alkenylen-R9, COO-R8, COO-(C₁-C₆)- Alkylen-R8, COO-(C₂-C₆)-Alkenylen-R9, Alkinylen-R9, (C₁-C₄-Alkyl)- Heterocyclus, wobei die Alkylengruppen mit F substituiert sein könne;
R8, R9 unabhängig voneinander H, F, Cl, Br, I, OH, CF₃, Aryl, Heterocyclus, (C₃-C₈)- Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)- Alkyl, NH₂, CON(R11)(R12), N(R13)(R14), SO₂-CH₃, COOH, COO-(C₁-C₆)- Alkyl, CONH₂;
R2 H, F, Cl, Br, J, OH, CF₃, CN, OCF₃, O-(C₁-C₆)-Alkyl, O-(C₁-C₄)-Alkoxy-(C₁- C₄)-alkyl, S-(C₁-C₆)-Alkyl, (C₁-C₆)-Alkyl, (C₂-C₆)-Alkenyl, (C₃-C₈)- Cycloalkyl, O-(C₃-C₈)-Cycloalkyl, (C₃-C₈)Cycloalkenyl, O-(C₃-C₈)- Cycloalkenyl, (C₂-C₆)-Alkinyl, Aryl, O-Aryl, (C₁-C₈)-Alylen-Aryl, O-(C₁-C₈)- AlkylenAryl, S-Aryl, CON(R11)(R12), (C₁-C₆)-Alkyl-N(R13)(R14), COOH, COO-(C₁-C₆)-Alkyl, COO-(C₁-C₆)-Alkenyl, CO-N((C₁-C₆)Alkyl)₂, Heterocyclus, wobei der Heterocyclus nicht über ein Stickstoffatom gebunden sein darf;
R3, R4, R5 unabhängig voneinander H, F, Cl, Br, J, OH, CF₃, NO₂, CN, OCF₃, -(C₁-C₆)- Alkyl, O-(C₁-C₄)-Alkoxy-(C₁-C₄)-alkyl, S-(C₁-C₆)-Alkyl, (C₁-C₆)-Alkyl, (C₂- C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, Aryl, O-Aryl (C₁-C₈)-Alkylen-Aryl, O-(C₁-C₈)-Alkylen-Aryl, S-Aryl, N((C₁-C₆)-Alkyl)₂, SO₂-CH₃, COOH, COO- (C₁-C₆)-Alkyl, CO-N((C₁-C₆)-Alkyl)₂;
R6 H, F, Cl, Br, J, OH, CF₃, NO₂, CN, OCF₃, O-(C₁-C₆)-Alkyl, O-(C₁₋₄)- Alkoxy-(C₁-C₄)-alkyl, S-(C₁-C₆)-Alkyl, (C₁-C₆)-Alkyl, (C₂-C₆)-Alkenyl, (C₃- C₈)-Cycloalkyl, O-(C₃-C₈)-Cycloalkyl, (C₃-C₈)-Cycloalkenyl, O-(C₃-C₈)- Cycloalkenyl, (C₂-C₆)-Alkinyl, (C₀-C₈)-Alkylen-Aryl, O-(C₀-C₈)-Alkylen-Aryl, S-Aryl, N((C₁-C₆)-Alkyl)₂, SO₂-CH₃, COOH, COO-(C₁-C₆)-Alkyl, CO-N((C₁- C₆)-Alkyl)₂;
sowie deren physiologisch verträglichen Salze;
mit der Maßgabe, dass die Verbindungen mit
a) R1= CH₂-Cyclohexyl, R2, R3, R4, R5, R6 = H, m =1, A = Phenyl und
b) R1 = 4-Cl-Phenyl, R2, R6 = H, m =1, A = Phenyl, R3, R4, R5 = H oder Methyl, wobei genau ein Rest aus R3, R4, R5 Methyl bedeutet,
ausgenommen sind.

2. Verbindungen der Formel I, gemäß Anspruch 1, **dadurch gekennzeichnet, daß** darin bedeuten
A 3-12 gliedriger mono-, bi- oder spirobicyclischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C₁-C₆)-Alkyl, S-(C₁-C₆)-Alkyl, N(R15)CO(C₁-C₆)Alkyl oder COO-(C₁-C₆)-Alkyl tragen kann;
R11, R12, R13, R14, R15 unabhängig voneinander H, (C₁-C₆)-Alkyl, Heterocyclus;
m 1;
n 1;
R1 R8, (C₁-C₆)-Alkylen-R8, (C₂-C₆)-Alkenylen-R9, (SO₂)-R8, (SO₂)-(C₁-C₆)- Alkylen-R8, (SO₂)-(C₂-C₆)-Alkenylen-R9, (C=O)-R8, (C=O)-(C₁-C₆)-Alkylen- R8, (C=O)NH-R8, (C=O)-(C₂-C₆)-Alkenylen-R9, (C=O)-NH- (C₁-C₆)- Alkylen-R8, (C=O)-NH- (C₁-C₆)-Alkenylen-R9, COO-R8, COO-(C₁-C₆)- Alkylen-R8, COO-(C₂-C₆)-Alkenylen-R9, Alkinylen-R9, (C₁-C₄-Alkyl)- Heterocyclus;
R8, R9 unabhängig voneinander H, F, Cl, Br, I, OH, CF₃, Aryl, Heterocyclus, (C₃-C₈)- Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)- Alkyl, NH₂, CON(R11)(R12), N(R13)(R14), SO₂-CH₃, COOH, COO-(C₁-C₆)- Alkyl, CONH₂;
R2 H, F, Cl, Br, J, OH, CF₃, CN, OCF₃, O-(C₁-C₆)-Alkyl, O-(C₁-C₄)-Alkoxy-(C₁- C₄)-alkyl, S-(C₁-C₆)-Alkyl, (C₁-C₆)-Alkyl, (C₂-C₆)-Alkenyl, (C₃-C₈)- Cycloalkyl, O-(C₃-C₈)-Cycloalkyl, (C₃-C₈)-Cycloalkenyl, O-(C₃-C₈)- Cycloalkenyl, (C₂-C₆)-Alkinyl, Aryl, O-Aryl, (C₁-C₈)-Alkylen-Aryl, O-(C₁-C₈)- Alkylen-Aryl, S-Aryl, CON(R11)(R12), (C₁-C₆)-Alkyl-N(R13)(R14), COOH, COO-(C₁-C₆)-Alkyl, COO-(C₂-C₆)-Alkenyl, CO-N((C₁-C₆)-Alkyl)₂, Heterocyclus, wobei der Heterocyclus nicht über ein Stickstoffatom gebunden sein darf;
R3, R4, R5 unabhängig voneinander H, F, Cl, Br, J, OH, CF₃, NO₂, CN, OCF₃, O-(C₁-C₆)- Alkyl, O-(C₁-C₄)-Alkoxy-(C₁-C₄)-alkyl, S-(C₁-C₆)-Alkyl, (C₁-C₆)-Alkyl, (C₂- C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, Aryl, O-Aryl (C₀-C₈)-Alkylen-Aryl, O-(C₀-C₈)-Alkylen-Aryl, S-Aryl, N((C₁-C₆)-Alkyl)₂, SO₂-CH₃, COOH, COO- (C₁-C₆)-Alkyl, CO-N((C₁-C₆)-Alkyl)₂;
R6 H, F, Cl, Br, J, OH, CF₃, NO₂, CN, OCF₃, O-(C₁-C₆)-Alkyl, O-(C₁-C₄)- Alkoxy-(C₁-C₄)-alkyl, S-(C₁-C₆)-Alkyl, (C₁-C₆)-Alkyl, (C₂-C₆)-Alkenyl, (C₃- C₈)-Cycloalkyl, O-(C₃-C₈)-Cycloalkyl, (C₃-C₈)-Cycloalkenyl, O-(C₃-C₈)- Cycloalkenyl, (C₂-C₆)-Alkinyl, Aryl, O-Aryl, (C₁-C₈)-Alkylen-Aryl, O-(C₁-C₈)- Alkylen-Aryl, S-Aryl, N((C₁-C₆)-Alkyl)₂, SO₂-CH₃, COOH, COO-(C₁-C₆)- Alkyl, CO-N(C₁-C₆)-Alkyl)₂;
sowie deren physiologisch verträglichen Salze.

3. Verbindungen der Formel I, gemäß Anspruch 1 oder 2, **dadurch gekennzeichnet, daß** darin bedeuten
A Aryl, wobei der Arylring substituiert sein kann mit F, Cl, Br, NO₂, CF₃, OCF₃, CN, (C₁-C₆)-Alkyl, Aryl, CON(R11)(R12), N(R13)(R14), OH, O-(C₁-C₆)- Alkyl, S-(C₁-C₆)-Alkyl, N(R15)CO(C₁-C₆)-Alkyl oder COO-(C₁-C₆)-Alkyl;
R11, R12, R13, R14, R15 unabhängig voneinander H, (C₁-C₆)-Alkyl;
m 1;
R1 (C₁-C₆)-Aelen-R8, (C₂-C₆)-Alkenylen-R9;
R8, R9 unabhängig voneinander H, F, Cl, Br, I, OH, CF₃, Aryl, Heterocyclus, (C₃-C₈)- Cycloalkyl, wobei die Ringe oder Ringssysteme bis zu 3-fach substituiert sein können mit F, Cl, Br, I, OH, CF₃, NO₂, CN, OCF₃, O-(C₁-C₆)-Alkyl, (C₁-C₆)- Alkyl, NH₂, CON(R11)(R12), N(R13)(R14), SO₂-CH₃, COOH, COO-(C₁-C₆)- Alkyl, CONH₂;
R2 H, F, Cl, Br, J, OH, CF₃, CN, OCF₃, O-(C₁-C₆)-Alkyl, (C₁-C₆)-Alkyl, COO- (C₁-C₆)-Alkenyl, (C₁-C₆)-Alkyl-N(R13)(R14);
R3 H
R4, R5 unabhängig voneinander H, F, Cl, Br, OH, CF₃, OCF₃, O-(C₁-C₆)-Akyl, (C₁- C₆)-Alkyl;
R6 H;
sowie deren physiologisch verträglichen Salze.

4. Verbindungen gemäß einem oder mehreren der Ansprüche 1 bis 3, **dadurch gekennzeichnet, daß** darin bedeuten
A Aryl, wobei der Arylring substituiert sein kann mit F, Cl, Br, NO₂, CF₃, OCF₃, CN, (C₁-C₆)-Alkyl, Aryl, CON(R11)(R12), N(R13)(R14), OH, O-(C₁-C₆)- Alkyl, S-(C₁-C₆)-Akyl, N(R15)CO(C₁-C₆)-Alkyl oder COO-(C₁-C₆)-Alkyl;
R11, R12, R13, R14, R15 unabhängig voneinander H, (C₁-C₆)-Alkyl;
m 1;
n 1;
R1 (C₁-C₆)-Alkyl, (C₂-C₆)-Alkenyl;
R2 H, OH, (C₁-C₆)-Alkyl, COO-(C₂-C₆)-Alkenyl, (C₁-C₆)-Alkyl-N(R13)(R14);
R3 H
R4 F, Cl, Br, OH, CF₃, OCF₃, O-(C₁-C₆)-Alkyl, (C₁-C₆)-Alkyl;
R5 H, F, Cl, Br, OH, CF₃, OCF₃, O-(C₁-C₆)-Alkyl, (C₁-C₆)-Alkyl;
R6 H;
sowie deren physiologisch verträglichen Salze.

5. Verbindungen gemäß einem oder mehreren der Ansprüche 1 bis 4 zur Anwendung als Arzneimittel.

6. Arzneimittel enthaltend eine oder mehrere der Verbindungen gemäß einem oder mehreren der Ansprüche 1 bis 4.

7. Arzneimittel enthaltend eine oder mehrere der Verbindungen gemäß einem oder mehreren der Ansprüche 1 bis 4 und ein oder mehrere anorektische Wirkstoffe.

8. Arzneimittel enthaltend eine oder mehrere der Verbindungen gemäß einem oder mehreren der Ansprüche 1 bis 4 und ein oder mehrere Statine.

9. Arzneimittel, gemäß Anspruch 6, **dadurch gekennzeichnet, daß** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MCH-Antagonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

10. Verbindungen gemäß einem oder mehreren der Ansprüche 1 bis 4 in Kombination mit mindestens einem weiteren anorektischen Wirkstoff zur Anwendung als Medikament zur Prophylaxe oder Behandlung der Adipositas.

11. Verbindungen gemäß einem oder mehreren der Ansprüche 1 bis 4 in Kombination mit mindestens einem weiteren anorektischen Wirkstoff zur Anwendung als Medikament zur Prophylaxe oder Behandlung des Typ II Diabetes.

12. Verfahren zur Herstellung eines Arzneimittels enthaltend eine oder mehrere der Verbindungen gemäß einem oder mehreren der Ansprüche 1 bis 4, **dadurch gekennzeichnet, dass** der Wirkstoff mit einem pharmazeutisch geeigneten Träger vermischt wird und diese Mischung in eine für die Verabreichung geeignete Form gebracht wird.

13. Verwendung der Verbindungen gemäß einem oder mehreren der Ansprüche 1 bis 4 oder Verbindungen der Formel I, 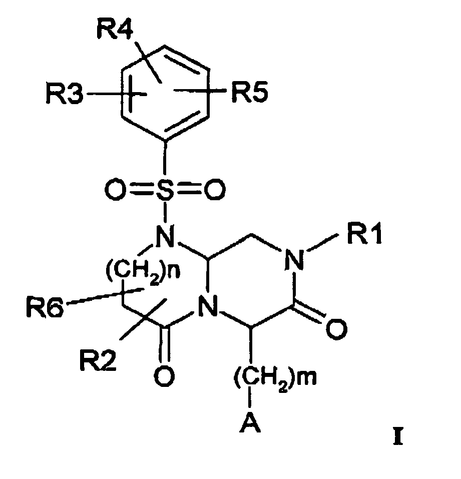 worin bedeuten
A 3-12 gliedriger mono-, bi- oder spirobieyelischer Ring, der ein oder mehrere Heteroatome aus der Gruppe N, O und S enthalten kann und der 3-12 gliedrige Ring weitere Substituenten wie F, Cl, Br, NO₂, CF₃, OCF₃, CN, (C₁-C₆)-Alkyl, Aryl, CON(R11)(R12), N(R13)(R14), OH, O-(C1-C₆)- Alkyl, S-(C₁-C₆)-Alkyl, N(R15)CO(C₁-C₆)-Alkyl oder COO-(C₁-C₆)-Alkyl tragen kann;
R11, R12, R13, R14, R15 unabhängig voneinander H, (C₁-C₆)-Alkyl, Heterocyclus;
n 0,1;
m 0, 1, 2, 3, 4, 5, 6;
R1 R8, (C₁-C₆)-Alkylen-R8, (C₂-C₆)-Alkenylen-R9, (SO₂)-R8, (SO₂)-(C₁-C₆)- Alkylen-R8, (SO₂)-(C₂-C₆)Alkenylen-R9, (C=O)-R8, (C=O)-(C₁-C₆)- Alkylen-R8, (C=O)NH-R8, (C=O)-(C₂-C₆)-Alkenylen-R9, (C=O)-NH- (C₁- C₆)-Alkylen-R8, (C=O)-NH-(C₂-C₆)-Alkenylen-R9, COO-R8, COO-(C₁- C₆)-Alkylen-R8, COO-(C₂-C₆)-Alkenylen-R9, Alkinylen-R9, (C₁-C₄-Alkyl)- Heterocyclus, wobei die Alkylengruppen mit F substituiert sein können;
R8, R9 unabhängig voneinander H, F, Cl, Br, I, OH, CF₃, Aryl, Heterocyclus, (C₃- C₈)-Cycloalkyl, wobei die Ringe oder Ringssysteme bis zu 3-fach substituiert sein können mit F, Cl, Br, I, OH, CF₃, NO₂, CN, OCF₃, O-(C₁- C₆)-Alkyl, (C₁-C₆)-Alkyl, NH₂, CON(R11)(R12), N(R13)(R14), SO₂-CH₃, COOH, COO-(C₁-C₆)-Alkyl, CONH₂;
R2 H, F, Cl, Br, J, OH, CF₃, CN, OCF₃, O-(C₁-C₆)-Alkyl, O-(C₁-C₄)-Alkoxy- (C₁-C₄)-alkyl, S-(C₁-C₆)-Alkyl, (C₁-C₆)-Alkyl, (C₂-C₆)-Alkenyl, (C₃-C₈)- Cycloalkyl, O-(C₃-C₈)-Cycloalkyl, (C₃-C₈)-Cycloalkenyl O-(C₃-C₈)- Cycloalkenyl, (C₂-C₆)-Alkinyl, Aryl, O-Aryl, (C₁-C₈)-Alkylen-Aryl, O-(C₁- C₈)-Alkylen-Aryl, S-Aryl, CON(R11)(R12), (C₁-C₆)-Alkyl-N(R13)(R14), COOH, COO-(C₁-C₆)-Alkyl, COO-(C₁-C₆)-Alkenyl, CO-N((C₁-C₆)-Alkyl)₂, Heterocyclus, wobei der Heterocyclus nicht über ein Stickstoffatom gebunden sein darf;
R3, R4, R5 unabhängig voneinander H, F, Cl, Br, J, OH, CF₃, NO₂, CN, OCF₃, O-(C₁- C₆)-Alkyl, O-(C₁-C₄)-Alkoxy-(C₁-C₄)alkyl, s-(C₁-C₆)-Alkyl, (C₁-C₆)-Alkyl, (C₂-C₆)-Alkenyl, (C₃-C₈)-Cycloalkyl, O-(C₃-C₈)-Cyeloalkyl, (C₃-C₈)- Cycloalkenyl, O-(C₃-C₈)-Cycloalkenyl, (C₂-C₆)-Alkinyl, Aryl, O-Aryl (C₁- C₈)-A1kylen-Aryl, O-(C₁-C₈)-Alkylen-Aryl, S-Aryl, N((C₁-C₆)Alkyl)₂, SO₂- CH₃, COOH, COO-(C₁-C₆)-Alkyl, CO-N((C₁-C₆)-Alkyl)₂;
R6 H, F, Cl, Br, J, OH, CF₃, NO₂, CN, OCF₃, O-(C₁-C₆)-Alkyl, O-(C₁-C₄)- Alkoxy-(C₁-C₄)-alkyl, S-(C₁-C₆)-Alkyl, (C₁-C₆)-Alkyl, (C₂-C₆)-Alkenyl, (C₃- C₈)-Cycloalkyl, O-(C₃-C₈)-Cycloalky), (C₃-C₈)-Cycloalkenyl, O-(C₃-C₈)- Cycloalkenyl, (C₂-C₆)-Alkinyl, (C₀-C₈)-Alkylen-Aryl, O-(C₀-C₈)-Alkylen- Aryl, S-Aryl, N((C₁-C₆)-Alkyl)₂, SO₂-CH₃, COOH, COO-(C₁-C₆)-Alkyl, CO-N((C₁-C₆)Alkyl)₂;
sowie deren physiologisch verträglichen Salze
zur Herstellung eines Medikaments zur Gewichtsreduktion bei Säugetieren.

14. Verwendung der Verbindungen gemäß einem oder mehreren der Ansprüche 1 bis 4 oder 13 zur Herstellung eines Medikaments zur Prophylaxe oder Behandlung der Adipositas.

15. Verwendung der Verbindungen gemäß einem oder mehreren der Ansprüche 1 bis 4 oder 13 zur Herstellung eines Medikaments zur Prophylaxe oder Behandlung des Typ II Diabetes.

16. Verwendung der Verbindungen gemäß einem oder mehreren der Ansprüche 1 bis 4 oder 13 zur Herstellung eines Medikaments zur Prophylaxe oder Behandlung des Metabolischen Syndroms.

17. Verwendung der Verbindungen gemäß einem oder mehreren der Ansprüche 1 bis 4 oder 13 zur Herstellung eines Medikaments zur Behandlung von weiblichen und männlichen Sexualstörungen.

## Claims

1. A compound of the formula I, 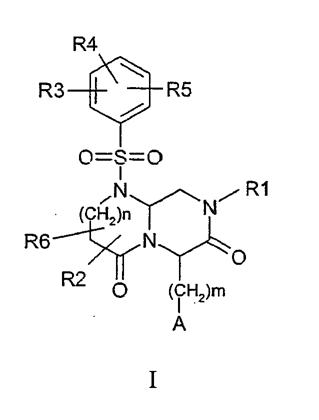 in which the meanings are
A 3-12 membered mono-, bi- or spirobicyclic ring which may comprise one or more heteroatoms from the group of N, O and S and the 3-12 membered ring may have further substituents such as F, Cl, Br, NO₂, CF₃, OCF₃, CN, (C₁-C₆)-alkyl, aryl, CON(R11)(R12), N(R13)(R14), OH, O-(C₁-C₆)-alkyl, S-(C₁-C₆)-alkyl, N(R15)CO(C₁- C₆)-alkyl or COO-(C₁-C₆)-alkyl;
R11, R12, R13, R14, R15 independently of one another H, (C₁-C₆)-alkyl, hetero- cycle;
n 1;
m 0, 1, 2, 3, 4, 5, 6;
R1 R8, (C₁-C₆)-alkylene-R8, (C₂-C₆)-alkenylene-R9, (SO₂)-R8 (SO₂)- (C₁-C₆)-alkylene-R8, (SO₂)-(C₂-C₆)-alkenylene-R9, (C=O)-R8, (C=O)-(C₁-C₆)-alkylene-R8, (C=O)NH-R8, (C=O)-(C₂-C₆)- alkenylene-R9, (C=O)-NH-(C₁-C₆)-alkylene-R8, (C=O)-NH-(C₂- C₆)-alkenylene-R9, COO-R8, COO-(C₁-C₆)-alkylene-R8, COO-(C₂- C₆)-alkenylene-R9, alkynylene-R9, (C₁-C₄-alkyl)-heterocycle, where the alkylene groups may be substituted by F;
R8, R9 independently of one another H, F, Cl, Br, I, OH, CF₃, aryl, heterocycle, (C₃-C₈)-cycloalkyl, where the rings or ring systems may be substituted up to 3 times by F, Cl, Br, I, OH, CF₃, NO₂, CN, OCF₃, O-(C₁-C₆)-alkyl, (C₁-C₆)-alkyl, NH₂, CON(R11)(R12), N(R13)(R14), SO₂-CH₃, COOH, COO-(C₁-C₆)-alkyl, CONH₂;
R2 H, F, Cl, Br, I, OH, CF₃, CN, OCF₃, O-(C₁-C₆)-alkyl, O-(C₁-C₄)- alkoxy-(C₁-C₄)-alkyl, S-(C₁-C₆)-alkyl, (C₁-C₆)-alkyl, (C₂-C₆)- alkenyl, (C₃-C₈)-cycloalkyl, O-(C₃-C₈)-cycloalkyl, (C₃-C₈)- cycloalkenyl, O-(C₃-C₈)-cycloalkenyl, (C₂-C₆)-alkynyl, aryl, O-aryl, (C₁-C₈)-alkylene-aryl, O-(C₁-C₈)-alkylene-aryl, S-aryl, CON(R11)(R12), (C₁-C₆)-alkyl-N(R13)(R14), COOH, COO-(C₁- C₆)-alkyl, COO-(C₁-C₆)-alkenyl, CO-N((C₁-C₆)-alkyl)₂, heterocycle, where the heterocycle may not be bonded via a nitrogen atom;
R3, R4, R5 independently of one another H, F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)- alkyl, (C₁-C₆)-alkyl, (C₂-C₆)-alkenyl, (C₃-C₈)-cycloalkyl, O-(C₃-C₈)- cycloalkyl, (C₃-C₈)-cycloalkenyl, O-(C₃-C₈)-cycloalkenyl, (C₂-C₆)- alkynyl, aryl, O-aryl (C₁-C₈)-alkylene-aryl, O-(C₁-C₈)-alkylene-aryl, S-aryl, N((C₁-C₆)-alkyl)₂, SO₂-CH₃, COOH, COO-(C₁-C₆)-alkyl, CO-N((C₁-C₆)-alkyl)₂;
R6 H, F, Cl, Br, I, OH, CF₃, NO₂, CN, OCF₃, O-(C₁-C₆)-alkyl, O-(C₁- C₄)-alkoxy-(Cᵢ-C₄)-alkyl, S-(C₁-C₆)-alkyl, (C₁-C₆)-alkyl, (C₂-C₆)- alkenyl, (C₃-C₈)-cycloalkyl, O-(C₃-C₈)-cycloalkyl, (C₃-C₈)- cycloalkenyl, O-(C₃-C₈)-cycloalkenyl, (C₂-C₆)-alkynyl, (C₀-C₈)- alkylene-aryl, O-(C₀-C₈)-alkylene-aryl, S-aryl, N((C₁-C₆)-alkyl)₂, SO₂-CH₃, COOH, COO-(C₁-C₆)-alkyl, CO-N((C₁-C₆)-alkyl)₂;
and the physiologically tolerated salts thereof;
with the proviso that the compounds where
a) R1 = CH₂-cyclohexyl, R2, R3, R4, R5, R6 = H, m = 1, A = phenyl and
b) R1 = 4-Cl-phenyl, R2, R6 = H, m = 1, A = phenyl, R3, R4, R5 = H or methyl, and precisely one radical from R3, R4, R5 is methyl,
are excluded.

2. A compound of the formula I as claimed in claim 1, wherein the meanings are
A 3-12 membered mono-, bi- or spirobicyclic ring which may comprise one or more heteroatoms from the group of N, O and S and the 3-12 membered ring may have further substituents such as F, Cl, Br, NO₂, CF₃, OCF₃, CN, (C₁-C₆)-alkyl, aryl, CON(R11)(R12), N(R13)(R14), OH, O-(C₁-C₆)-alkyl, S-(C₁-C₆)-alkyl, N(R15)CO(C₁-C₆)-alkyl or COO-(C₁-C₆)-alkyl;
R11, R12, R13, R14, R15 independently of one another H, (C₁-C₆)-alkyl, hetero- cycle;
m 1;
n 1;
R1 R8, (C₁-C₆)-alkylene-R8, (C₂-C₆)-alkenylene-R9, (SO₂)-R8, (SO₂)- (C₁-C₆)-alkylene-R8, (SO₂)-(C₂-C₆)-alkenylene-R9, (C=O)-R8, (C=O)-(C₁-C₆)-alkylene-R8, (C=O)NH-R8, (C=O)-(C₂-C₆)- alkenylene-R9, (C=O)-NH-(C₁-C₆)-alkylene-R8, (C=O)-NH-(C₂- C₆)-alkenylene-R9, COO-R8, COO-(C₁-C₆)-alkylene-R8, COO-(C₂- C₆)-alkenylene-R9, alkynylene-R9, (C₁-C₄-alkyl)-heterocycle;
R8, R9 independently of one another H, F, Cl, Br, I, OH, CF₃, aryl, heterocycle, (C₃-C₈)-cycloalkyl, where the rings or ring systems may be substituted up to 3 times by F, Cl, Br, I, OH, CF₃, NO₂, CN, OCF₃, O-(C₁-C₆)-alkyl, (C₁-C₆)-alkyl, NH₂, CON(R11)(R12), N(R13)(R14), SO₂-CH₃, COOH, COO-(C₁-C₆)-alkyl, CONH₂;
R2 H, F, Cl, Br, I, OH, CF₃, CN, OCF₃, O-(C₁-C₆)-alkyl, O-(C₁-C₄)- alkoxy-(C₁-C₄)-alkyl, S-(C₁-C₆)-alkyl, (C₁-C₆)-alkyl, (C₂-C₆)- alkenyl, (C₃₋C₈)-cycloalkyl, O-(C₃-C₈)-cycloalkyl, (C₃-C₈)- cycloalkenyl, O-(C₃-C₈)-cycloalkenyl, (C₂-C₆)-alkynyl, aryl, O-aryl, (C₁-C₈)-alkylene-aryl, O-(C₁-C₈)-alkylene-aryl, S-aryl, CON(R11)(R12), (C₁-C₆)-alkyl-N(R13)(R14), COOH, COO-(C₁- C₆)-alkyl, COO-(C₂-C₆)-alkenyl, CO-N((C₁-C₆)-alkyl)₂, heterocycle, where the heterocycle may not be bonded via a nitrogen atom;
R3, R4, R5 independently of one another H, F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)- alkyl, (C₁-C₆)-alkyl, (C₂-C₆)-alkenyl, (C₃-C₈)-cycloalkyl, O-(C₃-C₈)- cycloalkyl, (C₃-C₈)-cycloalkenyl, O-(C₃-C₈)-cycloalkenyl, (C₂-C₆)- alkynyl, aryl, O-aryl, (C₀-C₈)-alkylene-aryl, O-(C₀-C₈)-alkylene-aryl, S-aryl, N((C₁-C₆)-alkyl)₂, SO₂-CH₃, COOH, COO-(C₁-C₆)-alkyl, CO-N((C₁-C₆)-alkyl)₂;
R6 H, F, Cl, Br, I, OH, CF₃, NO₂, CN, OCF₃, O-(C₁-C₆)-alkyl, O-(C₁- C₄)-alkoxy-(C₁-C₄)-alkyl, S-(C₁-C₆)-alkyl, (C₁-C₆)-alkyl, (C₂-C₆)- alkenyl, (C₃-C₈)-cycloalkyl, O-(C₃-C₈)-cycloalkyl, (C₃-C₈)- cycloalkenyl, O-(C₃-C₈)-cycloalkenyl, (C₂-C₆)-alkynyl, aryl, O-aryl, (C₁-C₈)-alkylene-aryl, O-(C₁-C₈)-alkylene-aryl, S-aryl, N((C₁-C₆)- alkyl)₂, SO₂-CH₃, COOH, COO-(C₁-C₆)-alkyl, CO-N((C₁-C₆)- alkyl)₂;
and the physiologically tolerated salts thereof.

3. A compound of the formula I as claimed in claim 1 or 2, wherein the meanings are
A aryl, where the aryl ring may be substituted by F, Cl, Br, NO₂, CF₃, OCF₃, CN, (C₁-C₆)-alkyl, aryl, CON(R11)(R12), N(R13)(R14), OH, O-(C₁-C₆)-alkyl, S-(C₁-C₆)-alkyl, N(R15)CO(C₁-C₆)-alkyl or COO- (C₁-C₆)-alkyl;
R11, R12, R13, R14, R15 independently of one another H, (C₁-C₆)-alkyl;
m 1;
R1 (C₁-C₆)-alkylene-R8, (C₂-C₆)-alkenylene-R9;
R8, R9 independently of one another H, F, Cl, Br, I, OH, CF₃, aryl, heterocycle, (C₃-C₈)-cycloalkyl, where the rings or ring systems may be substituted up to 3 times by F, Cl, Br, I, OH, CF₃, NO₂, CN, OCF₃, O-(C₁-C₆)-alkyl, (C₁-C₆)-alkyl, NH₂, CON(R11)(R12), N(R13)(R14), SO₂-CH₃, COOH, COO-(C₁-C₆)-alkyl, CONH₂;
R2 H, F, Cl, Br, I, OH, CF₃, CN, OCF₃, O-(C₁-C₆)-alkyl, (C₁-C₆)-alkyl, COO-(C₂-C₆)-alkenyl, (C₁-C₆)-alkyl-N(R13)(R14);
R3 H;
R4, R5 independently of one another H, F, Cl, Br, OH, CF₃, OCF₃, O-(C₁- C₆)-alkyl, (C₁-C₆)-alkyl;
R6 H;
and the physiologically tolerated salts thereof.

4. A compound as claimed in one or more of claims 1 to 3, wherein the meanings are
A aryl, where the aryl ring may be substituted by F, Cl, Br, NO₂, CF₃, OCF₃, CN, (C₁-C₆)-alkyl, aryl, CON(R11)(R12), N(R13)(R14), OH, O-(C₁-C₆)-alkyl, S-(C₁-C₆)-alkyl, N(R15)CO(C₁-C₆)-alkyl or COO- (C₁-C₆)-alkyl;
R11, R12, R13, R14, R15 independently of one another H, (C₁-C₆)-alkyl;
m 1;
n 1;
R1 (C₁-C₆)-alkyl, (C₂-C₆)-alkenyl;
R2 H, OH, (C₁-C₆)-alkyl, COO-(C₂-C₆)-alkenyl, (C₁-C₆)-alkyl- N(R13)(R14);
R3 H;
R4 F, Cl, Br, OH, CF₃, OCF₃, O-(C₁-C₆)-alkyl, (C₁-C₆)-alkyl;
R5 H, F, Cl, Br, OH, CF₃, OCF₃, O-(C₁-C₆)-alkyl, (C₁-C₆)-alkyl;
R6 H;
and the physiologically tolerated salts thereof.

5. A compound as claimed in one or more of claims 1 to 4 for use as a medicament.

6. A medicament comprising one or more of the compounds as claimed in one or more of claims 1 to 4.

7. A medicament comprising one or more of the compounds as claimed in one or more of claims 1 to 4 and one or more anorectic active ingredients.

8. A medicament comprising one or more of the compounds as claimed in one or more of claims 1 to 4 and one or more statins.

9. A medicament as claimed in claim 6, wherein the other active ingredient comprises one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MCH antagonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

10. A compound as claimed in one or more of claims 1 to 4 in combination with at least one other anorectic active ingredient for use as medicament for the prophylaxis or treatment of obesity.

11. A compound as claimed in one or more of claims 1 to 4 in combination with at least one other anorectic active ingredient for use as medicament for the prophylaxis or treatment of type II diabetes.

12. A process for producing a medicament comprising one or more of the compounds as claimed in one or more of claims 1 to 4, which comprises mixing the active ingredient with a pharmaceutically suitable carrier and converting this mixture into a form suitable for administration.

13. The use of the compound as claimed in one or more of claims 1 to 4 or the compound of the formula I, 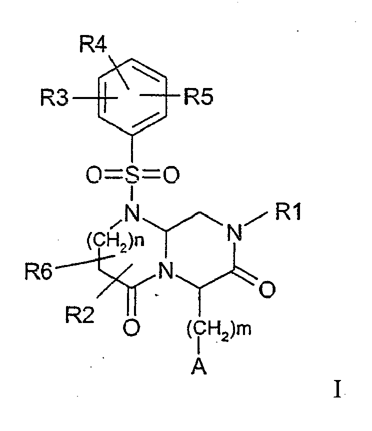 in which the meanings are
A 3-12 membered mono-, bi- or spirobicyclic ring which may comprise one or more heteroatoms from the group of N, O and S and the 3-12 membered ring may have further substituents such as F, Cl, Br, NO₂, CF₃, OCF₃, CN, (C₁-C₆)-alkyl, aryl, CON(R11)(R12), N(R13)(R14), OH, O-(C₁-C₆)-alkyl, S-(C₁-C₆)-alkyl, N(R15)CO(C₁- C₆)-alkyl or COO-(C₁-C₆)-alkyl;
R11, R12, R13, R14, R15 independently of one another H, (C₁-C₆)-alkyl, hetero- cycle;
n 0, 1;
m 0, 1, 2, 3, 4, 5, 6;
R1 R8, (C₁-C₆)-alkylene-R8, (C₂-C₆)-alkenylene-R9, (SO₂)-R8, (SO₂)- (C₁-C₆)-alkylene-R8, (SO₂)-(C₂-C₆)-alkenylene-R9, (C=O)-R8, (C=O)-(C₁-C₆)-alkylene-R8, (C=O)NH-R8, (C=O)-(C₂-C₆)- alkenylene-R9, (C=O)-NH-(C₁-C₆)-alkylene-R8, (C=O)-NH-(C₂- C₆)-alkenylene-R9, COO-R8, COO-(C₁-C₆)-alkylene-R8, COO-(C₂- C₆)-alkenylene-R9, alkynylene-R9, (C₁-C₄-alkyl)-heterocycle, where the alkylene groups may be substituted by F;
R8, R9 independently of one another H, F, Cl, Br, I, OH, CF₃, aryl, heterocycle, (C₃-C₈)-cycloalkyl, where the rings or ring systems may be substituted up to 3 times by F, Cl, Br, I, OH, CF₃, NO₂, CN, OCF₃, O-(C₁-C₆)-alkyl, (C₁-C₆)-alkyl, NH₂, CON(R11)(R12), N(R13)(R14), SO₂-CH₃, COOH, COO-(C₁-C₆)-alkyl, CONH₂;
R2 H, F, Cl, Br, I, OH, CF₃, CN, OCF₃, O-(C₁-C₆)-alkyl, O-(C₁-C₄)- alkoxy-(C₁-C₄)-alkyl, S-(C₁-C₆)-alkyl, (C₁-C₆)-alkyl, (C₂-C₆)- alkenyl, (C₃-C₈)-cycloalkyl, O-(C₃-C₈)-cycloalkyl, (C₃-C₈)- cycloalkenyl, O-(C₃-C₈)-cycloalkenyl, (C₂-C₆)-alkynyl, aryl, O-aryl, (C₁-C₈)-alkylene-aryl, O-(C₁-C₈)-alkylene-aryl, S-aryl, CON(R11)(R12), (C₁-C₆)-alkyl-N(R13)(R14), COOH, COO-(C₁- C₆)-alkyl, COO-(C₁-C₆)-alkenyl, CO-N((C₁-C₆)-alkyl)₂, heterocycle, where the heterocycle may not be bonded via a nitrogen atom;
R3, R4, R5 independently of one another H, F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)- alkyl, (C₁-C₆)-alkyl, (C₂-C₆)-alkenyl, (C₃-C₈)-cycloalkyl, O-(C₃-C₈)- cycloalkyl, (C₃-C₈)-cycloalkenyl, O-(C₃-C₈)-cycloalkenyl, (C₂-C₆)- alkynyl, aryl, O-aryl (C₁-C₈)-alkylene-aryl, O-(C₁-C₈)-alkylene-aryl, S-aryl, N((C₁-C₆)-alkyl)₂, SO₂-CH₃, COOH, COO-(C₁-C₆)-alkyl, CO-N((C₁-C₆)-alkyl)₂;
R6 H, F, Cl, Br, I, OH, CF₃, NO₂, CN, OCF₃, O-(C₁-C₆)-alkyl, O-(C₁- C₄)-alkoxy-(C₁-C₄)-alkyl, S-(C₁-C₆)-alkyl, (C₁-C₆)-alkyl, (C₂-C₆)- alkenyl, (C₃-C₈)-cycloalkyl, O-(C₃-C₈)-cycloalkyl, (C₃-C₈)- cycloalkenyl, O-(C₃-C₈)-cycloalkenyl, (C₂-C₆)-alkynyl, (C₀-C₈)- alkylene-aryl, O-(C₀-C₈)-alkylene-aryl, S-aryl, N((C₁-C₆)-alkyl)₂, SO₂-CH₃, COOH, COO-(C₁-C₆)-alkyl, CO-N((C₁-C₆)-alkyl)₂;
and the physiologically tolerated salts thereof
for producing a medicament for weight reduction in mammals.

14. The use of the compound as claimed in one or more of claims 1 to 4 or 13 for producing a medicament for the prophylaxis or treatment of obesity.

15. The use of the compound as claimed in one or more of claims 1 to 4 or 13 for producing a medicament for the prophylaxis or treatment of type II diabetes.

16. The use of the compound as claimed in one or more of claims 1 to 4 or 13 for producing a medicament for the prophylaxis or treatment of metabolic syndrome.

17. The use of the compound as claimed in one or more of claims 1 to 4 or 13 for producing a medicament for the treatment of female and male sexual disorders.

## Revendications

1. Composés de formule I, 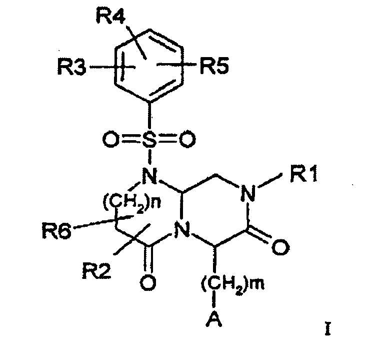 dans laquelle
A signifie un cycle monocyclique, bicyclique ou spirobicyclique de 3-12 chaînons, qui peut contenir un ou plusieurs hétéroatomes du groupe N, O et S et le cycle de 3-12 chaînons peut porter d'autres substituants, tels que F, Cl, Br, NO₂, CF₃, OCF₃, CN, (C₁-C₆)-alkyle, aryle, CON(R11)(R12), N(R13)(R14), OH, O-(C₁-C₆)-alkyle, S-(C₁-C₆)-alkyle, N(R15)CO(C₁-C₆)-alkyle ou COO-(C₁-C₆)-alkyle ;
R11, R12, R13, R14, R15 signifient, indépendamment l'un de l'autre H, (C₁-C₆)-alkyle, hétérocycle ;
n vaut 1 ;
m vaut 0, 1, 2, 3, 4, 5, 6 ;
R1 signifie R8, (C₁-C₆)-alkylène-R8, (C₂-C₆)- alcénylène-R9, (SO₂)-R8, (SO₂)-(C₁-C₆)-alkylène-R8, (SO₂)-(C₂-C₆)-alcénylène-R9, (C=O)-R8, (C=O)-(C₁- C₆)-alkylène-R8, (C=O)NH-R8, (C=O)-(C₂-C₆)- alcénylène-R9, (C=O)-NH-(C₁-C₆)-alkylène-R8, (C=O)- NH-(C₂-C₆)-alcénylène-R9, COO-R8, COO-(C₁-C₆)- alkylène-R8, COO-(C₂-C₆)-alcénylène-R9, alcynylène- R9, (C₁-C₄-alkyl)-hétérocycle, où les groupes alkylène peuvent être substitués par F ;
R8, R9 signifient indépendamment l'un de l'autre, H, F, Cl, Br, I, OH, CF₃, aryle, hétérocycle, (C₃-C₈)- cycloalkyle, où les cycles ou les systèmes cycliques peuvent être jusqu'à trisubstitués par F, Cl, Br, I, OH, CF₃, NO₂, CN, OCF₃, O-(C₁-C₆)- alkyle, (C₁-C₆)-alkyle, NH₂, CON(R11)(R12), N(R13)(R14), SO₂-CH₃, COOH, COO-(C₁-C₆)-alkyle, CONH₂ ;
R2 signifie H, F, Cl, Br, I, OH, CF₃, CN, OCF₃, O-(C₁- C₆)-alkyle, O-(C₁-C₄)-alcoxy-(C₁-C₄)-alkyle, S-(C₁- C₆)-alkyle, (C₁-C₆)-alkyle, (C₂-C₆)-alcényle, (C₃- C₈)-cycloalkyle, O-(C₃-C₈)-cycloalkyle, (C₃-C₈)- cycloalcényle, O-(C₃-C₈)-cycloalcényle, (C₂-C₆)- alcynyle, aryle, O-aryle, (C₁-C₈)-alkylène-aryle, O-(C₁-C₈)-alkylène-aryle, S-aryle, CON(R11)(R12), (C₁-C₆)-alkyl-N(R13)(R14), COOH, COO-(C₁-C₆)-alkyle, COO-(C₁-C₆)-alcényle, CO-N((C₁-C₆)-alkyle)₂, hétérocycle, où l'hétérocycle ne peut pas être lié via un atome d'azote ;
R3, R4, R5 signifient indépendamment l'un de l'autre H, F, Cl, Br, I, OH, CF₃, NO₂, CN, OCF₃, O- (C₁-C₆)-alkyle, O-(C₁-C₄)-alcoxy-(C₁-C₄)-alkyle, S- (C₁-C₆)-alkyle, (C₁-C₆)-alkyle, (C₂-C₆)-alcényle, (C₃-C₈)-cycloalkyle, O-(C₃-C₈)-cycloalkyle, (C₃-C₈)- cycloalcényle, O-(C₃-C₈)-cycloalcényle, (C₂-C₆)- alcynyle, aryle, O-aryle, (C₁-C₈)-alkylène-aryle, O-(C₁-C₈)-alkylène-aryle, S-aryle, N((C₁-C₆)- alkyle)₂, SO₂-CH₃, COOH, COO-(C₁-C₆)-alkyle, CO- N((C₁-C₆)-alkyle)₂ ;
R6 signifie H, F, Cl, Br, I, OH, CF₃, NO₂, CN, OCF₃, O-(C₁-C₆)-alkyle, O-(C₁-C₄)-alcoxy-(C₁-C₄)-alkyle, S-(C1-C₆)-alkyle, (C₁-C₆)-alkyle, (C₂-C₆)-alcényle, (C₃-C₈)-cycloalkyle, O-(C₃-C₈)-cycloalkyle, (C₃-C₈)- cycloalcényle, O-(C₃-C₈)-cycloalcényle, (C₂-C₆)- alcynyle, (C₀-C₈)-alkylène-aryle, O-(C₀-C₈)- alkylène-aryle, S-aryle, N((C₁-C₆)-alkyle)₂, SO₂- CH₃, COOH, COO-(C₁-C₆)-alkyle, CO-N((C₁-C₆)- alkyle)₂ ;
ainsi que leurs sels physiologiquement acceptables ;
à condition que les composés avec
a) R1 = CH₂-cyclohexyle, R2, R3, R4, R5, R6 = H, m = 1, A = phényle et
b) R1 = 4-Cl-phényle, R2, R6 = H, m = 1, A = phényle, R3, R4, R5 = H ou méthyle,
où exactement un radical parmi R3, R4, R5 signifie méthyle,
soient exclus.

2. Composés de formule I, selon la revendication 1, **caractérisés en ce que**
A signifie un cycle monocyclique, bicyclique ou spirobicyclique de 3-12 chaînons, qui peut contenir un ou plusieurs hétéroatomes du groupe N, O et S et le cycle de 3-12 chaînons peut porter d'autres substituants, tels que F, Cl, Br, NO₂, CF₃, OCF₃, CN, (C₁-C₆)-alkyle, aryle, CON(R11)(R12), N(R13) (R14), OH, O-(C₁-C₆)-alkyle, S-(C₁-C₆)-alkyle, N(R15)CO(C₁-C₆)-alkyle ou COO-(C₁-C₆)-alkyle ;
R11, R12, R13, R14, R15 signifient, indépendamment l'un de l'autre H, (C₁-C₆)-alkyle, hétérocycle ;
m vaut 1 ;
n vaut 1 ;
R1 signifie R8, (C₁-C₆)-alkylène-R8, (C₂-C₆)- alcénylène-R9, (SO₂)-R8, (SO₂)-(C₁-C₆)-alkylène-R8, (SO₂)-(C₂-C₆)-alcénylène-R9, (C=O)-R8, (C=O)-(C₁- C₆)-alkylène-R8, (C=O)NH-R8, (C=O)-(C₂-C₆)- alcénylène-R9, (C=O)-NH-(C₁-C₆)-alkylène-R8, (C=O)- NH-(C₂-C₆)-alcénylène-R9, COO-R8, COO-(C₁-C₆)- alkylène-R8, COO-(C₂-C₆)-alcénylène-R9, alcynylène- R9, (C₁-C₄-alkyl)-hétérocycle ;
R8, R9 signifient, indépendamment l'un de l'autre, H, F, Cl, Br, I, OH, CF₃, aryle, hétérocycle, (C₃-C₈)- cycloalkyle, où les cycles ou systèmes cycliques peuvent être jusqu'à trisubstitués par F, Cl, Br, I, OH, CF₃, NO₂, CN, OCF₃, O-(C₁-C₆)-alkyle, (C₁-C₆)- alkyle, NH₂, CON(R11)(R12), N(R13)(R14), SO₂-CH₃, COOH, COO-(C₁-C₆)-alkyle, CONH₂ ;
R2 signifie H, F, Cl, Br, I, OH, CF₃, CN, OCF₃, O-(C₁- C₆)-alkyle, O-(C₁-C₄)-alcoxy-(C₁-C₄)-alkyle, S-(C₁- C₆)-alkyle, (C₁-C₆)-alkyle, (C₂-C₆) -alcényle, (C₃- C₈)-cycloalkyle, O- (C₃-C₈) -cycloalkyle, (C₃-C₈)- cycloalcényle, O-(C₃-C₈)-cycloalcényle, (C₂-C₆)- alcynyle, aryle, O-aryle, (C₁-C₈)-alkylène-aryle, O-(C₁-C₈)-alkylène-aryle, S-aryle, CON(R11)(R12), (C₁-C₆)-alkyl-N(R13)(R14), COOH, COO-(C₁-C₆)-alkyle, COO- (C₂-C₆) -alcényle, CO-N((C₁-C₆)-alkyle)₂, hétérocycle, où l'hétérocycle ne peut pas être lié via un atome d'azote ;
R3, R4, R5 signifient indépendamment l'un de l'autre H, F, Cl, Br, I, OH, CF₃, NO₂, CN, OCF₃, O- (C₁-C₆)-alkyle, O-(C₁-C₄)-alcoxy-(C₁-C₄)-alkyle, S- (C₁-C₆)-alkyle, (C₁-C₆)-alkyle, (C₂-C₆)-alcényle, (C₃-C₈)-cycloalkyle, O-(C₃-C₈)-cycloalkyle, (C₃-C₈)- cycloalcényle, O-(C₃-C₈)-cycloalcényle, (C₂-C₆)- alcynyle, aryle, O-aryle, (C₀-C₈)-alkylène-aryle, O-(C₀-C₈)-alkylène-aryle, S-aryle, N((C₁-C₆)- alkyle)₂, SO₂-CH₃, COOH, COO-(C₁-C₆)-alkyle, CO- N((C₁-C₆)-alkyle)₂ ;
R6 signifie H, F, Cl, Br, I, OH, CF₃, NO₂, CN, OCF₃, O-(C₁-C₆)-alkyle, O-(C₁-C₄)-alcoxy-(C₁-C₄)-alkyle, S-(C₁-C₆)-alkyle, (C₁-C₆)-alkyle, (C₂-C₆) -alcényle, (C₃-C₈)-cycloalkyle, O-(C₃-C₈)-cycloalkyle, (C₃-C₈)- cycloalcényle, O-(C₃-C₈)-cycloalcényle, (C₂-C₆)- alcynyle, aryle, O-aryle, (C₁-C₈)-alkylène-aryle, O-(C₁-C₈)-alkylène-aryle, S-aryle, N((C₁-C₆)- allyle)₂, SO₂-CH₃, COOH, COO-(C₁-C₆)-alkyle, CO- N((C₁-C₆)-alkyle)₂ ;
ainsi que leurs sels physiologiquement acceptables.

3. Composés de formule I, selon la revendication 1 ou 2, **caractérisés en ce que**
A signifie aryle, où le cycle aryle peut être substitué par F, Cl, Br, NO₂, CF₃, OCF₃, CN, (C₁- C₆)-alkyle, aryle, CON(R11)(R12), N(R13)(R14), OH, O-(C₁-C₆)-alkyle, S-(C₁-C₆)-alkyle, N(R15)CO(C₁-C₆)- alkyle ou COO-(C₁-C₆)-alkyle ;
R11, R12, R13, R14, R15 signifient, indépendamment l'un de l'autre H, (C₁-C₆)-alkyle ;
m vaut 1 ;
R1 signifie (C₁-C₆)-alkylène-R8, (C₂-C₆)-alcénylène- R9 ;
R8, R9 signifient indépendamment l'un de l'autre, H, F, Cl, Br, I, OH, CF₃, aryle, hétérocycle, (C₃-C₈)- cycloalkyle, où les cycles ou les systèmes cycliques peuvent être jusqu'à trisubstitués par F, Cl, Br, I, OH, CF₃, NO₂, CN, OCF₃, O-(C₁-C₆)- alkyle, (C₁-C₆)-alkyle, NH₂, CON(R11)(R12), N(R13)(R14), SO₂-CH₃, COOH, COO-(C₁-C₆)-alkyle, CONH₂ ;
R2 signifie H, F, Cl, Br, I, OH, CF₃, CN, OCF₃, O-(C₁- C₆)-alkyle, (C₁-C₆)-alkyle, COO-(C₂-C₆)-alcényle, (C₁-C₆)-alkyl-N(R13)(R14) ;
R3 signifie H
R4, R5 signifient, indépendamment l'un de l'autre, H, F, Cl, Br, OH, CF₃, OCF₃, O-(C₁-C₆)-alkyle, (C₁-C₆)- alkyle ;
R6 signifie H ;
ainsi que leurs sels physiologiquement acceptables.

4. Composés selon l'une ou plusieurs des revendications 1 à 3, **caractérisés en ce que**
A signifie aryle, où le cycle aryle peut être substitué par F, Cl, Br, NO₂, CF₃, OCF₃, CN, (C₁- C₆)-alkyle, aryle, CON(R11)(R12), N(R13)(R14), OH, O-(C₁-C₆)-alkyle, S-(C₁-C₆)-alkyle, N(R15)CO(C₁-C₆)- alkyle ou COO-(C₁-C₆)-alkyle ;
R11, R12, R13, R14, R15 signifient, indépendamment l'un de l'autre H, (C₁-C₆)-alkyle ;
m vaut 1 ;
n vaut 1 ;
R1 signifie (C₁-C₆)-alkyle, (C₂-C₆)-alcényle ;
R2 signifie H, OH, (C₁-C₆) -alkyle, COO-(C₂-C₆)- alcényle, (C₁-C₆)-alkyl-N(R13)(R14) ;
R3 signifie H
R4 signifie F, Cl, Br, OH, CF₃, OCF₃, O-(C₁-C₆)-alkyle, (C₁-C₆)-alkyle ;
R5 signifie H, F, Cl, Br, OH, CF₃, OCF₃, O-(C₁-C₆)- alkyle, (C1-C6)-alkyle ;
R6 signifie H ;
ainsi que leurs sels physiologiquement acceptables.

5. Composés selon l'une ou plusieurs des revendications 1 à 4 destinés à être utilisés comme médicament.

6. Médicament contenant un ou plusieurs des composés selon l'une ou plusieurs des revendications 1 à 4.

7. Médicament contenant un ou plusieurs des composés selon l'une ou plusieurs des revendications 1 à 4 et une ou plusieurs substances actives anorexiques.

8. Médicament contenant un ou plusieurs des composés selon l'une ou plusieurs des revendications 1 à 4 et une ou plusieurs statines.

9. Médicament selon la revendication 6, **caractérisé en ce qu'**il contient comme autre substance active un(e) ou plusieurs antidiabétiques, substances actives hypoglycémiques, inhibiteurs de la HMGCoA-réductase, inhibiteurs de la résorption de cholestérol, agonistes de PPAR gamma, agonistes de PPAR alpha, agonistes de PPAR alpha/gamma, fibrates, inhibiteurs de MTP, inhibiteurs de la résorption de l'acide biliaire, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate lyase, inhibiteurs de la squalène synthétase, antagonistes de la lipoprotéine (a), inhibiteurs de lipase, insulines, sulfonylurées, biguanides, méglitinides, thiazolidinediones, inhibiteurs d'α-glucosidase, substances actives agissant sur le canal potassique dépendant de l'ATP des cellules bêta, agonistes de CART, agonistes de NPY, antagonistes de MCH, agonistes d'orexine, agonistes de H3, agonistes de TNF, agonistes de CRF, antagonistes de CRF BP, agonistes d'urocortine, agonistes de β3, agonistes de la MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, antagonistes de galanine, hormones de croissance, composés libérant les hormones de croissance, agonistes de TRH, modulateurs 2 ou 3 de la protéine découplante, agonistes de la leptine, agonistes de DA (bromocriptine, doprexine), inhibiteurs de lipase/amylase, modulateurs de PPAR, modulateurs de RXR ou agonistes de TR-β ou amphétamines.

10. Composés selon l'une ou plusieurs des revendications 1 à 4 en combinaison avec au moins une autre substance active anorexigène pour une utilisation comme médicament destiné à la prophylaxie ou au traitement de l'obésité.

11. Composés selon l'une ou plusieurs des revendications 1 à 4 en combinaison avec au moins une autre substance active anorexigène pour une utilisation comme médicament destiné à la prophylaxie ou au traitement du diabète de type II.

12. Procédé pour la préparation d'un médicament contenant un ou plusieurs des composés selon l'une ou plusieurs des revendications 1 à 4, **caractérisé en ce que** la substance active est mélangée avec un support pharmaceutiquement approprié et ce mélange est amené dans une forme appropriée pour l'administration.

13. Utilisation des composés selon l'une ou plusieurs des revendications 1 à 4 ou des composés de formule I 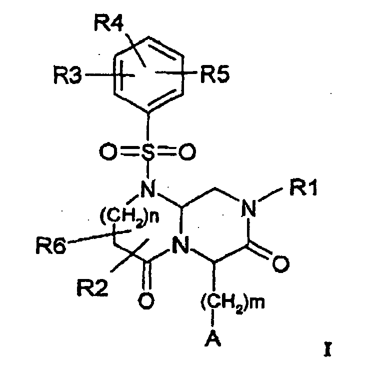 dans laquelle
A signifie un cycle monocyclique, bicyclique ou spirobicyclique de 3-12 chaînons, qui peut contenir un ou plusieurs hétéroatomes du groupe N, O et S et le cycle de 3-12 chaînons peut porter d'autres substituants, tels que F, Cl, Br, NO₂, CF₃, OCF₃, CN, (C₁-C₆)-alkyle, aryle, CON(R11)(R12), N(R13)(R14), OH, O-(C₁-C₆)-alkyle, S-(C₁-C₆)-alkyle, N(R15)CO(C₁-C₆)-alkyle ou COO-(C₁-C₆)-alkyle ;
R11, R12, R13, R14, R15 signifient, indépendamment l'un de l'autre H, (C₁-C₆)-alkyle, hétérocycle ;
n vaut 0, 1 ;
m vaut 0, 1, 2, 3, 4, 5, 6 ;
R1 signifie R8, (C₁-C₆)-alkylène-R8, (C₂-C₆)- alcénylène-R9, (SO₂)-R8, (SO₂)-(C₁-C₆)-alkylène-R8, (SO₂)-(C₂-C₆)-alcénylène-R9, (C=O)-R8, (C=O)-(C₁- C₆)-alkylène-R8, (C=O)NH-R8, (C=O)-(C₂-C₆)- alcénylène-R9, (C=O)-NH-(C₁-C₆)-alkylène-R8, (C=O)- NH-(C₂-C₆)-alcénylène-R9, COO-R8, COO-(C₁-C₆)- alkylène-R8, COO-(C₂-C₆)-alcénylène-R9, alcynylène- R9, (C₁-C₄-alkyl)-hétérocycle, où les groupes alkylène peuvent être substitués par F ;
R8, R9 signifient, indépendamment l'un de l'autre, H, F, Cl, Br, I, OH, CF₃, aryle, hétérocycle, (C₃-C₈)- cycloalkyle, où les cycles ou systèmes cycliques peuvent être jusqu'à trisubstitués par F, Cl, Br, I, OH, CF₃, NO₂, CN, OCF₃, O-(C₁-C₆)-alkyle, (C₁-C₆)- alkyle, NH₂, CON(R11)(R12), N(R13)(R14), SO₂-CH₃, COOH, COO-(C₁-C₆)-alkyle, CONH₂ ;
R2 signifie H, F, Cl, Br, I, OH, CF₃, CN, OCF₃, O-(C₁- C₆)-alkyle, O-(C₁-C₄)-alcoxy-(C₁-C₄)-alkyle, S-(C₁- C₆)-alkyle, (C₁-C₆)-alkyle, (C₂-C₆)-alcényle, (C₃- C₈)-cycloalkyle, O-(C₃-C₈)-cycloalkyle, (C₃-C₈)- cycloalcényle, O-(C₃-C₈)-cycloalcényle, (C₂-C₆)- alcynyle, aryle, O-aryle, (C₁-C₈)-alkylène-aryle, O-(C₁-C₈)-alkylène-aryle, S-aryle, CON(R11)(R12), (C₁-C₆)-alkyl-N(R13)(R14), COOH, COO-(C₁-C₆)-alkyle, COO- (C₁-C₆) -alcényle, CO-N((C₁-C₆)-alkyle)₂, hétérocycle, où l'hétérocycle ne peut pas être lié via un atome d'azote ;
R3, R4, R5 signifient indépendamment l'un de l'autre H, F, Cl, Br, I, OH, CF₃, NO₂, CN, OCF₃, O- (C₁-C₆)-alkyle, O-(C₁-C₄)-alcoxy-(C₁-C₄)-alkyle, S- (C₁-C₆)-alkyle, (C₁-C₆)-alkyle, (C₂-C₆)-alcényle, (C₃-C₈)-cycloalkyle, O-(C₃-C₈)-cycloalkyle, (C₃-C₈)- cycloalcényle, O-(C₃-C₈)-cycloalcényle, (C₂-C₆)- alcynyle, aryle, O-aryle, (C₁-C₈)-alkylène-aryle, O-(C₁-C₈)-alkylène-aryle, S-aryle, N((C₁-C₆)- alkyle)₂, SO₂-CH₃, COOH, COO-(C₁-C₆)-alkyle, CO- N((C₁-C₆)-alkyle)₂ ;
R6 signifie H, F, Cl, Br, I, OH, CF₃, NO₂, CN, OCF₃, O-(C₁-C₆)-alkyle, O-(C₁-C₄)-alcoxy-(C₁-C₄)-alkyle, S-(Cₗ-C₆)-alkyle, (C₁-C₆)-alkyle, (C₂-C₆)-alcényle, (C₃-C₈)-cycloalkyle, O-(C₃-C₈)-cycloalkyle, (C₃-C₈)- cycloalcényle, O-(C₃-C₈)-cycloalcényle, (C₂-C₆)- alcynyle, (C₀-C₈)-alkylène-aryle, O-(C₀-C₈)- alkylène-aryle, S-aryle, N((C₁-C₆)-alkyle)₂, SO₂- CH₃, COOH, COO-(C₁-C₆)-alkyle, CO-N((C₁-C₆)- alkyle)₂ ;
ainsi que leurs sels physiologiquement acceptables pour la préparation d'un médicament destiné à la réduction pondérale chez les mammifères.

14. Utilisation des composés selon l'une ou plusieurs des revendications 1 à 4 ou 13 pour la préparation d'un médicament destiné à la prophylaxie ou au traitement de l'obésité.

15. Utilisation des composés selon l'une ou plusieurs des revendications 1 à 4 ou 13 pour la préparation d'un médicament destiné à la prophylaxie ou au traitement du diabète de type II.

16. Utilisation des composés selon l'une ou plusieurs des revendications 1 à 4 ou 13 pour la préparation d'un médicament destiné à la prophylaxie ou au traitement du syndrome métabolique.

17. Utilisation des composés selon l'une ou plusieurs des revendications 1 à 4 ou 13 pour la préparation d'un médicament destiné au traitement de troubles sexuels féminins et masculins.
